# EUROPEAN PATENT APPLICATION

(11) **EP 2 871 236 A1**
(43) Date of publication of application: **13.05.2015**
(21) Application number: 13813692.4
(22) Date of filing: 05.07.2013
(51) Int. Cl.: C12N 15/09, A61K 45/00, A61P 35/00, C07K 14/47, C07K 14/71, C07K 14/82, C07K 19/00, C12Q 1/68, G01N 33/53, G01N 33/574

(54) **FGFR2 FUSION GENE**

(30) Priority: 05.07.2012 JP 2012151352
(71) Applicant: National Cancer Center, Tokyo 1040045 (JP); LSIP, LLC, Chiyoda-ku Tokyo 100-0005 (JP)
(72) Inventor: SHIBATA, Tatsuhiro, Tokyo 104-0045 (JP)
(74) Representative: Cabinet Plasseraud
(86) International application number: PCT/JP2013/068505
(87) International publication number: WO 2014/007369

(57) **Abstract**

In order to identify genes that can serve as indicators for predicting the effectiveness of drug treatments in cancers and provide novel methods for predicting the effectiveness of treatments with drugs targeting said genes, transcriptome sequencing was performed of biliary tract cancer. As a result, in-frame fusion transcripts between the FGFR2 gene and other gene (BICC1 or AHCYL1 gene) were identified. It was also found that said gene fusions induce activation of FGFR2 protein, thereby causing canceration of cells. Further, it was demonstrated that the FGFR2 protein activation and canceration caused by said gene fusions can be suppressed by using an FGFR2 inhibitor, and that treatments with an FGFR2 inhibitor are effective in patients with detection of said gene fusions.

## Description

### TECHNICAL FIELD

The present invention relates to FGFR2 fusion genes, and more particularly to polynucleotides encoding fusion polypeptides between FGFR2 protein and other protein, polypeptides encoded by said polynucleotides, and a method for detecting said polynucleotides or polypeptides. This invention also relates to a method for determining the effectiveness of cancer treatments with an FGFR2 inhibitor targeting said polynucleotides or polypeptides. This invention further relates to a method for cancer treatment using said effectiveness determination. Furthermore, this invention relates to agents for use in these methods.

### BACKGROUND ART

Biliary tract cancer is a very invasive cancer arising from biliary epithelial cells of intrahepatic biliary tracts (for intrahepatic biliary cancer) and extrahepatic biliary tracts (for extrahepatic biliary cancer). The frequency of this cancer is high mainly in East Asia, but has in recent years been increasing throughout the world including in Europe and the United States. Because of lack of clinical symptoms in the early stage of development, this cancer would in many cases be discovered in advanced stages, and its prognosis is poor. Surgical resection is the only procedure for achieving complete cure, but this cancer has a high rate of recurrence -- the 5-year survival rate of operable biliary tract cancer patients is in the range of 15 to 25%. There has not yet been known any chemotherapy that achieves an apparent complete response in inoperable or recurrent patients. However, it has recently been reported that the use of gemcitabine hydrochloride alone or in combination with a platinating agent yields a somewhat more positive outcome than other chemotherapies. Accordingly, there has been a strong need for a new effective therapy for biliary tract cancer, including molecular targeted therapies, and various candidates for the molecular target have heretofore been reported, including EGFR, VEGFR and c-MET.

As driver mutations for biliary tract cancer, KRAS and BRAF mutations have been reported, and the GOPC-ROS1 fusion which had been identified in a brain tumor as a tyrosine kinase fusion gene was also recently reported in biliary tract cancer. No driver mutation has still been identified in patients with no such alterations.

The FGFR (fibroblast growth factor receptor) family is a generic name for transmembrane tyrosine kinase molecules functioning as receptors for FGFs (fibroblast growth factors). There are four known types of FGFR family molecules in humans: FGFRs 1 to 4. The FGFR family contains three immunoglobulin (Ig)-like domains and one transmembrane domain in its extracellular region and a tyrosine kinase domain in its intracellular region. This family is known to contribute to the occurrence and development of many cancers including stomach cancer, breast cancer, uterine cancer, and bladder cancer, and is reported to induce activation in cancers due to various types of genetic alterations (Non-patent Document 1).

For example, as to FGFR1, it is known that gene amplification occurs in breast and ovarian cancers, gene mutation occurs in melanoma, and gene translocation occurs in leukaemia and breast cancer. As to FGFR2, it is known that gene amplification occurs in stomach and breast cancers, and gene mutation occurs in uterine and stomach cancers. As to FGFR3, it is known that gene amplification occurs in bladder and salivary gland cancers, and gene mutation occurs in bladder cancer, uterine cancer, myeloma, and prostate cancer.

The only fusion genes involving FGFR that have heretofore been reported are fusion genes with FGFR1, many of which have been identified in hematological malignancy. Exemplary FGFR1 fusion genes reported in hematological malignancy include FGFR1OP2-FGFR1 (Non-patent Document 2) and ZNF198-FGFR1 (Non-patent Document 3), and the FGFR1 fusion gene reported in solid cancer is FGFR1-ZNsF703 in breast cancer (Non-patent Document 4). Regarding aberrant FGFR signaling in biliary tract cancer, expression of the FGFR ligand, FGF, was reported in a literature (Non-patent Document 5).

At present, clinical development is underway of low-molecular-weight inhibitor compounds targeting FGFR, including AZD4547, TKI258, E3810, E7080, BIBF1120, Masitinib, and BGJ398. The development of an antibody therapeutic agent targeting FGFR is also currently in progress (Non-patent Document 1).

On the other hand, BICC1 protein is a molecule having an RNA binding domain and a SAM (sterile alpha motif) domain involved in protein-protein interaction, and negatively regulates Wnt pathway. It was reported that functional abnormalities in this protein cause polycystic kidney disease and pancreas development defects (Non-patent Document 6). Also, AHCYL1 protein is known to have a similar domain to S-adenosyl-L-homocysteine hydrolase and to be involved in secretion in epithelial cells (Non-patent Document 7).

However, the presence of fusion genes involving any of the FGFR2, BICC1 and AHCYL1 genes, and the presence or absence of relationship between such a fusion gene and cancer have yet to be clarified. Also, elucidation of fusion genes and other genes in cancers including biliary tract cancer has not yet been fully achieved. So there is a demand for identifying mutant genes and fusion genes that can serve as indicators for predicting the effectiveness of drug treatments.

### CITATION LIST

### NON-PATENT DOCUMENTS

Non-patent Document 1: Ahmad I, et al., Biochim Biophys Acta., 2012, vol. 1823, p. 850-860
Non-patent Document 2: Popovici C., et al., Blood, 1999, vol. 93, p. 1381-1389
Non-patent Document 3: Xiao S., et al., Nature Genet., 1998, vol. 18, p. 84-87 Non-patent Document 4: "Catalogue of Somatic Mutations in Cancer" (Translocation; Genes: FGFR1/ZNF703", [online], last update: 28 September 2011, Wellcome Trust Sanger Institute, U.K., website <URL: http://www.sanger.ac.uk/perl/genetics/CGP/cosmic?action=translocations&id=174&fused=5 9536>
Non-patent Document 5: Ogasawara S., et al., Hepatol Res., 2001, vol. 20, p. 97-113
Non-patent Document 6: Kraus MR., et al., Hum Mutat., 2012, vol. 33, p. 86-90
Non-patent Document 7: Yang D., et al., J Clin Invest., 2011, vol. 121, p. 956-965

### SUMMARY OF INVENTION

### TECHNICAL PROBLEM

The present invention has been made in consideration of the above-described problems with the prior art, and has as its object to identify genes that can serve as indicators for predicting the effectiveness of drug treatments in biliary tract cancer and other cancers. Another object of this invention is to provide novel methods for predicting the effectiveness of drug treatments targeting said genes and expression products thereof. Still another object of this invention is to provide methods for treating biliary tract cancer and other cancers on the basis of the prediction of the effectiveness of drug treatments targeting said genes and expression products thereof. Yet another object of this invention is to provide agents for use in detecting said genes and expression products thereof in these methods.

### SOLUTION TO PROBLEM

As a result of intensive studies to achieve the above-mentioned objects, the present inventors have identified in-frame fusion transcripts between the FGFR2 gene and other gene (BICC1 gene or AHCYL1 gene) by performing transcriptome sequencing of 8 biliary tract cancer (BTC) specimens. The inventors have found that the fusion gene between the FGFR2 gene and the BICC1 gene (FGFR2-BICC1 fusion gene) is generated by an inversion in chromosome 10, and that the fusion gene between the FGFR2 gene and the AHCYL1 gene (FGFR2-AHCYL1 fusion gene) is generated by a reciprocal translocation between chromosome 1 and chromosome 10. We also have investigated the appearance frequencies of these fusion genes in 102 BTC specimens and, as a result, have found that the appearance frequencies of the FGFR2-AHCYL1 and FGFR2-BICC1 fusion genes are about 6.9% and about 2.0%, respectively.

Further, it is considered that these gene fusions induce activation of FGFR2 protein and hence FGFR2 inhibitors may be therapeutically effective in patients with such activation. Thus, the inventors have introduced each of these fusion genes into normal cells and, as a result, have demonstrated that these cells acquire anchorage-independent colony-forming ability, in other words become cancerous. We also have found that in these cells, the FGFR2 protein kinase is activated and also phosphorylation of its downstream signal, MAPK, is increased.

Further, the inventors have subcutaneously transplanted FGFR2 fusion gene-expressing cells into nude mice and, as a result, have demonstrated that said cells have *in vivo* tumorigenic ability.

On the other hand, it has also been demonstrated that the activation of FGFR2 protein kinase, the phosphorylation of MAPK, the anchorage-independent colony-forming ability, and the *in vivo* tumorigenic ability are significantly suppressed by using an FGFR2 inhibitor or through inactivation of the kinase domains of the FGFR2 fusion polypeptide.

On the basis of the above-described findings, the present inventors have found that it is possible to predict the effectiveness of treatments with an FGFR2 inhibitor targeting these gene fusions in biliary tract cancer and other cancers, and that efficient treatments can be performed by administering the inhibitor to patients in whom the treatments with the inhibitor have been determined to be effective on the basis of this prediction; thus, the inventors have completed the present invention.

Therefore, the present invention relates to polynucleotides encoding fusion polypeptides between FGFR2 protein and other protein, polypeptides encoded by said polynucleotides, a method for detecting said polynucleotides or polypeptides, a method for determining the effectiveness of cancer treatments with an FGFR2 inhibitor using the presence of said polynucleotides or polypeptides as an indicator, a method for treatment of cancer utilizing said effectiveness determination, and agents for use in these methods. More specifically, this invention provides the following:
<1> A polynucleotide encoding a polypeptide in which FGFR2 protein and other protein are fused together, wherein the polypeptide is expressed in a cancer cell.
<2> The polynucleotide as set forth in <1>, wherein said other protein is BICC1 protein or AHCYL1 protein.
<3> The polynucleotide as set forth in <1> or <2>, wherein the cancer cell is a biliary tract cancer cell.
<4> A polypeptide encoded by the polynucleotide as set forth in any one of <1> to <3>.
<5> A method for detecting the presence or absence in a sample of the polynucleotide as set forth in any one of <1> to <3> or of the polypeptide as set forth in <4>, the method comprising the steps of:
   (a) contacting the sample with an agent intended for specifically detecting the presence or absence of the polynucleotide or the polypeptide in the sample; and
   (b) detecting the presence or absence of the polynucleotide or the polypeptide.
<6> An agent for detecting the presence or absence in a sample of the polynucleotide as set forth in any one of <1> to <3> or of the polypeptide as set forth in <4> by the method as set forth in <5>, the agent comprising a polynucleotide or polynucleotides as set forth below in any one of (a) to (c), the polynucleotide or polynucleotides having a chain length of at least 15 nucleotides, or an antibody as set forth below in (d):
   (a) a polynucleotide or polynucleotides that are at least one probe selected from the group consisting of a probe that hybridizes to a polynucleotide encoding FGFR2 protein and a probe that hybridizes to a polynucleotide encoding other protein;
   (b) a polynucleotide that is a probe that hybridizes to a point of fusion between a polynucleotide encoding FGFR2 protein and a polynucleotide encoding other protein;
   (c) polynucleotides that are a pair of primers designed to sandwich a point of fusion between a polynucleotide encoding FGFR2 protein and a polynucleotide encoding other protein; and
   (d) an antibody that binds to a polypeptide in which FGFR2 protein and other protein are fused together.
<7> A method for determining the effectiveness of a cancer treatment with an FGFR2 inhibitor, the method comprising the step of detecting the presence or absence in a sample isolated from a patient of the polynucleotide as set forth in any one of <1> to <3> or of the polypeptide as set forth in <4>, wherein in a case where the presence of the polynucleotide or the polypeptide is detected, the cancer treatment with the FGFR2 inhibitor is determined to be highly effective in the patient.
<8> An agent for determining the effectiveness of a cancer treatment with an FGFR2 inhibitor by the method as set forth in <7>, the agent comprising a polynucleotide or polynucleotides as set forth below in any one of (a) to (c), the polynucleotide or polynucleotides having a chain length of at least 15 nucleotides, or an antibody as set forth below in (d):
   (a) a polynucleotide or polynucleotides that are at least one probe selected from the group consisting of a probe that hybridizes to a polynucleotide encoding FGFR2 protein and a probe that hybridizes to a polynucleotide encoding other protein;
   (b) a polynucleotide that is a probe that hybridizes to a point of fusion between a polynucleotide encoding FGFR2 protein and a polynucleotide encoding other protein;
   (c) polynucleotides that are a pair of primers designed to sandwich a point of fusion between a polynucleotide encoding FGFR2 protein and a polynucleotide encoding other protein; and
   (d) an antibody that binds to a polypeptide in which FGFR2 protein and other protein are fused together.
<9> A method for treatment of cancer, comprising the step of administering an FGFR2 inhibitor to a patient in whom a cancer treatment with the FGFR2 inhibitor has been determined to be highly effective by the method as set forth in <7>.
<10> A therapeutic agent for cancer, comprising an FGFR2 inhibitor as an active ingredient, the agent which is to be administered to a patient in whom a cancer treatment with the FGFR2 inhibitor has been determined to be highly effective by the method as set forth in <7>.

### ADVANTAGEOUS EFFECTS OF INVENTION

The present invention enables effective detection of fusion genes between the FGFR2 gene and other gene, and expression products thereof. This invention also makes it possible to predict the effectiveness of various treatments on cancers, in particular the effectiveness of cancer treatments with an FGFR2 inhibitor, on the basis of the detection of said fusion genes and expression products thereof This prediction makes it possible to avoid administration of a drug to cancer patients conceivably not responsive to the administration of the drug, thereby allowing efficient cancer treatments.

### BRIEF DESCRIPTION OF DRAWINGS

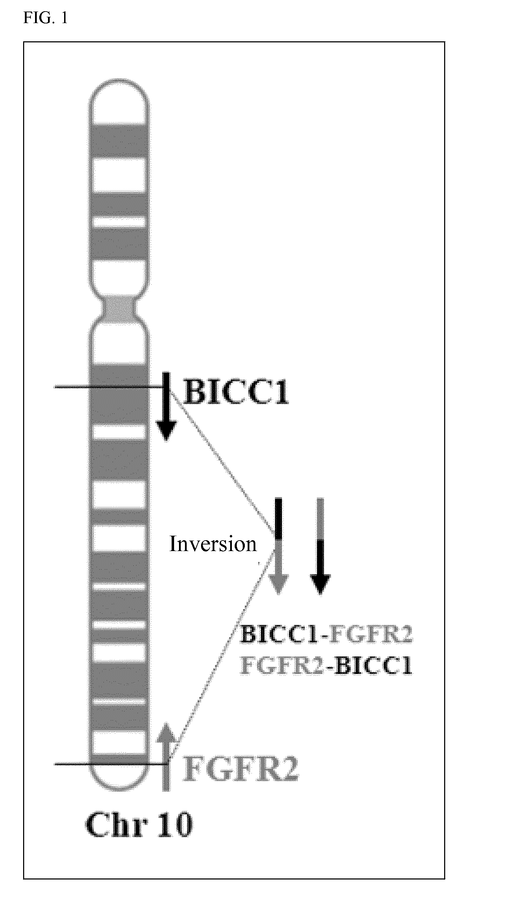

FIG. 1 is a schematic diagram showing the fusion between the FGFR2 gene and the BICC1 gene generated by an inversion in human chromosome 10.

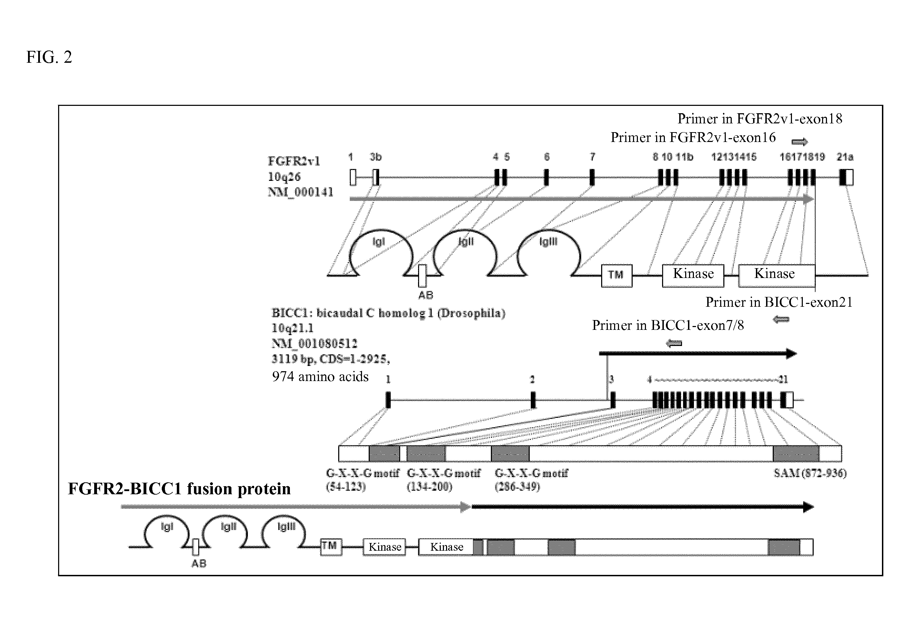

FIG. 2 is a schematic diagram showing the fusion between FGFR2 protein and BICC1 protein. More specifically, this schematic diagram shows that the extra-membranous domains (IgI to IgIII), transmembrane (TM) domain and kinase domains of FGFR2 protein are fused with the C-terminal moiety of BICC1 protein. This diagram also shows that a polynucleotide encoding the entire FGFR2 fusion polypeptide including the FGFR2 kinase region can be detected by using RT-PCR primers each hybridizing to exon 16 or 18 in the kinase region of the FGFR2 gene and to exon 7/8 or 21 in the BICC1 gene.

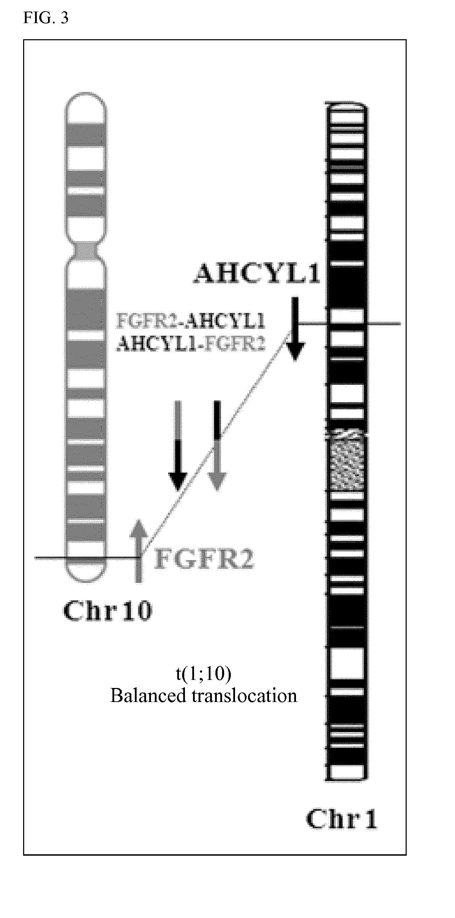

FIG. 3 is a schematic diagram showing the fusion between the FGFR2 gene and the AHCYL1 gene generated by a balanced translocation (reciprocal translation) between human chromosome 1 and human chromosome 10.

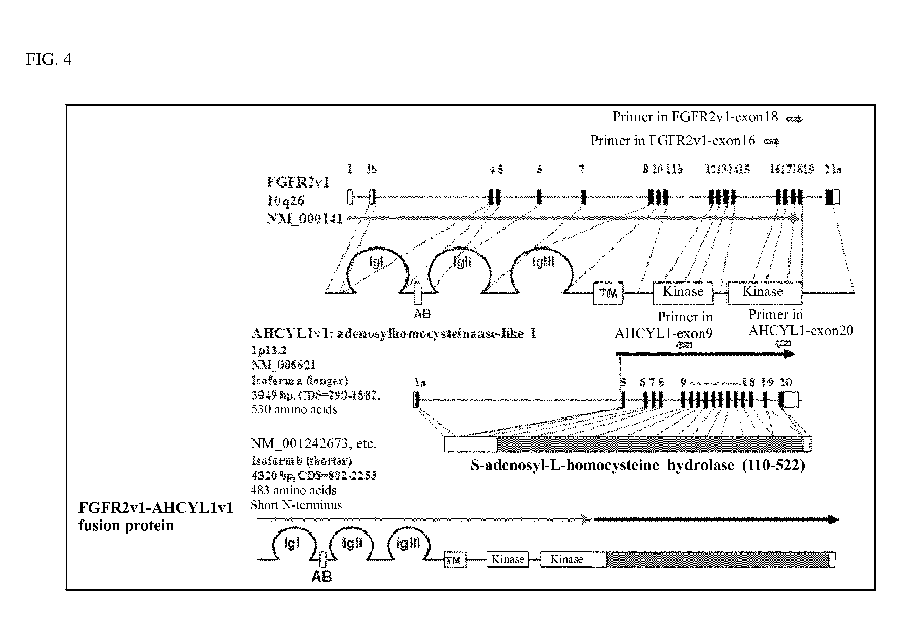

FIG. 4 is a schematic diagram showing the fusion between FGFR2 protein and AHCYL1 protein. More specifically, this schematic diagram shows that the extra-membranous domains (IgI to IgIII), transmembrane (TM) domain and kinase domains of FGFR2 protein are fused with the C-terminal moiety of AHCYL1 protein. This diagram also shows that a polynucleotide encoding the entire FGFR2 fusion polypeptide including the FGFR2 kinase region can be detected by using RT-PCR primers each hybridizing to exon 16 or 18 in the kinase region of the FGFR2 gene and to exon 9 or 20 in the AHCYL1 gene.

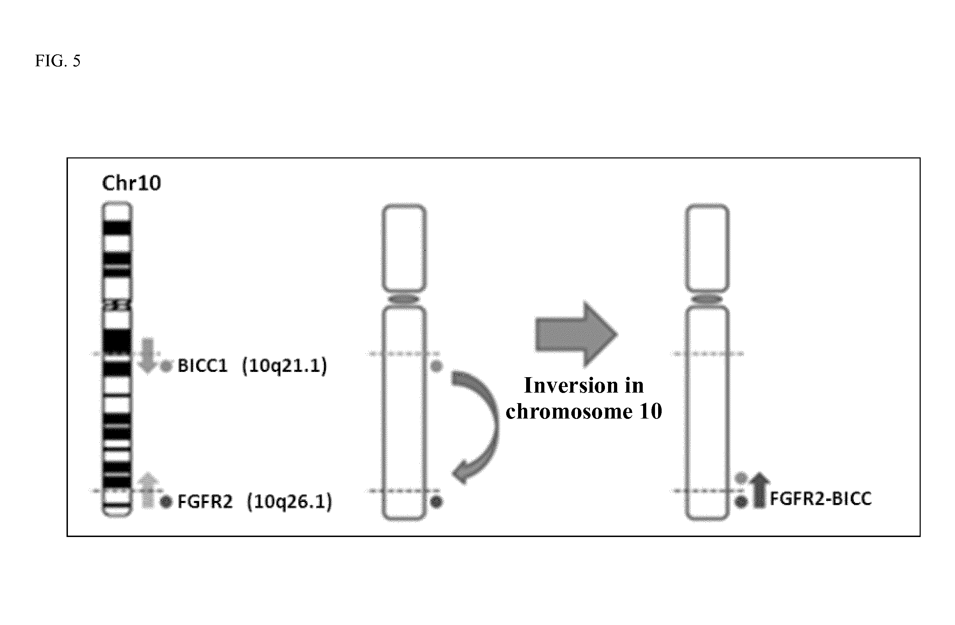

FIG. 5 is a schematic diagram showing a method for detecting the inventive fusion gene by the FISH method. More specifically, this schematic diagram shows that in the case of the FGFR2-BICC1 fusion gene, the presence of the FGFR2-BICC1 fusion gene can be detected by designing FISH probes each specific for a portion toward the 5' end of the FGFR2 gene or for a portion toward the 3' end of the BICC1 gene.

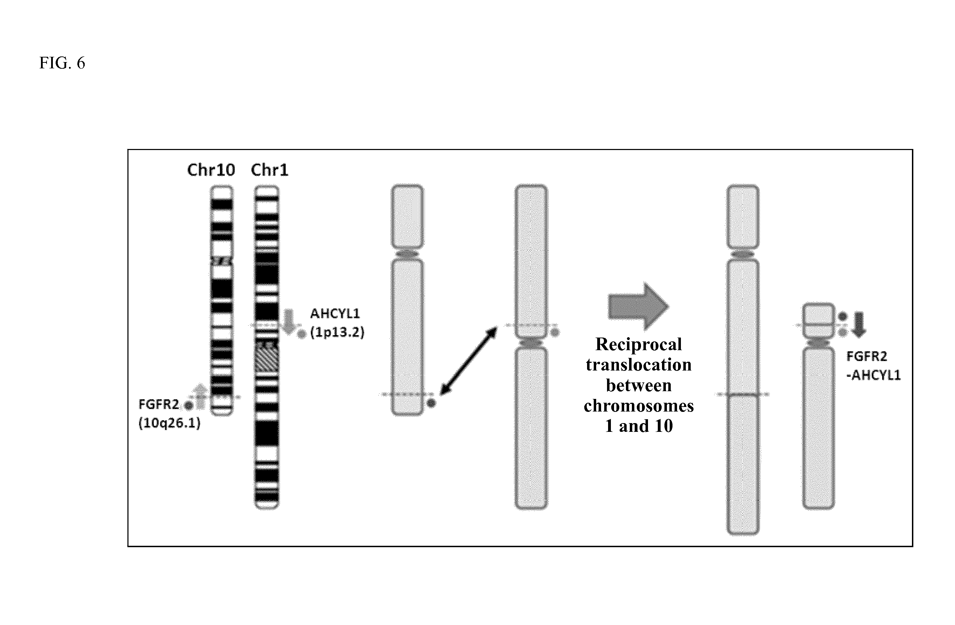

FIG. 6 is a schematic diagram showing a method for detecting the inventive fusion gene by the FISH method. More specifically, this schematic diagram shows that in the case of the FGFR2-AHCYL1 fusion gene, the presence of the FGFR2-AHCYL1 fusion gene can be detected by designing FISH probes each specific for a portion toward the 5' end of the FGFR2 gene or for a portion toward the 3' end of the AHCYL1 gene.

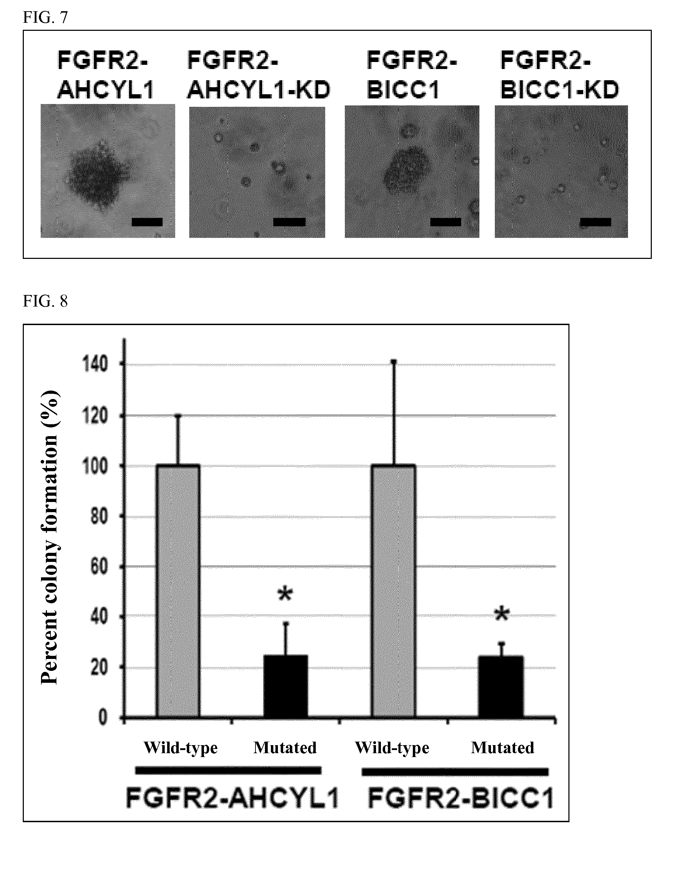

FIG. 7 is a set of photos showing the results of observing, under a microscope, the anchorage-independent colony formation in normal murine fibroblast lines each stably expressing the wild-type or the mutated FGFR2 fusion polypeptide, which were each seeded in a soft agar medium. In this figure, the "FGFR2-AHCYL1" panel shows the result for a cell line expressing the wild-type FGFR2-AHCYL1 fusion polypeptide, the "FGFR2-AHCYL1-KD" panel shows the result for a cell line expressing the mutated FGFR2-AHCYL1 fusion polypeptide, the "FGFR2-BICC1" panel shows the result for a cell line expressing the wild-type FGFR2-BICC1 fusion polypeptide, and the "FGFR2-BICC1-KD" panel shows the result for a cell line expressing the mutated FGFR2-BICC1 fusion polypeptide. The bars in these panels indicate the length of 100 µm.
FIG. 8 is a graph showing the results of analyzing the anchorage-independent colony-forming ability of normal murine fibroblast lines each stably expressing the wild-type or the mutated FGFR2 fusion polypeptide, which were each seeded in a soft agar medium. In this graph, the vertical axis represents a relative value of the number of colonies formed in each of the cell lines expressing either one of the mutated FGFR2 fusion polypeptides, with respect to that number in each of the corresponding cell lines expressing either one of the wild-type FGFR2 fusion polypeptides, which is taken as 100. An asterisk shows that a significant difference was observed (the p-value is 0.05 or less).

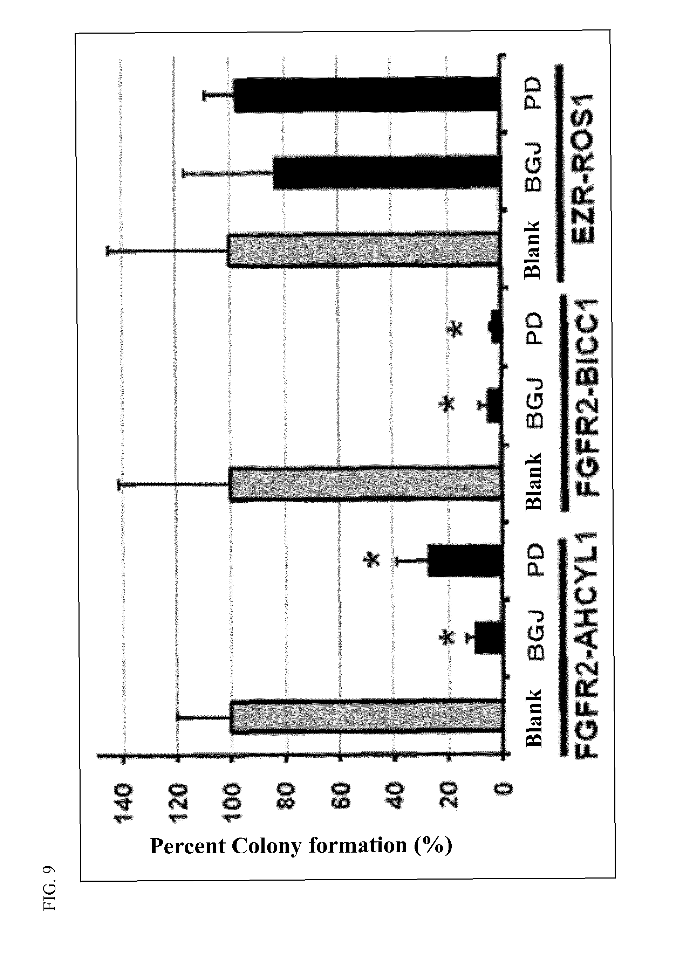

FIG. 9 is a graph showing the results of analyzing the anchorage-independent colony-forming ability of normal murine fibroblast lines stably expressing either one of the wild-type FGFR2 fusion polypeptides and a normal murine fibroblast line stably expressing the EZR-ROS1 fusion polypeptide, which were each seeded in a soft agar supplemented with an FGFR kinase inhibitor (BGJ398 or PD173074). In this graph, the "BGJ" bars show the results of colony formation in the presence of BGJ398 (at a BGJ398 concentration in medium of 0.2 nmol/mL), the "PD" bars show the results of colony formation in the presence of PD173074 (at a PD173074 concentration in medium of 0.2 nmol/mL), and the "Blank" bars show the results of colony formation in the absence of an FGFR kinase inhibitor. The vertical axis represents a relative value of the number of colonies with respect to the number of colonies formed in each of the cell lines expressing any one of said fusion polypeptides in the absence of an FGFR kinase inhibitor, which is taken as 100. An asterisk shows that a significant difference was observed (the p-value is 0.05 or less).

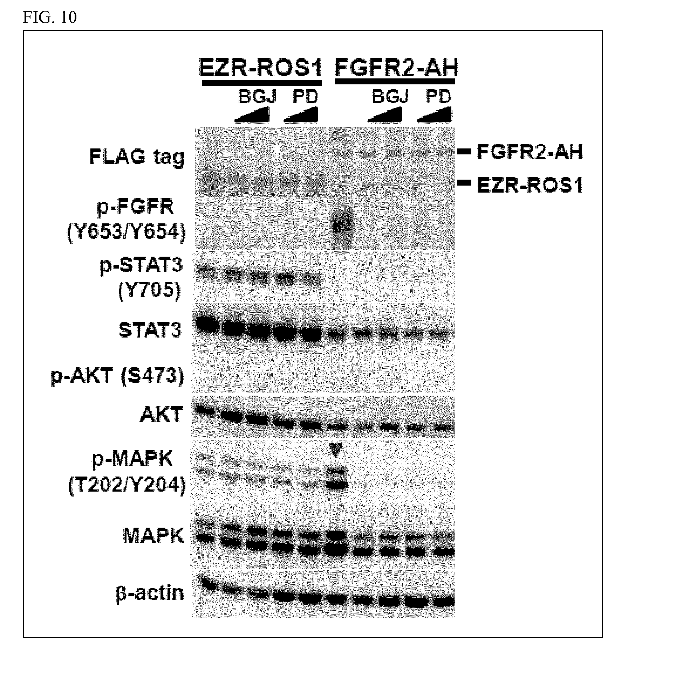

FIG. 10 is a set of photos showing the results of analyzing by Western blotting the phosphorylation of various proteins in a normal murine fibroblast line stably expressing the wild-type FGFR2-AHCYL1 fusion polypeptide ("FGFR2-AH") or a normal murine fibroblast line stably expressing the EZR-ROS1 fusion polypeptide ("EZR-ROS1"), which were subjected to serum starvation and then cultured in the presence of an FGFR kinase inhibitor (BGJ398 or PD173074). In this figure, the 1st and 6th lanes from the left show the results of culturing in the absence of an FGFR kinase inhibitor; the 2nd and 7th lanes from the left show the results of culturing in the presence of BGJ398 (at a BGJ398 concentration in medium of 0.2 nmol/mL); the 3rd and 8th lanes from the left show the results of culturing in the presence of BGJ398 (at a BGJ398 concentration in medium of 1.0 nmol/mL); the 4th and 9th lanes from the left show the results of culturing in the presence of PD173074 (at a PD173074 concentration in medium of 0.2 nmol/mL); and the 5th and 10th lanes from the left show the results of culturing in the presence of PD173074 (at a PD173074 concentration of 1.0 nmol/mL). Since these fusion polypeptides further have the FLAG tag bound thereto and express it intracellularly, the "FLAG tag" row in this figure shows the results of detection of the respective fusion polypeptides through this tag. The "p-FGFR (Y653/Y654)" row shows the results of detection of phosphorylated FGFR. The "STAT3" and "p-STAT3 (Y705)" rows show the results of detection of STAT3 and phosphorylated STAT3, respectively. The "AKT" and "p-AKT (S473)" show the results of detection of AKT and phosphorylated AKT, respectively. The "MAPK" and "p-MAPK (T202/Y204)" rows show the results of detection of MAPK and phosphorylated MAPK, respectively. The "β-actin" row shows that the amount of protein used as an internal standard is uniform among the lanes.

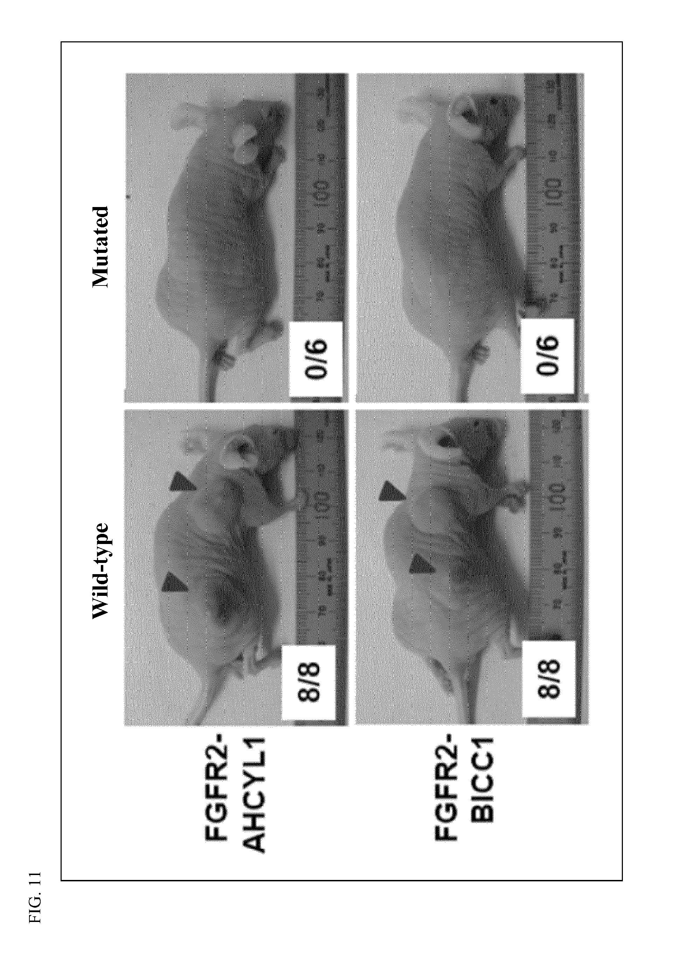

FIG. 11 is a set of photos showing the results of subcutaneously transplanting each of normal murine fibroblast lines expressing the wild-type or the mutated FGFR2 fusion polypeptide into immunodeficient mice. In this figure, the "Wild-type" panels are photos taken upon the observation of the immunodeficient mice 18 days after the mice were transplanted subcutaneously with any of the cell lines expressing either one of the wild-type FGFR2 fusion polypeptides. Triangles indicate formed tumors. "8/8" represents that each type of said cells was transplanted into two sites of each of four mice, a total of eight sites, and as a result, tumorigenesis was observed in all of the eight sites. The "mutated" panels are photos taken upon the observation of the immunodeficient mice 18 days after the mice were transplanted subcutaneously with each of the cell lines expressing either one of the mutated FGFR2 fusion polypeptides. "0/6" represents that each type of said cells was transplanted into two sites of each of three mice, a total of six sites, and as a result, no tumorigenesis was observed in any of the six sites.

### DESCRIPTION OF EMBODIMENTS

### <Polynucleotides encoding fusion polypeptides between FGFR2 protein and other protein, and polypeptides encoded by the polynucleotides>

As disclosed below in Examples, multiple cases of fusion between the FGFR2 gene and other gene were first discovered according to the present invention. Therefore, this invention provides a polynucleotide encoding a fusion polypeptide between FGFR2 protein and other protein (said polynucleotide and said fusion polypeptide are to be hereinafter also referred to as the "FGFR2 fusion polynucleotide" and the "FGFR2 fusion polypeptide", respectively).

The "FGFR2 fusion polypeptide" as referred to in the present invention means a polypeptide in which the full length or part of FGFR2 protein is fused with the full length or part of other protein. Also, the "FGFR2 fusion polynucleotide" as referred to in this invention means a polynucleotide in which a polynucleotide encoding the full length or part of FGFR2 protein is fused with a polynucleotide encoding the full length or part of other protein.

Examples of the FGFR2 fusion polynucleotide include: a polynucleotide encoding a fusion polypeptide between FGFR2 protein and BICC1 protein (said polynucleotide and said fusion polypeptide are to be hereinafter also referred to as the "FGFR2-BICC1 fusion polynucleotide" and the "FGFR2-BICC1 fusion polypeptide", respectively); and a polynucleotide encoding a fusion polypeptide between FGFR2 protein and AHCYL1 protein (said polynucleotide and said fusion polypeptide are to be hereinafter also referred to as the "FGFR2-AHCYL1 fusion polynucleotide" and the "FGFR2-AHCYL1 fusion polypeptide", respectively).

The "FGFR2 (fibroblast growth factor receptor 2) protein" according to the present invention refers to a protein encoded by the gene located at a long arm of chromosome 10 (10q26) in humans. In this invention, the "FGFR2 protein", as far as it is derived from humans, can be exemplified by: the protein identified by RefSeq ID: NP_000132 (isoform 1); the protein identified by RefSeq ID: NP_075259 (isoform 2); the protein identified by RefSeq ID: NP_001138385 (isoform 3); the protein identified by RefSeq ID: NP_001138386 (isoform 4); the protein identified by RefSeq ID: NP_001138387 (isoform 5); the protein identified by RefSeq ID: NP_001138388 (isoform 6); the protein identified by RefSeq ID: NP_001138389 (isoform 7); the protein identified by RefSeq ID: NP_001138390 (isoform 8); and the protein identified by RefSeq ID: NP_001138391 (isoform 9). These FGFR2 protein isoforms are slightly different from each other in terms of extra-membranous domain structure (e.g., the presence or absence of IgI; refer to FIGs. 2 and 4). In this invention, the "FGFR2 protein", as far as it is derived from humans, is typified by the protein consisting of the amino acid sequence of SEQ ID NO: 2 (isoform 1). The polynucleotide encoding the FGFR2 protein is typified by the polynucleotide consisting of the nucleotide sequence of SEQ ID NO: 1.

The "BICC1 (bicaudal C homolog 1 (Drosophila)) protein" according to the present invention refers to a protein encoded by the gene located at a long arm of chromosome 10 (10q21.1) in humans. In this invention, the "BICC1 protein", as far as it is derived from humans, is typified by the protein consisting of the amino acid sequence of SEQ ID NO: 4. The polynucleotide encoding the BICC1 protein is typified by the polynucleotide consisting of the nucleotide sequence of SEQ ID NO: 3.

The "AHCYL1 (S-adenosyl-L-homocysteine hydrolase-like 1; AHCY-like 1) protein" according to the present invention is also referred to as IRBIT (inositol 1,4,5-trisphosphate receptor 1 (ITPR1)-binding protein released with inositol 1,4,5-trisphosphate, IRBIT, ITPR1-binding protein released with IP3) or DCAL (dendritic cell-expressed AHCY-like protein), and means a protein encoded by the gene located at a short arm of chromosome 1 (1p13.2) in humans. In this invention, the "AHCYL1 protein", as far as it is derived from humans, can be exemplified by: the protein identified by RefSeq ID: NP_006612 (isoform a); and the protein identified by RefSeq ID: NP_001229603 (isoform b). AHCYL1 protein isoform a is characterized by having a longer N-terminus than isoform b. In this invention, the "AHCYL1 protein", as far as it is derived from humans, is typified by the protein consisting of the amino acid sequence of SEQ ID NO: 6 (isoform a). The polynucleotide encoding the AHCYL1 protein is typified by the polynucleotide consisting of the nucleotide sequence of SEQ ID NO: 5.

The FGFR2 fusion polynucleotide is typified by a polynucleotide encoding a polypeptide in which the N-terminal moiety of FGFR2 protein is fused with the C-terminal moiety of other protein.

In the present invention, the "N-terminal moiety of FGFR2 protein" is typified by a moiety comprising the extra-membranous domains, transmembrane domain, and kinase domains of the FGFR2 protein (refer to FIGs. 2 and 4).

For the purpose of the present invention, the "polynucleotide encoding a polypeptide in which the N-terminal moiety of FGFR2 protein is fused with the C-terminal moiety of BICC1 protein" is typified by a polynucleotide generated by an inversion in chromosome 10, as shown in FIGs. 1 and 5. More specifically, this term refers to a polynucleotide encoding a polypeptide in which a polypeptide region (extra-membranous domains, transmembrane domain, and kinase domains) of the FGFR2 protein that is encoded by exons 1-19 is fused with a polypeptide region of the BICC1 protein that is encoded by exons 3-21 (e.g., the polypeptide consisting of the amino acid sequence of SEQ ID NO: 8). Such a polynucleotide is exemplified by the polynucleotide consisting of the nucleotide sequence of SEQ ID NO: 7.

For the purpose of the present invention, the "polynucleotide encoding a polypeptide in which the N-terminal moiety of FGFR2 protein is fused with the C-terminal moiety of AHCYL1 protein" is typified by a polynucleotide generated by a reciprocal translocation between chromosome 1 and chromosome 10, as shown in FIGs. 3 and 6. More specifically, this term refers to a polynucleotide encoding a polypeptide in which a polypeptide region (extra-membranous domains, transmembrane domain, and kinase domains) of the FGFR2 protein that is encoded by exons 1-19 are fused with a polypeptide region of the AHCYL1 protein that is encoded by exons 5-20 (e.g., the polypeptide consisting of the amino acid sequence of SEQ ID NO: 10). Such a polynucleotide is exemplified by the polynucleotide consisting of the nucleotide sequence of SEQ ID NO: 9.

In the present invention, the amino acid sequences of "FGFR2 protein", "BICC1 protein" and "AHCYL1 protein", and the nucleotide sequences of the genes encoding said proteins can mutate in nature (i.e., in a non-artificial way). Thus, the amino acid sequence of the "FGFR2 fusion polypeptide" and the nucleotide sequence of the "FGFR2 fusion polynucleotide" can also mutate in nature (i.e., in a non-artificial way). Said amino acid sequences and nucleotide sequences may be artificially modified. Such mutants are also encompassed by this invention.

Certain exemplary mutants of the FGFR2-BICC1 fusion polypeptide include proteins consisting of an amino acid sequence derived from the amino acid sequence of SEQ ID NO: 8 by substitution, deletion, addition and/or insertion of one or more amino acids. Certain exemplary mutants of the FGFR2-AHCYL1 fusion polypeptide include proteins consisting of an amino acid sequence derived from the amino acid sequence of SEQ ID NO: 10 by substitution, deletion, addition and/or insertion of one or more amino acids.

As used herein, the term "more" refers to generally 50 or fewer amino acids, preferably 30 or fewer amino acids, more preferably 10 or fewer amino acids, and particularly preferably several or fewer amino acids (for example, five or fewer amino acids, three or fewer amino acids, two or one amino acid, one amino acid).

Other exemplary mutants of the FGFR2-BICC1 fusion polypeptide include polypeptides encoded by a DNA that hybridizes under stringent conditions to a DNA consisting of the nucleotide sequence of SEQ ID NO: 7. Other exemplary mutants of the FGFR2-AHCYL1 fusion polypeptide include polypeptides encoded by a DNA that hybridizes under stringent conditions to a DNA consisting of the nucleotide sequence of SEQ ID NO: 9.

Exemplary high stringent hybridization conditions are 0.2×SSC at 65°C, and exemplary low stringent hybridization conditions are 2.0×SSC at 50°C.

Still other exemplary mutants of the FGFR2-BICC1 fusion polypeptide include polypeptides consisting of an amino acid sequence having at least 80% (for example, at least 85%, 90%, 95%, 97%, 99%) homology to the amino acid sequence of SEQ ID NO: 8. Still other exemplary mutants of the FGFR2-AHCYL1 fusion polypeptide include polypeptides consisting of an amino acid sequence having at least 80% (for example, at least 85%, 90%, 95%, 97%, 99%) homology to the amino acid sequence of SEQ ID NO: 10.

Sequence homology can be determined using the BLASTP (amino acid level) program (Altschul, et al., J. Mol. Biol., 1990, 215: 403-410). This program is based on the algorithm BLAST developed by Karlin and Altschul (Proc. Natl. Acad. Sci. USA, 1990, 87: 2264-2268; and Proc. Natl. Acad. Sci. USA, 1993, 90: 5873-5877). When amino acid sequence analysis is performed using BLASTP, the parameters are typically set as follows: score = 50 and wordlength = 3. Amino acid sequence analysis using the Gapped BLAST program can be performed as per the descriptions in Altschul, et al. (Nucleic Acids Res., 1997, 25: 3389-3402). When amino acid sequence analysis is performed using the BLAST and Gapped BLAST programs, the default parameters of these programs are used. The specific procedures for conducting these analyses are known.

Exemplary mutants of the FGFR2-BICC1 fusion polynucleotide include polynucleotides encoding the above-mentioned mutants of the FGFR2-BICC1 fusion polypeptide, and polynucleotides encoding degenerate variants of said polypeptide which have no amino acid mutation. Exemplary mutants of the FGFR2-AHCYL1 fusion polynucleotide include polynucleotides encoding the above-mentioned mutants of the FGFR2-AHCYL1 fusion polypeptide, and polynucleotides encoding degenerate variants of said polypeptide which have no amino acid mutation.

Exemplary forms of the "FGFR2 fusion polynucleotide" according to the present invention include mRNA, cDNA, and genomic DNA. It is possible for those skilled in the art using a known hybridization technique to isolate the "FGFR2 fusion polynucleotide" from a cDNA library or genomic DNA library prepared from BTC or other cancers that harbor a fusion gene between the FGFR2 gene and other gene. The polynucleotide can also be prepared by amplification utilizing a known gene amplification technique (PCR), with the mRNA, cDNA or genomic DNA prepared from BTC or other cancers being used as a template.

Furthermore, after the thus-prepared polynucleotide is inserted into an appropriate expression vector, the vector is introduced into a cell-free protein synthesis system (e.g., reticulocyte extract, wheat germ extract) and the system is incubated, or alternatively the vector is introduced into appropriate cells (e.g., *E coli.*, yeast, insect cells, animal cells) and the resulting transformant is cultured; in either way, the FGFR2 fusion polypeptide can be prepared.

As mentioned above, the "FGFR2 fusion polypeptide" and "FGFR2 fusion polynucleotide" according to the present invention encompasses, in a broad sense, both those having naturally occurring sequences (including those mutated in nature) and those having artificially modified sequences. However, it should be noted that the "FGFR2 fusion polypeptide" and "FGFR2 fusion polynucleotide", particularly if these terms are used as an object of the detection as described below, mainly refer to those having naturally occurring sequences (including those mutated in nature).

### <Method for detecting the presence or absence of the FGFR2 fusion polypeptides or the FGFR2 fusion polynucleotides>

The present invention also provides a method for detecting the presence or absence of the FGFR2 fusion polynucleotides or the FGFR2 fusion polypeptides in a sample. The detection method of this invention comprises the steps of: (a) contacting the sample with an agent intended for specifically detecting the presence or absence of the polynucleotide or the polypeptide in the sample; and (b) detecting the presence or absence of the polynucleotide or the polypeptide.

For the purpose of the present invention, the term "sample" includes not only biological samples (for example, cells, tissues, organs, body fluids (e.g., blood, lymphs), digestive juices, sputum, bronchoalveolar/bronchial lavage fluids, urine, and feces), but also nucleic acid extracts from these biological samples (for example, genomic DNA extracts, mRNA extracts, and cDNA and cRNA preparations from mRNA extracts) and protein extracts. The sample may also be the one that is fixed with formalin or alcohol, frozen, or embedded in paraffin.

Further, the genomic DNA, mRNA, cDNA or protein can be prepared by those skilled in the art through considering various factors including the type and state of the sample and selecting a known technique suitable therefor.

In the present invention, the "detection of the presence or absence of the FGFR2 fusion polynucleotide or the FGFR2 fusion polypeptide" can be performed on genomic DNAs encoding said fusion polypeptide, transcripts from said genomic DNAs, or translation products from said transcripts.

Since a genomic DNA encoding the FGFR2-BICC1 fusion polypeptide is formed by an inversion in chromosome 10, the "detection of the presence or absence of the FGFR2-BICC1 fusion polynucleotide" may be achieved by detecting this phenomenon of inversion (refer to FIGs. 1 and 5). The detection of such an inversion may be achieved, for example, by detecting a split between the portion consisting of the exon 19-coding region of the FGFR2 gene and a region upstream from said coding region toward the 5' end, and the portion consisting of the exon 20-coding region of the FGFR2 gene and a region downstream from said coding region toward the 3' end, or by detecting a split between the portion consisting of the exon 2-coding region of the BICC1 gene and a region upstream from said coding region toward the 5' end, and the portion consisting of the exon 3-coding region of the BICC1 gene and a region downstream from said coding region toward the 3' end.

Since a genomic DNA encoding the FGFR2-AHCYL1 fusion polypeptide is formed by a reciprocal translocation between chromosome 1 and chromosome 10, the "detection of the presence or absence of the FGFR2-AHCYL1 fusion polynucleotide" may be achieved by detecting this phenomenon of reciprocal translocation (refer to FIGs. 3 and 6). The detection of such a reciprocal translocation may be achieved, for example, by detecting a split between the portion consisting of the exon 19-coding region of the FGFR2 gene and a region upstream from said coding region toward the 5' end, and the portion consisting of the exon 20-coding region of the FGFR2 gene and a region downstream from said coding region toward the 3' end, or by detecting a split between the portion consisting of the exon 1a-coding region of the AHCYL1 gene and a region upstream from said coding region toward the 5' end, and the portion consisting of the exon 5-coding region of the AHCYL1 gene and a region downstream from said coding region toward the 3' end.

The "detection of the presence of absence of the FGFR2 fusion polynucleotide" according to the present invention can be performed using a known method. Exemplary known methods that can be used in the detection on the "genomic DNAs encoding said fusion polypeptide" include *in situ* hybridization (ISH) using fluorescence or other means, genomic PCR, direct sequencing, Southern blotting, and genome microarray analysis. Exemplary known methods that can be used in the detection on the "transcripts from said genomic DNAs" include RT-PCR, direct sequencing, Northern blotting, dot blotting, and cDNA microarray analysis.

According to *in situ* hybridization, genomic DNAs encoding the FGFR2 fusion polypeptide can be detected by contacting a biological sample with the polynucleotide or polynucleotides noted below in (a) or (b), which have a chain length of at least 15 nucleotides:
(a) a polynucleotide or polynucleotides that are at least one probe selected from the group consisting of a probe that hybridizes to a polynucleotide encoding FGFR2 protein and a probe that hybridizes to a polynucleotide encoding other protein; or
(b) a polynucleotide that is a probe that hybridizes to a point of fusion between a polynucleotide encoding FGFR2 protein and a polynucleotide encoding other protein.

In relation to the detection of genomic DNAs encoding the FGFR2 fusion polypeptide, the "polynucleotide encoding FGFR2 protein" according to the present invention, as far as it is derived from humans, is typified by the gene consisting of the DNA sequence of positions 1 to 120129 in the genome sequence identified by RefSeq ID: NG_012449 ("FGFR2 gene").

The "polynucleotide encoding BICC1 protein" according to the present invention, as far as it is derived from humans, is typified by the gene consisting of the DNA sequence of positions 1 to 315942 in the genome sequence identified by RefSeq ID: NG_029759 ("BICC1 gene").

The "polynucleotide encoding AHCYL1 protein" according to the present invention, as far as it is derived from humans, is typified by the gene consisting of the DNA sequence of positions 1 to 38979 in the genome sequence identified by RefSeq ID: NG_029182 ("AHCYL1 gene").

However, the DNA sequences of the genes can vary in nature (i.e., in a non-artificial way) due to their mutations and the like. Thus, such naturally occurring mutants can also be encompassed by the present invention (the same applies hereinafter).

The polynucleotide(s) of (a) or (b) according to the present invention has a length of at least 15 nucleotides, preferably at least 20 nucleotides, more preferably from 100 to 1000 nucleotides.

The polynucleotide(s) of (a) according to the present invention can be of any type as far as it is capable of detecting the presence of a genomic DNA encoding the FGFR2 fusion polypeptide in the foregoing biological sample by hybridizing to a nucleotide sequence targeted by said polynucleotide, or more specifically to a polynucleotide encoding FGFR2 protein or a polynucleotide encoding other protein. The polynucleotide(s) of (a) is preferably any of the polynucleotides noted below in (a1) to (a5):
(a1) a combination of a polynucleotide that hybridizes to the portion consisting of the exon 19-coding region of the FGFR2 gene and a region upstream from said coding region toward the 5' end (this polynucleotide is to be hereinafter also referred to as "5' FGFR2 probe"), and a polynucleotide that hybridizes to the portion consisting of the exon 3-coding region of the BICC1 gene and a region downstream from said coding region toward the 3' end (this polynucleotide is to be hereinafter also referred to as "3' BICC1 probe");
(a2) a combination of 5' FGFR2 probe and a polynucleotide that hybridizes to the portion consisting of the exon 5-coding region of the AHCYL1 gene and a region downstream from said coding region toward the 3' end (this polynucleotide is to be hereinafter also referred to as "3' AHCYL1 probe");
(a3) a combination of 5' FGFR2 probe and a polynucleotide that hybridizes to the portion consisting of the exon 20-coding region of the FGFR2 gene and a region downstream from said coding region toward the 3' end (this polynucleotide is to be hereinafter also referred to as "3' FGFR2 probe");
(a4) a combination of a polynucleotide that hybridizes to the portion consisting of the exon 2-coding region of the BICC1 gene and a region upstream from said coding region toward the 5' end (this polynucleotide is to be hereinafter also referred to as "5' BICC1 probe"), and 3' BICC1 probe; and
(a5) a combination of a polynucleotide that hybridizes to the portion consisting of the exon 1a-coding region of the AHCYL1 gene and a region upstream from said coding region toward the 5' end (this polynucleotide is to be hereinafter also referred to as "5' AHCYL1 probe"), and 3' AHCYL1 probe.

In the present invention, it is preferred from the viewpoints of specificity for a target nucleotide sequence and detection sensitivity that the region to which the pair of polynucleotides of (a1) or (a2) as used for *in situ* hybridization is to hybridize (such a region is to be hereinafter referred to as the "target nucleotide sequence") should be a region extending for not more than 1000000 nucleotides from a point of fusion between a polynucleotide encoding FGFR2 protein and a polynucleotide encoding other protein. And it is preferred from the viewpoints of the same factors that the region to which the pair of polynucleotides of (a3), (a4) or (a5) as used *for in situ* hybridization is to hybridize should be a region extending for not more than 1000000 nucleotides from a breakpoint in a polynucleotide encoding FGFR2 protein, in a polynucleotide encoding BICC1 protein, or in a polynucleotide encoding AHCYL1 protein.

In the present invention, the polynucleotide of (b) as used *for in situ* hybridization can be of any type as far as it is capable of detecting the presence of a genomic DNA encoding the FGFR2 fusion polypeptide in the foregoing biological sample by hybridizing to a nucleotide sequence targeted by said polynucleotide, more specifically to a point of fusion between a polynucleotide encoding FGFR2 protein and a polynucleotide encoding other protein. Typical examples of the polynucleotide of (b) are those which each hybridize to a genomic DNA encoding a polynucleotide consisting of the nucleotide sequence of SEQ ID NO: 7 or 9, for example, those which each hybridize to a point of fusion between a polynucleotide encoding FGFR2 protein and a polynucleotide encoding other protein.

Further, in the present invention, it is preferred from the viewpoints of further improvement of specificity for a target nucleotide sequence and detection sensitivity that the polynucleotide or polynucleotides of (a) or (b) as used *for in situ* hybridization be a group consisting of multiple types of polynucleotides which can cover the entire target nucleotide sequence. In such a case, the polynucleotides constituting said group each have a length of at least 15 nucleotides, preferably at least 20 nucleotides, more preferably from 100 to 1000 nucleotides.

The polynucleotide or polynucleotides of (a) or (b) as used *for in situ* hybridization are preferably labeled for detection with a fluorescent dye or other means. Examples of such a fluorescent dye include, but are not limited to, DEAC, FITC, R6G, TexRed, and Cy5. Aside from fluorescent dyes, the polynucleotide may also be labeled with a dye (chromogen) such as DAB or with silver or other means based on enzymatic metal deposition.

In the process of *in situ* hybridization, a probe for a polynucleotide encoding FGFR2 protein and a probe for a polynucleotide encoding other protein are preferably each labeled with a different dye. If, as the result of *in situ* hybridization using the probe combination of (a1) or (a2) which consists of probes labeled with different dyes, an overlap is observed between signals emitted from labels on these probes, then it can be determined that a genomic DNA encoding the FGFR2 fusion polypeptide has been detected successfully. Also, if, as the result of *in situ* hybridization using the probe combination of (a3), (a4) or (a5) which consists of probes labeled with different dyes, a split is observed between signals emitted from labels on these probes, then it can be determined that a genomic DNA encoding the FGFR2 fusion polypeptide has been detected successfully.

Polynucleotide labeling can be effected by a known method. For example, the polynucleotides can be labeled by nick translation or random priming, in which the polynucleotides are caused to incorporate substrate nucleotides labeled with a fluorescent dye or other means.

The conditions for contacting the foregoing biological sample with the polynucleotide(s) of (a) or (b) in the process of *in situ* hybridization can vary with various factors including the length of said polynucleotide(s); and exemplary high stringent hybridization conditions are 0.2×SSC at 65°C, and exemplary low stringent hybridization conditions are 2.0×SSC at 50°C. Those skilled in the art could realize comparable stringent hybridization conditions to those mentioned above, by appropriately selecting salt concentration (e.g., SSC dilution rate), temperature, and various other conditions including concentrations of surfactant (e.g., NP-40) and formamide, and pH.

In addition to *in situ* hybridization, other examples of the method for detecting a genomic DNA encoding the FGFR2 fusion polypeptide using the polynucleotide(s) of (a) or (b) include Southern blotting, Northern blotting and dot blotting. According to these methods, the fusion gene is detected by hybridizing the polynucleotide(s) of (a) or (b) to a membrane in which a nucleic acid extract from the foregoing biological sample is transcribed. In the case of using the polynucleotide(s) of (a), if a polynucleotide that hybridizes to a polynucleotide encoding FGFR2 protein and a polynucleotide that hybridizes to a polynucleotide encoding other protein recognize the same band present in the membrane, then it can be determined that a genomic DNA encoding the FGFR2 fusion polypeptide has been detected successfully.

Additional examples of the method for detecting a genomic DNA encoding the FGFR2 fusion polypeptide using the polynucleotide of (b) include genome microarray analysis and DNA microarray analysis. According to these methods, the genomic DNA is detected by preparing an array in which the polynucleotide of (b) is immobilized on a substrate and bringing the foregoing biological sample into contact with the polynucleotide immobilized on the array.

In the process of PCR or sequencing, the polynucleotides noted below in (c) can be used to specifically amplify part or all of the FGFR2 fusion polynucleotide using a DNA (genomic DNA, cDNA) or RNA prepared from the foregoing biological sample as a template:
(c) polynucleotides that are a pair of primers designed to sandwich a point of fusion between a polynucleotide encoding FGFR2 protein and a polynucleotide encoding other protein.

The "polynucleotides that are a pair of primers" refers to a primer set designed such that in the foregoing fusion polynucleotide or the like to be targeted, one of the primers hybridizes to a region of the FGFR2 gene and the other primer hybridizes to a region of other gene. These polynucleotides have a length of generally 15-100 nucleotides, preferably 17-30 nucleotides.

Also, it is preferred from the viewpoints of the accuracy and sensitivity of PCR detection that the polynucleotides of (c) according to the present invention should each consist of a sequence complementary to the nucleotide sequence of said fusion polynucleotide which extends for not more than 5000 nucleotides from a point of fusion between a polynucleotide encoding FGFR2 protein and a polynucleotide encoding other protein.

The "polynucleotides that are a pair of primers" can be designed by a known method as appropriate based on the nucleotide sequence of the FGFR2 fusion polynucleotide or the like to be targeted. Exemplary known methods include a method using the Primer Express® software (ABI).

Preferred examples of the "polynucleotides that are a pair of primers" are preferably the polynucleotides noted below in (c1) and (c2):
(c1) a combination of a polynucleotide that hybridizes to the portion consisting of the exon 19-coding region of the FGFR2 gene and a region upstream from said coding region toward the 5' end (this polynucleotide is to be hereinafter also referred to as "5' FGFR2 primer"), and a polynucleotide that hybridizes to the portion consisting of the exon 3-coding region of the BICC1 gene and a region downstream from said coding region toward the 3' end (this polynucleotide is to be hereinafter also referred to as "3' BICC1 primer"); and
(c2) a combination of 5' FGFR2 primer and a polynucleotide that hybridizes to the portion consisting of the exon 5-coding region of the AHCYL1 gene and a region downstream from said coding region toward the 3' end (this polynucleotide is to be hereinafter also referred to as "3' AHCYL1 primer").

For example, in the process of detection of the FGFR2-BICC1 fusion polynucleotide, primers are each designed for exon 16 or 18 in the kinase region of the FGFR2 gene, or for exon 7/8 or 21 in the BICC1 gene, so that detection can be made of all variants containing the FGFR2 kinase region, or more specifically all fusion genes in which the BICC1 gene is fused with each of the FGFR2 gene isoforms 1-9 which are different from each other in terms of extra-membranous domain structure (e.g., the presence or absence of IgI) (refer to FIG. 2). In the process of detection of the FGFR2-AHCYL1 fusion polynucleotide, primers are each designed for exon 16 or 18 in the kinase region of the FGFR2 gene, or for exon 9 or 20 in the AHCYL1 gene, so that detection can be made of all variants containing the FGFR2 kinase region, or more specifically all fusion genes in which each of the FGFR2 gene isoforms 1-9 which are different from each other in terms of extra-membranous domain structure is fused with each of the AHCYL1 gene isoforms a and b which are different from each other in the length of the N-terminal moiety (refer to FIG. 4).

In the present invention, the method for detecting a translation product of the FGFR2 fusion polynucleotide can be exemplified by immunostaining, Western blotting, ELISA, flow cytometry, immunoprecipitation, and antibody array analysis. These methods use an antibody binding to the FGFR2 fusion polypeptide. Examples of such an antibody include an antibody specific to a polypeptide containing a point of fusion between FGFR2 protein and other protein (hereinafter also referred to as the "fusion point-specific antibody"), an antibody binding to a polypeptide consisting of that region of FGFR2 protein which extends toward the N-terminus with respect to said point of fusion (hereinafter also referred to as the "FGFR2-N terminal antibody"), and an antibody binding to a polypeptide consisting of that region of other protein which extends toward the C-terminus with respect to said point of fusion (hereinafter also referred to as the "other protein-C terminal antibody"). As referred to herein, the "fusion point-specific antibody" means an antibody that specifically binds to a polypeptide containing said point of fusion but does not bind to either wild-type (normal) FGFR2 protein or other wild-type (normal) protein.

The FGFR2 fusion polypeptide can be detected by the fusion point-specific antibody or a combination of the FGFR2-N terminal antibody and the other protein-C terminal antibody.

The "antibody binding to the FGFR2 fusion polypeptide" can be prepared by those skilled in the art through selection of a known method as appropriate. Examples of such a known method include: a method in which the polypeptide comprising the C-terminal moiety of other protein, the FGFR2 fusion polypeptide, the polypeptide comprising the N-terminal moiety of FGFR2 protein, and/or the like are inoculated into immune animals, the immune systems of the animals are activated, and then the serums (polyclonal antibodies) of the animals are collected; as well as monoclonal antibody preparation methods such as hybridoma method, recombinant DNA method, and phage display method. If an antibody having a labeling agent attached thereto is used, a target protein can be directly detected by detecting this label. The labeling agent is not particularly limited as long as it is capable of binding to an antibody and is detectable, and examples include peroxidase, β-D-galactosidase, microperoxidase, horseradish peroxidase (HRP), fluorescein isothiocyanate (FITC), rhodamine isothiocyanate (RITC), alkaline phosphatase, biotin, and radioactive materials. In addition to the direct detection of a target protein using an antibody having a labeling agent attached thereto, the target protein can also be indirectly detected using a secondary antibody having a labeling agent attached thereto, Protein G or A, or the like.

### <Method for determining the effectiveness of cancer treatments with an FGFR2 inhibitor>

As disclosed below in Examples, gene fusions between the FGFR2 gene and other gene are believed to induce activation of FGFR2 protein and hence contribute to malignant transformation of cancers and other pathological conditions. Thus, it is highly probable that cancer patients with detection of such a fusion are responsive to treatments with an FGFR2 inhibitor.

Therefore, the present invention provides a method for determining the effectiveness of a cancer treatment with an FGFR2 inhibitor, the method comprising the step of detecting the presence or absence of the FGFR2 fusion polynucleotide or the FGFR2 fusion polypeptide in a sample isolated from a patient, wherein in a case where the presence of the polynucleotide or polypeptide is detected, the cancer treatment with the FGFR2 inhibitor is determined to be highly effective in the patient.

For the purpose of the present invention, the "patient" can be not only a human suffering from a cancer but also a human suspected of having a cancer. The "cancer" to which the method of this invention is to be applied is not particularly limited as long as it is a cancer with expression of an FGFR2 fusion gene. The cancer is preferably a biliary tract cancer. Collection of a biological sample from the patient can be performed by a known method depending on the type of the biological sample.

For the purpose of the present invention, the "FGFR2 inhibitor", the cancer treatment with which is to be evaluated for effectiveness, is not particularly limited as long as it is a substance capable of directly or indirectly suppressing the function of FGFR2 protein. Examples of known FGFR2 inhibitors that can be applied to the present invention include: 3-[2,4-dimethy-5-[[(Z)-2,3-dihydro-2-oxo-1H-indol-3-ylidene]methyl]-1H-pyrrol-3-yl]propanoic acid (e.g., SU6668 (generic name: orantinib)); N-{5-[2-(3,5-dimethoxyphenyl)ethyl]-1H-pyrazol-3-yl}-4-[(3R,5S)-3,5-dimethylpiperazin-1-yl]benzamide (e.g., AZD4547); 1-[(2R,4S,5S)-4-azido-5-(hydroxymethyl)oxolan-2-yl]-5-methylpyrimidine-2,4-dione (e.g., DS4152 (generic name: tecogalan sodium); FGFR2-IIIb-specific antibody (e.g., AV369b); 3-(2,6-dichloro-3,5-dimethoxyphenyl)-1-[6-[[4-(4-ethylpiperazin-1-yl)phenyl]amino]pyrimidin-4-yl]-1-methylurea (e.g., BGJ398); (S)-(R)-1-((4-((4-fluoro-2-methyl-1H-indo1-5-yl)oxy)-5-methylpyrrolo[2,1-f][1,2,4]triazin-6-yl)oxy)propan-2-yl 2-aminopropionate (e.g., BMS-582664 (generic name: brivanib alaninate); and N-[2-[[4-(diethylamino)butyl]amino]-6-(3,5-dimethoxyphenyl)pyrido[2,3-d]pyrimidin-7-yl]-N'-(1,1-dimethylethyl)-urea (e.g., PD173074).

The definition of the term "sample", the method for extracting a DNA, an RNA or the like from the sample, the procedure for detecting the presence or absence of the FGFR2 fusion polynucleotide or the FGFR2 fusion polypeptide, and other related information are as described above.

If the presence of the FGFR2 fusion polynucleotide or the FGFR2 fusion polypeptide in a sample isolated from a patient is detected according to the inventive method, the patient will be determined to be highly responsive to a cancer treatment with an FGFR2 inhibitor. If the presence of the polynucleotide or polypeptide is not detected, the patient will be determined to be less responsive to a cancer treatment with an FGFR2 inhibitor.

### <Agents for detecting the presence or absence of the FGFR2 fusion polynucleotide or the FGFR2 fusion polypeptide, and agents for determining the effectiveness of cancer treatments with an FGFR2 inhibitor>

As described above, the polynucleotides noted below in (a) to (c), which each have a chain length of at least 15 nucleotides, can be used advantageously for detecting the presence or absence of the FGFR2 fusion polynucleotide. Thus, said polynucleotides can also be used advantageously for determining the effectiveness of cancer treatments with an FGFR2 inhibitor.
(a) A polynucleotide or polynucleotides that are at least one probe selected from the group consisting of a probe that hybridizes to a polynucleotide encoding FGFR2 protein and a probe that hybridizes to a polynucleotide encoding other protein;
(b) a polynucleotide that is a probe that hybridizes to a point of fusion between a polynucleotide encoding FGFR2 protein and a polynucleotide encoding other protein; and
(c) polynucleotides that are a pair of primers designed to sandwich a point of fusion between a polynucleotide encoding FGFR2 protein and a polynucleotide encoding other protein.

Said polynucleotides each have a nucleotide sequence complementary to a particular nucleotide sequence of a target gene. As referred to herein, the term "complementary" may not necessarily refer to perfect complementarity as long as hybridization is achieved. Said polynucleotides have generally at least 80% homology, preferably at least 90% homology, more preferably at least 95% homology, and particularly preferably at least 100% homology with such a particular nucleotide sequence.

The polynucleotides of (a) to (c) may be a DNA or a RNA, or may be such that part or all of the nucleotides are substituted by an artificial nucleic acid such as PNA (polyamide nucleic acid: a peptide nucleic acid), LNA^{™} (Locked Nucleic Acid; a bridged nucleic acid), ENA® (2'-O,4'-C-Ethylene-bridged Nucleic Acid), GNA (glycerol nucleic acid) or TNA (threose nucleic acid).

As described above, the antibody binding to an FGFR2 fusion polypeptide can be used advantageously for detecting translation products (FGFR2 fusion polypeptides) of the FGFR2 fusion polypeptide.

The agents of the present invention can contain not only the foregoing substance (e.g., polynucleotide, antibody) as an active ingredient but also other pharmacologically acceptable components. Such other components include buffer agents, emulsifying agents, suspending agents, stabilizing agents, antiseptic agents, and physiological saline. As buffer agents, there can be used phosphates, citrates, acetates and the like. As emulsifying agents, there can be used gum arabic, sodium alginate, tragacanth, and the like. As suspending agents, there can be used glyceryl monostearate, aluminum monostearate, methylcellulose, carboxymethyl cellulose, hydroxymethyl cellulose, sodium lauryl sulfate, and the like. As stabilizing agents, there can be used propylene glycol, diethylene sulfite, ascorbic acid, and the like. As antiseptic agents, there can be used sodium azide, benzalkonium chloride, paraoxybenzoic acid, chlorobutanol, and the like.

A specimen containing the inventive polynucleotide or antibody may also be combined with other specimens such as a substrate required for detecting a label attached to the polynucleotide or the antibody, a positive control (e.g., FGFR2 fusion polynucleotide, FGFR2 fusion polypeptide, or cells bearing the same), a negative control, a counterstaining reagent for use for *in situ* hybridization or the like (e.g., DAPI), a molecule required for antibody detection (e.g., secondary antibody, Protein G, Protein A), and a buffer solution for use in sample dilution or washing, so that a kit for use in the method of the present invention can be provided. The inventive kit can contain instructions for use thereof. Therefore, the present invention also provides the foregoing kit for use in the inventive method.

### <Method for treatment of cancer, and therapeutic agents for cancer>

As described above, if the presence of the FGFR2 fusion polynucleotide or the FGFR2 fusion polypeptide is detected in a patient by the method of the present invention, the patient is considered to be highly responsive to a cancer treatment with an FGFR2 inhibitor. Thus, efficient cancer treatment is possible by administering an FGFR2 inhibitor selectively to those cancer patients who carry the FGFR2 fusion gene. Therefore, this invention provides a method for treatment of cancer, comprising the step of administering an FGFR2 inhibitor to a patient in whom a cancer treatment with the FGFR2 inhibitor has been determined to be highly effective according to the foregoing determination method of this invention.

Further, the present invention provides a therapeutic agent for cancer, comprising an FGFR2 inhibitor as an active ingredient, the agent which is to be administered to a patient in whom a cancer treatment with the FGFR2 inhibitor has been determined to be highly effective according to the foregoing determination method of this invention.

As described above, the "FGFR2 inhibitor" is not particularly limited as long as it is a substance capable of directly or indirectly suppressing the function of FGFR2 protein. Examples of known FGFR2 inhibitors that can be applied to the present invention are as given above.

The dosage form for administering an FGFR2 inhibitor to a patient is selected as appropriate depending on various factors including the type of the inhibitor and the type of cancer, and examples of the dosage form that can be adopted include oral, intravenous, intraperitoneal, transdermal, intramuscular, intratracheal (aerosol), rectal, intravaginal and other administrations.

### EXAMPLES

On the pages that follow, the present invention will be more specifically described on the basis of Examples, but this invention is not limited to the examples given below.

### <Test samples>

Eight surgically resected and frozen specimens of KRAS/BRAF mutation-negative biliary tract cancer (tumor tissues) were used as an object to be tested. As a negative control, normal tissues (liver tissues containing biliary epithelium) were also treated and analyzed by the methods described below as in the case of the tumor tissues.

### <RNA extraction>

The tumor tissues cryopreserved in liquid nitrogen were pulverized with cryoPREP (product name: CP02; Covaris). Then, total RNA was extracted from the pulverized tumor tissues using a total RNA purification kit (product name: RNAeasy; QIAGEN).

### <RNA sequencing>

Library synthesis was performed with an mRNAseq sample preparation kit (Illumina) using 2 µg of the total RNA prepared hereinabove. More specifically, 2 µg of the total RNA was fragmented by treatment at 94°C for 5 minutes and subjected to cDNA synthesis. Next, a sequencing adapter was ligated to each end of the resulting cDNA, which was then subjected to electrophoresis on agarose gel and purified. PCR was performed using the purified cDNA as a template to construct a 300 bp cDNA library. The sequences of 50 bp from both ends of the constructed cDNA library were sequenced using a high-throughput sequencer (GA2X; Illumina).

### <Analysis of sequence information>

The obtained sequence information, from which overlapping clones generated by PCR were excluded, was mapped to known databases (Refseq and Ensemble) using the Bowtie software to extract clones whose end sequences are derived from different genes (refer to Kohno T., et al., Nature Med., 2012, vol. 18, p. 375-377).

### <Verification by RT-PCR and Sanger method>

The same total RNA as used in RNA sequencing was subjected to cDNA synthesis anew using a reverse transcriptase (SuperScript III First-Strand Synthesis System; Invitrogen). PCR primers were synthesized based on the resulting sequences. PCR was performed using ExTaq HS (Takara), and then the PCR products were verified by electrophoresis. Further, the PCR products were extracted from the agarose gel and sequenced by the Sanger method using the same primers, the BigDye Terminator v3.1 Cycle Sequencing Kit (Applied Biosystems) and the ABI 3730 Sequencer. The obtained results were used to identify points of fusion of fusion genes and reading frames. The conditions for reverse transcription reaction and PCR are as follows.

### <Reverse transcription reaction>

First, 5 µg (8 µL) of the total RNA was mixed with 1 µL of Random Hexamer Primer (50 ng/µL) and 1 µL of 10mM dNTP Mix, and the mixture was reacted at 65°C for 5 minutes and quenched on ice. Next, 2 µL of 10×RT buffer, 4 µL of 25 mM MgCl₂, 2 µL of 0.1M DTT, 1 µL of RNaseOUT (40 U/µL), and 1 µL of SuperScript III RT (200 U/µL) were added in this order, and reverse transcription reaction was effected under the following conditions: 25°C for 10 minutes, 50°C for 50 minutes, 85°C for 5 minutes, and 4°C for 5 minutes. To the resulting reverse transcripts, 1 µL of RNaseH was added to digest the total RNA at 37°C over 20 minutes. The completed reverse transcription reactions were stored at -20°C until use.

### <PCR>

First, 2 µL of the hereinabove prepared 1st strand cDNA, 1 µL of 10×ExTaq buffer, 1.2 µL of 2.5 mM dNTPs, 4.7 µL of H₂O, 0.1 µL of ExTaq HS, and 1 µL of 2 µM CF/CR primer pair were mixed, and the mixture was subjected to PCR under the following conditions: the reaction started with 95°C for 3 minutes, followed by 35 cycles consisting of 94°C for 30 seconds, 58°C for 30 seconds, and 72°C for 30 seconds, and ended with 72°C for 5 minutes.

### (Example 1)

### [Identification of novel kinase fusion genes by RNA sequencing]

From each of 8 KRAS/BRAF-negative BTC clinical specimens, there were obtained at least 5.2 × 10⁷ paired nucleotide sequences, with overlapping clones generated by PCR being excluded. As the result of comparison of these sequences to existing gene databases, the following two fusion gene candidates as shown in FIGs. 2 and 4 were detected each in one specimen: a fusion gene between the FGFR2 gene and the BICC1 gene (hereinafter also referred to the "FGFR2-BICC1 fusion gene"), and a fusion gene between the FGFR2 gene and the AHCYL1 gene (hereinafter also referred to as the "FGFR2-AHCYL1 fusion gene").

### [Verification by RT-PCR and Sanger sequencing]

Next, the same specimens were verified for fusion genes by RT-PCR and Sanger sequencing. As a result, RT-PCR revealed that specimen-specific amplification is observed for both of the two fusion genes, or in other words that these fusion genes are expressed only in BTC tissues and not in normal tissues (liver tissues containing biliary epithelium).

Further, sequencing of the obtained PCR products revealed that in both of these fusion genes, two genes are fused together without inconsistency in their reading frames.

More specifically, it was found that in the fusion gene between the FGFR2 gene and the BICC1 gene, as shown in FIG. 2 and SEQ ID NO: 7, exon 19 of the FGFR2 gene (isoform 1) (i.e., the polynucleotide consisting of the nucleotide sequence of positions 2843 to 2948 in SEQ ID NO: 1 or 7) was directly bound and fused to exon 3 of the BICC1 gene (i.e., the polynucleotide consisting of the nucleotide sequence of positions 238 to 307 in SEQ ID NO: 3) without inconsistency in the reading frames of these genes.

It was also found that in the fusion gene between the FGFR2 gene and the AHCYL1 gene, as shown in FIG. 4 and SEQ ID NO: 9, exon 19 of the FGFR2 gene (isoform 1) (i.e., the polynucleotide consisting of the nucleotide sequence of positions 2843 to 2948 in SEQ ID NO: 1 or 7) was directly bound and fused to exon 5 of the AHCYL1 gene (isoform a) (i.e., the polynucleotide consisting of the nucleotide sequence of positions 410 to 521 in SEQ ID NO: 5) without inconsistency in the reading frames of these genes.

Accordingly, the results presented hereinabove show that the inversion in chromosome 10 and the reciprocal translocation between chromosome 1 and chromosome 10, which are specific to biliary tract and other cancer cells, result in a fusion between the FGFR2 gene and the BICC1 gene, and a fusion between the FGFR2 gene and the AHCYL1 gene, respectively.

### (Example 2)

### [Analysis of the appearance frequencies of the FGFR2 fusion genes]

The hereinabove-identified cancer cell-specific fusion genes, FGFR2-AHCYL1 and FR2-BICC1 fusion genes, were each analyzed for their appearance frequencies. More specifically, 102 cancer tissues derived from biliary tract cancer (BTC) patients were subjected to RT-PCR analysis of the appearance frequencies of the respective fusion genes.

As a result, among the 102 BTC specimens, the FGFR2/AHCYL1 fusion was observed in 7 specimens (appearance frequency: about 6.9%), and the FGFR2/BICC1 fusion was observed in 2 specimens (appearance frequency: about 2.0%).

### (Example 3)

### [Analyses of the function of the FGFR2 fusion polypeptides, and of the effectiveness of FGFR inhibitors against cancer cells expressing said polypeptides]

It is considered that the above-mentioned gene fusions induce activation of FGFR2 protein, and that this activation induces activation of a downstream signal, thereby causing canceration of cells. Therefore, it is conceivable that FGFR2 inhibitors may be therapeutically effective in patients with such activations. In order to verify these points, analysis of the FGFR2 fusion genes was made by following appropriate conventional methods, as described below.

First, a cDNA encoding a FLAG epitope tag was joined to the 5' end of each of the cDNAs of the FGFR2 fusion genes obtained from BCT patients' cancer tissues (FGFR2-AHCYL1 fusion gene cDNA or FGFR2-BICC1 fusion gene cDNA), by aligning their translated reading frames with each other. The resulting products were each cloned into the pMXs retroviral vector.

Also, site-directed mutagenesis was performed on the vectors to construct vectors encoding a kinase activity mutant containing a substitution of 2 amino acids in an FGFR2 kinase region (KD-mutated FGFR2 fusion polypeptides: Y568F/Y569F).

Next, normal murine fibroblast line NIH-3T3 cells were infected with each of the thus-prepared retroviral vectors to obtain cell lines stably expressing the wild-type or the mutated FGFR2 fusion polypeptide.

The same procedures were performed for a tyrosine kinase fusion gene detected in lung cancer (fusion gene between the EZR gene and the ROS1 gene; hereinafter also referred to as the "EZR-ROS1 fusion gene") -- a retroviral vector encoding this gene was constructed, and NIH-3T3 cells were infected with this vector to prepare a cell line stably expressing the EZR-ROS1 fusion polypeptide. This cell line was subjected to the tests described below as a control group.

The cell lines stably expressing the wild-type or the mutated FGFR2 fusion polypeptide were each seeded in a soft agar medium (at an agar concentration in medium of 4 mg/mL; the same applies hereunder) and evaluated for their anchorage-independent colony-forming ability to thereby investigate the transforming ability of the FGFR2 fusion polypeptides. The results are shown in FIGs. 7 and 8.

Further, the cell lines stably expressing the wild-type FGFR2 fusion polypeptides and the cell line stably expressing the EZR-ROS1 fusion polypeptide were each seeded in a soft agar medium supplemented with a low-molecular-weight FGFR kinase inhibitor (BGJ398 or PD173074) and evaluated for their anchorage-independent colony-forming ability to thereby investigate the effect of FGFR kinase inhibitors against transformation induced by the FGFR2 fusion polypeptides. The results are shown in FIG. 9.

In addition, the cell line stably expressing the wild-type FGFR2-AHCYL1 fusion polypeptide and the cell line stably expressing the EZR-ROS1 fusion polypeptide were each cultured in a liquid medium, subjected to serum starvation, and then cultured again with the medium being replaced with the one supplemented with an FGFR kinase inhibitor (BGJ398 or PD173074). Proteins from each of the thus-obtained cell lines were extracted and subjected to Western blotting analysis using antibodies against phosphorylated or unphosphorylated proteins to thereby analyze the downstream signals of the FGFR2 fusion polypeptide. The results are shown in FIG. 10.

Furthermore, the cell lines stably expressing the wild-type or the mutated FGFR2 fusion polypeptide were each subcutaneously transplanted into immunodeficient mice (BALB/c-nu/nu) at a dose of 1 × 10⁶ cells per spot to thereby investigate the *in vivo* tumorigenic ability of the cells expressing these FGFR2 fusion polypeptides. The results are shown in FIG. 11.

As is evident from the results shown in FIGs. 7 and 8, NIH-3T3 cells showed anchorage-independent colony formation due to the expression of the FGFR2 fusion polypeptides. On the other hand, it was found that the anchorage-independent colony-forming ability of the FGFR2 fusion polypeptides is significantly suppressed by inactivating the kinase activity of FGFR2 protein.

It was also found as shown in FIG. 9 that the anchorage-independent colony-forming ability of the FGFR2 fusion polypeptides is significantly suppressed by using an FGFR kinase inhibitor.

As is evident from the results given in FIG. 10, NIH-3T3 cells showed strong phosphorylation of the FGFR kinase domain due to the expression of the FGFR2 fusion polypeptide (refer to the 5th lane from the right in the "p-FGFR (Y653/Y654)" row of FIG. 10). It was also found that this phosphorylation is significantly suppressed by treatment with an FGFR kinase inhibitor (refer to the 4th to 1st lanes from the right in the "p-FGFR (Y653/Y654)" row of FIG. 10).

As shown in FIG. 10, the phosphorylation of MAPK was also strongly induced by the FGFR2 fusion polypeptide. However, it was found that this phosphorylation is also significantly suppressed by treatment with an FGFR kinase inhibitor (refer to the 5th lane v.s. the 4th to 1st lanes from the right in the "p-MAPK (T202/Y204)" row of FIG. 10). Additionally, the phosphorylations of AKT and STAT3 were not increased by the expression of the FGFR2 fusion polypeptide.

As shown in FIG. 11, tumorigenesis was observed within 14 days after NIH-3T3 cells expressing the wild-type FGFR2 fusion polypeptide were subcutaneously transplanted into immunodeficient mice. On the other hand, NIH-3T3 cells expressing the mutated FGFR2 fusion polypeptide were also subcutaneously transplanted into immunodeficient mice, but no tumorigenesis was observed at all for 30 days after the transplantation.

Accordingly, these findings demonstrated that the FGFR2 fusion genes serve as an oncogene with transforming ability, and that their transforming ability requires the activation of the FGFR2 kinase activity.

It is also found that the transforming ability of said FGFR2 fusion polypeptides is suppressed using an FGFR kinase inhibitor by inhibiting the activations of the FGFR2 kinase in said fusion polypeptides and its downstream signal, MAPK.

### INDUSTRIAL APPLICABILITY

As described above, the present invention enables detection of polynucleotides encoding fusion polypeptides between FGFR2 protein and other protein, and expression products thereof, and also this detection makes it possible to predict the effectiveness of cancer treatments with an FGFR2 inhibitor. As described above, an in-frame fusion between the FGFR2 gene and other gene was found in multiple cases, and this fusion induces the activation of FGFR2, and in turn causes canceration of cells. Further, as demonstrated above, said FGFR2 activation and canceration can be significantly suppressed by using an FGFR2 inhibitor. Therefore, since such fusions between the FGFR2 gene and other gene can be targeted by FGFR2 inhibitors, the present invention is very useful in improving the efficiency of cancer treatments.

## Claims

1. A polynucleotide encoding a polypeptide in which FGFR2 protein and other protein are fused together, wherein the polypeptide is expressed in a cancer cell.

2. The polynucleotide according to claim 1, wherein said other protein is BICC1 protein or AHCYL1 protein.

3. The polynucleotide according to claim 1 or 2, wherein the cancer cell is a biliary tract cancer cell.

4. A polypeptide encoded by the polynucleotide according to any one of claims 1 to 3.

5. A method for detecting the presence or absence in a sample of the polynucleotide according to any one of claims 1 to 3 or of the polypeptide according to claim 4, the method comprising the steps of:
(a) contacting the sample with an agent intended for specifically detecting the presence or absence of the polynucleotide or the polypeptide in the sample; and
(b) detecting the presence or absence of the polynucleotide or the polypeptide.

6. An agent for detecting the presence or absence in a sample of the polynucleotide according to any one of claims 1 to 3 or of the polypeptide according to claim 4 by the method according to claim 5, the agent comprising a polynucleotide or polynucleotides as set forth below in any one of (a) to (c), the polynucleotide or polynucleotides having a chain length of at least 15 nucleotides, or an antibody as set forth below in (d):
(a) a polynucleotide or polynucleotides that are at least one probe selected from the group consisting of a probe that hybridizes to a polynucleotide encoding FGFR2 protein and a probe that hybridizes to a polynucleotide encoding other protein;
(b) a polynucleotide that is a probe that hybridizes to a point of fusion between a polynucleotide encoding FGFR2 protein and a polynucleotide encoding other protein;
(c) polynucleotides that are a pair of primers designed to sandwich a point of fusion between a polynucleotide encoding FGFR2 protein and a polynucleotide encoding other protein; and
(d) an antibody that binds to a polypeptide in which FGFR2 protein and other protein are fused together.

7. A method for determining the effectiveness of a cancer treatment with an FGFR2 inhibitor, the method comprising the step of detecting the presence or absence in a sample isolated from a patient of the polynucleotide according to any one of claims 1 to 3 or of the polypeptide according to claim 4, wherein in a case where the presence of the polynucleotide or the polypeptide is detected, the cancer treatment with the FGFR2 inhibitor is determined to be highly effective in the patient.

8. An agent for determining the effectiveness of a cancer treatment with an FGFR2 inhibitor by the method according to claim 7, the agent comprising a polynucleotide or polynucleotides as set forth below in any one of (a) to (c), the polynucleotide or polynucleotides having a chain length of at least 15 nucleotides, or an antibody as set forth below in (d):
(a) a polynucleotide or polynucleotides that are at least one probe selected from the group consisting of a probe that hybridizes to a polynucleotide encoding FGFR2 protein and a probe that hybridizes to a polynucleotide encoding other protein;
(b) a polynucleotide that is a probe that hybridizes to a point of fusion between a polynucleotide encoding FGFR2 protein and a polynucleotide encoding other protein;
(c) polynucleotides that are a pair of primers designed to sandwich a point of fusion between a polynucleotide encoding FGFR2 protein and a polynucleotide encoding other protein; and
(d) an antibody that binds to a polypeptide in which FGFR2 protein and other protein are fused together.

9. A method for treatment of cancer, comprising the step of administering an FGFR2 inhibitor to a patient in whom a cancer treatment with the FGFR2 inhibitor has been determined to be highly effective by the method according to claim 7.

10. A therapeutic agent for cancer, comprising an FGFR2 inhibitor as an active ingredient, the agent which is to be administered to a patient in whom a cancer treatment with the FGFR2 inhibitor has been determined to be highly effective by the method according to claim 7.


SEQUENCE LISTING
<110>  NATIONAL CANCER CENTER
<120>  FGFR2 FUSION GENE
<130>  IBPF13-537WO
<150>  JP2012/151352
<151>  2012-07-05
<160>  10
<170>  PatentIn version 3.1

<210>  1
<211>  4654
<212>  DNA
<213>  Homo sapiens
<220>
<221>  CDS
<222>  (648)..(3113)
<223>
<400>  1
ggcggcggct ggaggagagc gcggtggaga gccgagcggg cgggcggcgg gtgcggagcg      60
ggcgagggag cgcgcgcggc cgccacaaag ctcgggcgcc gcggggctgc atgcggcgta     120
cctggcccgg cgcggcgact gctctccggg ctggcggggg ccggccgcga gccccggggg     180
ccccgaggcc gcagcttgcc tgcgcgctct gagccttcgc aactcgcgag caaagtttgg     240
tggaggcaac gccaagcctg agtcctttct tcctctcgtt ccccaaatcc gagggcagcc     300
cgcgggcgtc atgcccgcgc tcctccgcag cctggggtac gcgtgaagcc cgggaggctt     360
ggcgccggcg aagacccaag gaccactctt ctgcgtttgg agttgctccc cgcaaccccg     420
ggctcgtcgc tttctccatc ccgacccacg cggggcgcgg ggacaacaca ggtcgcggag     480
gagcgttgcc attcaagtga ctgcagcagc agcggcagcg cctcggttcc tgagcccacc     540
gcaggctgaa ggcattgcgc gtagtccatg cccgtagagg aagtgtgcag atgggattaa     600
cgtccacatg gagatatgga agaggaccgg ggattggtac cgtaacc atg gtc agc      656
tgg ggt cgt ttc atc tgc ctg gtc gtg gtc acc atg gca acc ttg tcc      704
ctg gcc cgg ccc tcc ttc agt tta gtt gag gat acc aca tta gag cca      752
gaa gag cca cca acc aaa tac caa atc tct caa cca gaa gtg tac gtg      800
gct gcg cca ggg gag tcg cta gag gtg cgc tgc ctg ttg aaa gat gcc      848
gcc gtg atc agt tgg act aag gat ggg gtg cac ttg ggg ccc aac aat      896
agg aca gtg ctt att ggg gag tac ttg cag ata aag ggc gcc acg cct      944
aga gac tcc ggc ctc tat gct tgt act gcc agt agg act gta gac agt      992
gaa act tgg tac ttc atg gtg aat gtc aca gat gcc atc tca tcc gga     1040
gat gat gag gat gac acc gat ggt gcg gaa gat ttt gtc agt gag aac     1088
agt aac aac aag aga gca cca tac tgg acc aac aca gaa aag atg gaa     1136
aag cgg ctc cat gct gtg cct gcg gcc aac act gtc aag ttt cgc tgc     1184
cca gcc ggg ggg aac cca atg cca acc atg cgg tgg ctg aaa aac ggg      1232
aag gag ttt aag cag gag cat cgc att gga ggc tac aag gta cga aac      1280
cag cac tgg agc ctc att atg gaa agt gtg gtc cca tct gac aag gga      1328
aat tat acc tgt gta gtg gag aat gaa tac ggg tcc atc aat cac acg      1376
tac cac ctg gat gtt gtg gag cga tcg cct cac cgg ccc atc ctc caa      1424
gcc gga ctg ccg gca aat gcc tcc aca gtg gtc gga gga gac gta gag      1472
ttt gtc tgc aag gtt tac agt gat gcc cag ccc cac atc cag tgg atc      1520
aag cac gtg gaa aag aac ggc agt aaa tac ggg ccc gac ggg ctg ccc      1568
tac ctc aag gtt ctc aag gcc gcc ggt gtt aac acc acg gac aaa gag      1616
att gag gtt ctc tat att cgg aat gta act ttt gag gac gct ggg gaa      1664
tat acg tgc ttg gcg ggt aat tct att ggg ata tcc ttt cac tct gca      1712
tgg ttg aca gtt ctg cca gcg cct gga aga gaa aag gag att aca gct      1760
tcc cca gac tac ctg gag ata gcc att tac tgc ata ggg gtc ttc tta      1808
atc gcc tgt atg gtg gta aca gtc atc ctg tgc cga atg aag aac acg      1856
acc aag aag cca gac ttc agc agc cag ccg gct gtg cac aag ctg acc      1904
aaa cgt atc ccc ctg cgg aga cag gta aca gtt tcg gct gag tcc agc      1952
tcc tcc atg aac tcc aac acc ccg ctg gtg agg ata aca aca cgc ctc      2000
tct tca acg gca gac acc ccc atg ctg gca ggg gtc tcc gag tat gaa      2048
ctt cca gag gac cca aaa tgg gag ttt cca aga gat aag ctg aca ctg      2096
ggc aag ccc ctg gga gaa ggt tgc ttt ggg caa gtg gtc atg gcg gaa      2144
gca gtg gga att gac aaa gac aag ccc aag gag gcg gtc acc gtg gcc      2192
gtg aag atg ttg aaa gat gat gcc aca gag aaa gac ctt tct gat ctg      2240
gtg tca gag atg gag atg atg aag atg att ggg aaa cac aag aat atc      2288
ata aat ctt ctt gga gcc tgc aca cag gat ggg cct ctc tat gtc ata      2336
gtt gag tat gcc tct aaa ggc aac ctc cga gaa tac ctc cga gcc cgg      2384
agg cca ccc ggg atg gag tac tcc tat gac att aac cgt gtt cct gag      2432
gag cag atg acc ttc aag gac ttg gtg tca tgc acc tac cag ctg gcc      2480
aga ggc atg gag tac ttg gct tcc caa aaa tgt att cat cga gat tta      2528
gca gcc aga aat gtt ttg gta aca gaa aac aat gtg atg aaa ata gca      2576
gac ttt gga ctc gcc aga gat atc aac aat ata gac tat tac aaa aag      2624
acc acc aat ggg cgg ctt cca gtc aag tgg atg gct cca gaa gcc ctg      2672
ttt gat aga gta tac act cat cag agt gat gtc tgg tcc ttc ggg gtg      2720
tta atg tgg gag atc ttc act tta ggg ggc tcg ccc tac cca ggg att      2768
ccc gtg gag gaa ctt ttt aag ctg ctg aag gaa gga cac aga atg gat      2816
aag cca gcc aac tgc acc aac gaa ctg tac atg atg atg agg gac tgt      2864
tgg cat gca gtg ccc tcc cag aga cca acg ttc aag cag ttg gta gaa      2912
gac ttg gat cga att ctc act ctc aca acc aat gag gaa tac ttg gac      2960
ctc agc caa cct ctc gaa cag tat tca cct agt tac cct gac aca aga      3008
agt tct tgt tct tca gga gat gat tct gtt ttt tct cca gac ccc atg      3056
cct tac gaa cca tgc ctt cct cag tat cca cac ata aac ggc agt gtt      3104
aaa aca tga atgactgtgt ctgcctgtcc ccaaacagga cagcactggg              3153
aacctagcta cactgagcag ggagaccatg cctcccagag cttgttgtct ccacttgtat    3213
atatggatca gaggagtaaa taattggaaa agtaatcagc atatgtgtaa agatttatac    3273
agttgaaaac ttgtaatctt ccccaggagg agaagaaggt ttctggagca gtggactgcc 3333
acaagccacc atgtaacccc tctcacctgc cgtgcgtact ggctgtggac cagtaggact 3393
caaggtggac gtgcgttctg ccttccttgt taattttgta ataattggag aagatttatg 3453
tcagcacaca cttacagagc acaaatgcag tatataggtg ctggatgtat gtaaatatat 3513
tcaaattatg tataaatata tattatatat ttacaaggag ttattttttg tattgatttt 3573
aaatggatgt cccaatgcac ctagaaaatt ggtctctctt tttttaatag ctatttgcta 3633
aatgctgttc ttacacataa tttcttaatt ttcaccgagc agaggtggaa aatacttttt 3693
gctttcaggg aaaatggtat aacgttaatt tattaataaa ttggtaatat acaaaacaat 3753
taatcattta tagttttttt tgtaatttaa gtggcatttc tatgcaggca gcacagcaga 3813
ctagttaatc tattgcttgg acttaactag ttatcagatc ctttgaaaag agaatattta 3873
caatatatga ctaatttggg gaaaatgaag ttttgattta tttgtgttta aatgctgctg 3933
tcagacgatt gttcttagac ctcctaaatg ccccatatta aaagaactca ttcataggaa 3993
ggtgtttcat tttggtgtgc aaccctgtca ttacgtcaac gcaacgtcta actggacttc 4053
ccaagataaa tggtaccagc gtcctcttaa aagatgcctt aatccattcc ttgaggacag 4113
accttagttg aaatgatagc agaatgtgct tctctctggc agctggcctt ctgcttctga 4173
gttgcacatt aatcagatta gcctgtattc tcttcagtga attttgataa tggcttccag 4233
actctttggc gttggagacg cctgttagga tcttcaagtc ccatcataga aaattgaaac 4293
acagagttgt tctgctgata gttttgggga tacgtccatc tttttaaggg attgctttca 4353
tctaattctg gcaggacctc accaaaagat ccagcctcat acctacatca gacaaaatat 4413
cgccgttgtt ccttctgtac taaagtattg tgttttgctt tggaaacacc cactcacttt 4473
gcaatagccg tgcaagatga atgcagatta cactgatctt atgtgttaca aaattggaga 4533
aagtatttaa taaaacctgt taatttttat actgacaata aaaatgtttc tacagatatt 4593
aatgttaaca agacaaaata aatgtcacgc aacttatttt tttaataaaa aaaaaaaaaa 4653
a 4654

<210> 2
<211> 821
<212> PRT
<213> Homo sapiens
<400> 2

<210>  3
<211>  3119
<212>  DNA
<213>  Homo sapiens
<220>
<221>  CDS
<222>  (1)..(2925)
<223>
<400>  3
atg gcc gcc cag gga gag ccc ggc tac ctg gcg gcg cag tcg gac ccc        48
Met Ala Ala Gln Gly Glu Pro Gly Tyr Leu Ala Ala Gln Ser Asp Pro
1               5                   10                  15
ggc tcc aac agc gag cgc agc acc gac tcc cca gtg ccc ggc tcc gag        96
Gly Ser Asn Ser Glu Arg Ser Thr Asp Ser Pro Val Pro Gly Ser Glu
            20                  25                  30
gac gac ttg gtc gcc ggg gcg acc ctg cac agc ccg gag tgg agc gag       144
Asp Asp Leu Val Ala Gly Ala Thr Leu His Ser Pro Glu Trp Ser Glu
        35                  40                  45
gag cgc ttc cgc gtg gac agg aag aaa ctt gag gcc atg tta caa gct       192
Glu Arg Phe Arg Val Asp Arg Lys Lys Leu Glu Ala Met Leu Gln Ala
    50                  55                  60
gct gct gaa ggg aaa ggc aga agt ggg gaa gac ttt ttt caa aag atc       240
Ala Ala Glu Gly Lys Gly Arg Ser Gly Glu Asp Phe Phe Gln Lys Ile
65                  70                  75                  80
atg gag gaa aca aat acg cag att gct tgg cca tca aaa ctg aag atc       288
Met Glu Glu Thr Asn Thr Gln Ile Ala Trp Pro Ser Lys Leu Lys Ile
                85                  90                  95
gga gcc aaa tcc aag aaa gat ccc cat att aag gtt tct gga aag aaa       336
Gly Ala Lys Ser Lys Lys Asp Pro His Ile Lys Val Ser Gly Lys Lys
            100                 105                 110
gaa gat gtt aaa gaa gcc aag gaa atg atc atg tct gtc tta gac aca       384
Glu Asp Val Lys Glu Ala Lys Glu Met Ile Met Ser Val Leu Asp Thr
        115                 120                 125
aaa agc aat cga gtc aca ctg aag atg gat gtt tca cat aca gaa cat       432
Lys Ser Asn Arg Val Thr Leu Lys Met Asp Val Ser His Thr Glu His
    130                 135                 140
tca cat gta atc ggc aaa ggt ggc aac aat att aaa aaa gtg atg gaa       480
Ser His Val Ile Gly Lys Gly Gly Asn Asn Ile Lys Lys Val Met Glu
145                 150                 155                 160
gaa acc gga tgc cat atc cac ttt cca gat tcc aac agg aat aac caa       528
Glu Thr Gly Cys His Ile His Phe Pro Asp Ser Asn Arg Asn Asn Gln
                165                 170                 175
gca gaa aaa agc aac cag gta tct ata gcg gga caa cca gca gga gta       576
Ala Glu Lys Ser Asn Gln Val Ser Ile Ala Gly Gln Pro Ala Gly Val
            180                 185                 190
gaa tct gcc cga gtt aga att cgg gag ctg ctt cct ttg gtg ctg atg       624
Glu Ser Ala Arg Val Arg Ile Arg Glu Leu Leu Pro Leu Val Leu Met
        195                 200                 205
ttt gag cta cca att gct gga att ctt caa ccg gtt cct gat cct aat       672
Phe Glu Leu Pro Ile Ala Gly Ile Leu Gln Pro Val Pro Asp Pro Asn
    210                 215                 220
tcc ccc tct att cag cat ata tca caa acg tac aat att tca gta tca       720
Ser Pro Ser Ile Gln His Ile Ser Gln Thr Tyr Asn Ile Ser Val Ser
225                 230                 235                 240

ttt aaa cag cgt tcc cga atg tat ggt gct act gtc ata gta cga ggg 768
Phe Lys Gln Arg Ser Arg Met Tyr Gly Ala Thr Val Ile Val Arg Gly
                245                 250                 255
tct cag aat aac act agt gct gtg aag gaa gga act gcc atg ctg tta 816
Ser Gln Asn Asn Thr Ser Ala Val Lys Glu Gly Thr Ala Met Leu Leu
            260                 265                 270
gaa cat ctt gct ggg agc tta gca tca gct att cct gtg agc aca caa 864
Glu His Leu Ala Gly Ser Leu Ala Ser Ala Ile Pro Val Ser Thr Gln
        275                 280                 285
cta gat att gca gct caa cat cat ctc ttt atg atg ggt cga aat ggg 912
Leu Asp Ile Ala Ala Gln His His Leu Phe Met Met Gly Arg Asn Gly
    290                 295                 300
agc aac atc aaa cat atc atg cag aga aca ggt gct cag atc cac ttt 960
Ser Asn Ile Lys His Ile Met Gln Arg Thr Gly Ala Gln Ile His Phe
305                 310                 315                 320
cct gat ccc agt aat cca caa aag aaa tct acc gtc tac ctc cag ggc 1008
Pro Asp Pro Ser Asn Pro Gln Lys Lys Ser Thr Val Tyr Leu Gln Gly
                325                 330                 335
acc att gag tct gtc tgt ctt gca agg caa tat ctc atg ggt tgt ctt 1056
Thr Ile Glu Ser Val Cys Leu Ala Arg Gln Tyr Leu Met Gly Cys Leu
            340                 345                 350
cct ctt gtg ttg atg ttt gat atg aag gaa gaa att gaa gta gat cca 1104
Pro Leu Val Leu Met Phe Asp Met Lys Glu Glu Ile Glu Val Asp Pro
        355                 360                 365
caa ttc att gcg cag ttg atg gaa cag ctt gat gtc ttc atc agt att 1152
Gln Phe Ile Ala Gln Leu Met Glu Gln Leu Asp Val Phe Ile Ser Ile
    370                 375                 380
aaa cca aag ccc aaa cag cca agc aag tct gtg att gtg aaa agt gtt 1200
Lys Pro Lys Pro Lys Gln Pro Ser Lys Ser Val Ile Val Lys Ser Val
385                 390                 395                 400
gag cga aat gcc tta aat atg tat gaa gca agg aaa tgt ctc ctc gga 1248
Glu Arg Asn Ala Leu Asn Met Tyr Glu Ala Arg Lys Cys Leu Leu Gly
                405                 410                 415
ctt gaa agc agt ggg gtt acc ata gca acc agt cca tcc cca gca tcc 1296
Leu Glu Ser Ser Gly Val Thr Ile Ala Thr Ser Pro Ser Pro Ala Ser
            420                 425                 430
tgc cct gcc ggc ctg gca tgt ccc agc ctg gat atc tta gct tca gca 1344
Cys Pro Ala Gly Leu Ala Cys Pro Ser Leu Asp Ile Leu Ala Ser Ala
        435                 440                 445
ggc ctt gga ctc act gga cta ggt ctt ttg gga ccc acc acc tta tct 1392
Gly Leu Gly Leu Thr Gly Leu Gly Leu Leu Gly Pro Thr Thr Leu Ser
    450                 455                 460
ctg aac act tca aca acc cca aac tca ctc ttg aat gct ctt aat agc 1440
Leu Asn Thr Ser Thr Thr Pro Asn Ser Leu Leu Asn Ala Leu Asn Ser
465                 470                 475                 480
tca gtc agt cct ttg caa agt cca agt tct ggt aca ccc agc ccc aca 1488
Ser Val Ser Pro Leu Gln Ser Pro Ser Ser Gly Thr Pro Ser Pro Thr
                485                 490                 495
tta tgg gca ccc cca ctt gct aat act tca agt gcc aca ggt ttt tct 1536
Leu Trp Ala Pro Pro Leu Ala Asn Thr Ser Ser Ala Thr Gly Phe Ser
            500                 505                 510
gct ata cca cac ctt atg att cca tct act gcc caa gcc aca tta act 1584
Ala Ile Pro His Leu Met Ile Pro Ser Thr Ala Gln Ala Thr Leu Thr
        515                 520                 525
aat att ttg ttg tct gga gtg ccc acc tat ggg cac aca gct cca tct 1632
Asn Ile Leu Leu Ser Gly Val Pro Thr Tyr Gly His Thr Ala Pro Ser
    530                 535                 540
ccc cct cct ggc ttg act cct gtt gat gtc cat atc aac agt atg cag 1680
Pro Pro Pro Gly Leu Thr Pro Val Asp Val His Ile Asn Ser Met Gln
545                 550                 555                 560
acc gaa ggc aaa aaa atc tct gct gct tta aat gga cat gca cag tct 1728
Thr Glu Gly Lys Lys Ile Ser Ala Ala Leu Asn Gly His Ala Gln Ser
                565                 570                 575

cca gat ata aaa tat ggt gca ata tcc act tca tca ctt gga gaa aaa 1776
Pro Asp Ile Lys Tyr Gly Ala Ile Ser Thr Ser Ser Leu Gly Glu Lys
            580                 585                 590

gtg ctg agt gca aat cac ggg gat ccg tcc atc cag aca agt ggg tct 1824
Val Leu Ser Ala Asn His Gly Asp Pro Ser Ile Gln Thr Ser Gly Ser
        595                 600                 605

gag cag aca tct ccc aaa tca agc ccc act gaa ggt tgt aat gat gct 1872
Glu Gln Thr Ser Pro Lys Ser Ser Pro Thr Glu Gly Cys Asn Asp Ala
    610                 615                 620

ttt gtt gaa gta ggc atg cct cga agt cct tcc cat tct ggg aat gct 1920
Phe Val Glu Val Gly Met Pro Arg Ser Pro Ser His Ser Gly Asn Ala
625                 630                 635                 640

ggt gac ttg aaa cag atg atg tgt ccc tcc aag gtt tcc tgt gcc aaa 1968
Gly Asp Leu Lys Gln Met Met Cys Pro Ser Lys Val Ser Cys Ala Lys
                645                 650                 655

agg cag aca gtg gaa cta ttg caa ggc acg aaa aac tca cac tta cac 2016
Arg Gln Thr Val Glu Leu Leu Gln Gly Thr Lys Asn Ser His Leu His
            660                 665                 670

agc act gac agg ttg ctc tca gac cct gaa ctg agt gct acc gaa agc 2064
Ser Thr Asp Arg Leu Leu Ser Asp Pro Glu Leu Ser Ala Thr Glu Ser
        675                 680                 685

cct ttg gct gac aag aag gct cca ggg agt gag cgc gct gca gag agg 2112
Pro Leu Ala Asp Lys Lys Ala Pro Gly Ser Glu Arg Ala Ala Glu Arg
    690                 695                 700

gca gca gct gcc cag caa aac tcc gaa agg gcc cac ctt gct cca cgg 2160
Ala Ala Ala Ala Gln Gln Asn Ser Glu Arg Ala His Leu Ala Pro Arg
705                 710                 715                 720

tca tca tat gtc aac atg cag gca ttt gac tat gaa cag aag aag cta 2208
Ser Ser Tyr Val Asn Met Gln Ala Phe Asp Tyr Glu Gln Lys Lys Leu
                725                 730                 735

tta gcc acc aaa gct atg tta aag aaa cca gtg gtg acg gag gtc aga 2256
Leu Ala Thr Lys Ala Met Leu Lys Lys Pro Val Val Thr Glu Val Arg
            740                 745                 750

acg ccc aca aat acc tgg agt ggc ctg ggt ttt tct aaa tcc atg cca 2304
Thr Pro Thr Asn Thr Trp Ser Gly Leu Gly Phe Ser Lys Ser Met Pro
        755                 760                 765

gct gaa act atc aag gag ttg aga agg gcc aat cat gtg tcc tat aag 2352
Ala Glu Thr Ile Lys Glu Leu Arg Arg Ala Asn His Val Ser Tyr Lys
    770                 775                 780

ccc aca atg aca acc act tat gag ggc tca tcc atg tcc ctt tca cgg 2400
Pro Thr Met Thr Thr Thr Tyr Glu Gly Ser Ser Met Ser Leu Ser Arg
785                 790                 795                 800

tcc aac agt cgt gag cac ttg gga ggt gga agc gaa tct gat aac tgg 2448
Ser Asn Ser Arg Glu His Leu Gly Gly Gly Ser Glu Ser Asp Asn Trp
                805                 810                 815

aga gac cga aat gga att gga cct gga agt cat agt gaa ttt gca gct 2496
Arg Asp Arg Asn Gly Ile Gly Pro Gly Ser His Ser Glu Phe Ala Ala
            820                 825                 830

tct att ggc agc cct aag cgt aaa caa aac aaa tca acg gaa cac tat 2544
Ser Ile Gly Ser Pro Lys Arg Lys Gln Asn Lys Ser Thr Glu His Tyr
        835                 840                 845

ctc agc agt agc aat tac atg gac tgc att tcc tcg ctg aca gga agc 2592
Leu Ser Ser Ser Asn Tyr Met Asp Cys Ile Ser Ser Leu Thr Gly Ser
    850                 855                 860

aat ggc tgt aac tta aat agc tct ttc aaa ggt tct gac ctc cct gag 2640
Asn Gly Cys Asn Leu Asn Ser Ser Phe Lys Gly Ser Asp Leu Pro Glu
865                 870                 875                 880

ctc ttc agc aaa ctg ggc ctg ggc aaa tac aca gat gtt ttc cag caa 2688
Leu Phe Ser Lys Leu Gly Leu Gly Lys Tyr Thr Asp Val Phe Gln Gln
                885                 890                 895

caa gag atc gat ctt cag aca ttc ctc act ctc aca gat cag gat ctg 2736
Gln Glu Ile Asp Leu Gln Thr Phe Leu Thr Leu Thr Asp Gln Asp Leu
            900                 905                 910

aag gag ctg gga ata act act ttt ggt gcc agg agg aaa atg ctg ctt 2784
Lys Glu Leu Gly Ile Thr Thr Phe Gly Ala Arg Arg Lys Met Leu Leu
        915                 920                 925
gca att tca gaa cta aat aaa aac cga aga aag ctt ttt gaa tcg cca 2832
Ala Ile Ser Glu Leu Asn Lys Asn Arg Arg Lys Leu Phe Glu Ser Pro
    930                 935                 940
aat gca cgc acc tct ttc ctg gaa ggt gga gcg agt gga agg cta ccc 2880
Asn Ala Arg Thr Ser Phe Leu Glu Gly Gly Ala Ser Gly Arg Leu Pro
945                 950                 955                 960
cgt cag tat cac tca gac att gct agt gtc agt ggc cgc tgg tag 2925
Arg Gln Tyr His Ser Asp Ile Ala Ser Val Ser Gly Arg Trp
                965                 970
cagcaccctc ttggcacatg cccgctgact aactgtaaag tggacacagg agatgtatga 2985
acagccttca cagcacacca tccttagcac tctgggtgtc tggtatcagg accaaagcat 3045
tttattcgca cctgtacttt atggcaaaaa ggaagaagag agagaagatg ttcttatgat 3105
gtcatacaga acac 3119

<210> 4
<211> 974
<212> PRT
<213> Homo sapiens
<400> 4
Met Ala Ala Gln Gly Glu Pro Gly Tyr Leu Ala Ala Gln Ser Asp Pro
1               5                   10                  15
Gly Ser Asn Ser Glu Arg Ser Thr Asp Ser Pro Val Pro Gly Ser Glu
            20                  25                  30
Asp Asp Leu Val Ala Gly Ala Thr Leu His Ser Pro Glu Trp Ser Glu
        35                  40                  45
Glu Arg Phe Arg Val Asp Arg Lys Lys Leu Glu Ala Met Leu Gln Ala
    50                  55                  60
Ala Ala Glu Gly Lys Gly Arg Ser Gly Glu Asp Phe Phe Gln Lys Ile
65                  70                  75                  80
Met Glu Glu Thr Asn Thr Gln Ile Ala Trp Pro Ser Lys Leu Lys Ile
                85                  90                  95
Gly Ala Lys Ser Lys Lys Asp Pro His Ile Lys Val Ser Gly Lys Lys
            100                 105                 110
Glu Asp Val Lys Glu Ala Lys Glu Met Ile Met Ser Val Leu Asp Thr
        115                 120                 125
Lys Ser Asn Arg Val Thr Leu Lys Met Asp Val Ser His Thr Glu His
    130                 135                 140
Ser His Val Ile Gly Lys Gly Gly Asn Asn Ile Lys Lys Val Met Glu
145                 150                 155                 160
Glu Thr Gly Cys His Ile His Phe Pro Asp Ser Asn Arg Asn Asn Gln
                165                 170                 175
Ala Glu Lys Ser Asn Gln Val Ser Ile Ala Gly Gln Pro Ala Gly Val
            180                 185                 190
Glu Ser Ala Arg Val Arg Ile Arg Glu Leu Leu Pro Leu Val Leu Met
        195                 200                 205
Phe Glu Leu Pro Ile Ala Gly Ile Leu Gln Pro Val Pro Asp Pro Asn
    210                 215                 220
Ser Pro Ser Ile Gln His Ile Ser Gln Thr Tyr Asn Ile Ser Val Ser
225                 230                 235                 240
Phe Lys Gln Arg Ser Arg Met Tyr Gly Ala Thr Val Ile Val Arg Gly
                245                 250                 255
Ser Gln Asn Asn Thr Ser Ala Val Lys Glu Gly Thr Ala Met Leu Leu
            260                 265                 270
Glu His Leu Ala Gly Ser Leu Ala Ser Ala Ile Pro Val Ser Thr Gln
        275                 280                 285
Leu Asp Ile Ala Ala Gln His His Leu Phe Met Met Gly Arg Asn Gly
    290                 295                 300
Ser Asn Ile Lys His Ile Met Gln Arg Thr Gly Ala Gln Ile His Phe
305                 310                 315                 320
Pro Asp Pro Ser Asn Pro Gln Lys Lys Ser Thr Val Tyr Leu Gln Gly

                325                 330                 335
Thr Ile Glu Ser Val Cys Leu Ala Arg Gln Tyr Leu Met Gly Cys Leu
            340                 345                 350
Pro Leu Val Leu Met Phe Asp Met Lys Glu Glu Ile Glu Val Asp Pro
        355                 360                 365
Gln Phe Ile Ala Gln Leu Met Glu Gln Leu Asp Val Phe Ile Ser Ile
    370                 375                 380
Lys Pro Lys Pro Lys Gln Pro Ser Lys Ser Val Ile Val Lys Ser Val
385                 390                 395                 400
Glu Arg Asn Ala Leu Asn Met Tyr Glu Ala Arg Lys Cys Leu Leu Gly
                405                 410                 415
Leu Glu Ser Ser Gly Val Thr Ile Ala Thr Ser Pro Ser Pro Ala Ser
            420                 425                 430
Cys Pro Ala Gly Leu Ala Cys Pro Ser Leu Asp Ile Leu Ala Ser Ala
        435                 440                 445
Gly Leu Gly Leu Thr Gly Leu Gly Leu Leu Gly Pro Thr Thr Leu Ser
    450                 455                 460
Leu Asn Thr Ser Thr Thr Pro Asn Ser Leu Leu Asn Ala Leu Asn Ser
465                 470                 475                 480
Ser Val Ser Pro Leu Gln Ser Pro Ser Ser Gly Thr Pro Ser Pro Thr
                485                 490                 495
Leu Trp Ala Pro Pro Leu Ala Asn Thr Ser Ser Ala Thr Gly Phe Ser
            500                 505                 510
Ala Ile Pro His Leu Met Ile Pro Ser Thr Ala Gln Ala Thr Leu Thr
        515                 520                 525
Asn Ile Leu Leu Ser Gly Val Pro Thr Tyr Gly His Thr Ala Pro Ser
    530                 535                 540
Pro Pro Pro Gly Leu Thr Pro Val Asp Val His Ile Asn Ser Met Gln
545                 550                 555                 560
Thr Glu Gly Lys Lys Ile Ser Ala Ala Leu Asn Gly His Ala Gln Ser
                565                 570                 575
Pro Asp Ile Lys Tyr Gly Ala Ile Ser Thr Ser Ser Leu Gly Glu Lys
            580                 585                 590
Val Leu Ser Ala Asn His Gly Asp Pro Ser Ile Gln Thr Ser Gly Ser
        595                 600                 605
Glu Gln Thr Ser Pro Lys Ser Ser Pro Thr Glu Gly Cys Asn Asp Ala
    610                 615                 620
Phe Val Glu Val Gly Met Pro Arg Ser Pro Ser His Ser Gly Asn Ala
625                 630                 635                 640
Gly Asp Leu Lys Gln Met Met Cys Pro Ser Lys Val Ser Cys Ala Lys
                645                 650                 655
Arg Gln Thr Val Glu Leu Leu Gln Gly Thr Lys Asn Ser His Leu His
            660                 665                 670
Ser Thr Asp Arg Leu Leu Ser Asp Pro Glu Leu Ser Ala Thr Glu Ser
        675                 680                 685
Pro Leu Ala Asp Lys Lys Ala Pro Gly Ser Glu Arg Ala Ala Glu Arg
    690                 695                 700
Ala Ala Ala Ala Gln Gln Asn Ser Glu Arg Ala His Leu Ala Pro Arg
705                 710                 715                 720
Ser Ser Tyr Val Asn Met Gln Ala Phe Asp Tyr Glu Gln Lys Lys Leu
                725                 730                 735
Leu Ala Thr Lys Ala Met Leu Lys Lys Pro Val Val Thr Glu Val Arg
            740                 745                 750
Thr Pro Thr Asn Thr Trp Ser Gly Leu Gly Phe Ser Lys Ser Met Pro
        755                 760                 765
Ala Glu Thr Ile Lys Glu Leu Arg Arg Ala Asn His Val Ser Tyr Lys
    770                 775                 780
Pro Thr Met Thr Thr Thr Tyr Glu Gly Ser Ser Met Ser Leu Ser Arg
785                 790                 795                 800
Ser Asn Ser Arg Glu His Leu Gly Gly Gly Ser Glu Ser Asp Asn Trp
                805                 810                 815
Arg Asp Arg Asn Gly Ile Gly Pro Gly Ser His Ser Glu Phe Ala Ala
            820                 825                 830

Ser Ile Gly Ser Pro Lys Arg Lys Gln Asn Lys Ser Thr Glu His Tyr
        835                 840                 845
Leu Ser Ser Ser Asn Tyr Met Asp Cys Ile Ser Ser Leu Thr Gly Ser
    850                 855                 860
Asn Gly Cys Asn Leu Asn Ser Ser Phe Lys Gly Ser Asp Leu Pro Glu
865                 870                 875                 880
Leu Phe Ser Lys Leu Gly Leu Gly Lys Tyr Thr Asp Val Phe Gln Gln
                885                 890                 895
Gln Glu Ile Asp Leu Gln Thr Phe Leu Thr Leu Thr Asp Gln Asp Leu
            900                 905                 910
Lys Glu Leu Gly Ile Thr Thr Phe Gly Ala Arg Arg Lys Met Leu Leu
        915                 920                 925
Ala Ile Ser Glu Leu Asn Lys Asn Arg Arg Lys Leu Phe Glu Ser Pro
    930                 935                 940
Asn Ala Arg Thr Ser Phe Leu Glu Gly Gly Ala Ser Gly Arg Leu Pro
945                 950                 955                 960
Arg Gln Tyr His Ser Asp Ile Ala Ser Val Ser Gly Arg Trp
                965                 970

<210>  5
<211>  3949
<212>  DNA
<213>  Homo sapiens
<220>
<221>  CDS
<222>  (290)..(1882)
<223>
<400>  5
gctttgcgtg cgtgtttgcg tacagcggag gtggcggcgc gggcaggtcg gagctcggag      60
ctgctgcttc tggttctctt gtggccgccg tcgctgtccg gctgccttgg gctgccgaac     120
agacaaggcg tgggccacag cacctcagaa gccgacgcag ctcgacgcag gggccggcag     180
gagggtgggc gatcgcgtgt cggagggcgc cgcgcgggca ggcgggcggg cgccagaggg     240
ggaaagaggc gggggcggcg ggtcagccgc tggccgggcc ggccgggga atg tcg atg     298
                                                    Met Ser Met
                                                    1
cct gac gcg atg ccg ctg ccc ggg gtc ggg gag gag ctg aag cag gcc      346
Pro Asp Ala Met Pro Leu Pro Gly Val Gly Glu Glu Leu Lys Gln Ala
    5                   10                  15
aag gag atc gag gac gcc gag aag tac tcc ttc atg gcc acc gtc acc      394
Lys Glu Ile Glu Asp Ala Glu Lys Tyr Ser Phe Met Ala Thr Val Thr
20                  25                  30                  35
aag gcg ccc aag aag caa atc cag ttt gct gat gac atg cag gag ttc      442
Lys Ala Pro Lys Lys Gln Ile Gln Phe Ala Asp Asp Met Gln Glu Phe
                40                  45                  50
acc aaa ttc ccc acc aaa act ggc cga aga tct ttg tct cgc tcg atc      490
Thr Lys Phe Pro Thr Lys Thr Gly Arg Arg Ser Leu Ser Arg Ser Ile
            55                  60                  65
tca cag tcc tcc act gac agc tac agt tca gct gca tcc tac aca gat      538
Ser Gln Ser Ser Thr Asp Ser Tyr Ser Ser Ala Ala Ser Tyr Thr Asp
        70                  75                  80
agc tct gat gat gag gtt tct ccc cga gag aag cag caa acc aac tcc      586
Ser Ser Asp Asp Glu Val Ser Pro Arg Glu Lys Gln Gln Thr Asn Ser
    85                  90                  95
aag ggc agc agc aat ttc tgt gtg aag aac atc aag cag gca gaa ttt      634
Lys Gly Ser Ser Asn Phe Cys Val Lys Asn Ile Lys Gln Ala Glu Phe
100                 105                 110                 115
gga cgc cgg gag att gag att gca gag caa gac atg tct gct ctg att      682
Gly Arg Arg Glu Ile Glu Ile Ala Glu Gln Asp Met Ser Ala Leu Ile
                120                 125                 130
tca ctc agg aaa cgt gct cag ggg gag aag ccc ttg gct ggt gct aaa      730
Ser Leu Arg Lys Arg Ala Gln Gly Glu Lys Pro Leu Ala Gly Ala Lys
            135                 140                 145
ata gtg ggc tgt aca cac atc aca gcc cag aca gcg gtg ttg att gag      778

Ile Val Gly Cys Thr His Ile Thr Ala Gln Thr Ala Val Leu Ile Glu
        150                 155                 160
aca ctc tgt gcc ctg ggg gct cag tgc cgc tgg tct gct tgt aac atc      826
Thr Leu Cys Ala Leu Gly Ala Gln Cys Arg Trp Ser Ala Cys Asn Ile
    165                 170                 175
tac tca act cag aat gaa gta gct gca gca ctg gct gag gct gga gtt      874
Tyr Ser Thr Gln Asn Glu Val Ala Ala Ala Leu Ala Glu Ala Gly Val
180                 185                 190                 195
gca gtg ttc gct tgg aag ggc gag tca gaa gat gac ttc tgg tgg tgt      922
Ala Val Phe Ala Trp Lys Gly Glu Ser Glu Asp Asp Phe Trp Trp Cys
                200                 205                 210
att gac cgc tgt gtg aac atg gat ggg tgg cag gcc aac atg atc ctg      970
Ile Asp Arg Cys Val Asn Met Asp Gly Trp Gln Ala Asn Met Ile Leu
            215                 220                 225
gat gat ggg gga gac tta acc cac tgg gtt tat aag aag tat cca aac     1018
Asp Asp Gly Gly Asp Leu Thr His Trp Val Tyr Lys Lys Tyr Pro Asn
        230                 235                 240
gtg ttt aag aag atc cga ggc att gtg gaa gag agc gtg act ggt gtt     1066
Val Phe Lys Lys Ile Arg Gly Ile Val Glu Glu Ser Val Thr Gly Val
    245                 250                 255
cac agg ctg tat cag ctc tcc aaa gct ggg aag ctc tgt gtt ccg gcc     1114
His Arg Leu Tyr Gln Leu Ser Lys Ala Gly Lys Leu Cys Val Pro Ala
260                 265                 270                 275
atg aac gtc aat gat tct gtt acc aaa cag aag ttt gat aac ttg tac     1162
Met Asn Val Asn Asp Ser Val Thr Lys Gln Lys Phe Asp Asn Leu Tyr
                280                 285                 290
tgc tgc cga gaa tcc att ttg gat ggc ctg aag agg acc aca gat gtg     1210
Cys Cys Arg Glu Ser Ile Leu Asp Gly Leu Lys Arg Thr Thr Asp Val
            295                 300                 305
atg ttt ggt ggg aaa caa gtg gtg gtg tgt ggc tat ggt gag gta ggc     1258
Met Phe Gly Gly Lys Gln Val Val Val Cys Gly Tyr Gly Glu Val Gly
        310                 315                 320
aag ggc tgc tgt gct gct ctc aaa gct ctt gga gca att gtc tac att     1306
Lys Gly Cys Cys Ala Ala Leu Lys Ala Leu Gly Ala Ile Val Tyr Ile
    325                 330                 335
acc gaa atc gac ccc atc tgt gct ctg cag gcc tgc atg gat ggg ttc     1354
Thr Glu Ile Asp Pro Ile Cys Ala Leu Gln Ala Cys Met Asp Gly Phe
340                 345                 350                 355
agg gtg gta aag cta aat gaa gtc atc cgg caa gtc gat gtc gta ata     1402
Arg Val Val Lys Leu Asn Glu Val Ile Arg Gln Val Asp Val Val Ile
                360                 365                 370
act tgc aca gga aat aag aat gta gtg aca cgg gag cac ttg gat cgc     1450
Thr Cys Thr Gly Asn Lys Asn Val Val Thr Arg Glu His Leu Asp Arg
            375                 380                 385
atg aaa aac agt tgt atc gta tgc aat atg ggc cac tcc aac aca gaa     1498
Met Lys Asn Ser Cys Ile Val Cys Asn Met Gly His Ser Asn Thr Glu
        390                 395                 400
atc gat gtg acc agc ctc cgc act ccg gag ctg acg tgg gag cga gta     1546
Ile Asp Val Thr Ser Leu Arg Thr Pro Glu Leu Thr Trp Glu Arg Val
    405                 410                 415
cgt tct cag gtg gac cat gtc atc tgg cca gat ggc aaa cga gtt gtc     1594
Arg Ser Gln Val Asp His Val Ile Trp Pro Asp Gly Lys Arg Val Val
420                 425                 430                 435
ctc ctg gca gag ggt cgt cta ctc aat ttg agc tgc tcc aca gtt ccc     1642
Leu Leu Ala Glu Gly Arg Leu Leu Asn Leu Ser Cys Ser Thr Val Pro
                440                 445                 450
acc ttt gtt ctg tcc atc aca gcc aca aca cag gct ttg gca ctg ata     1690
Thr Phe Val Leu Ser Ile Thr Ala Thr Thr Gln Ala Leu Ala Leu Ile
            455                 460                 465
gaa ctc tat aat gca ccc gag ggg cga tac aag cag gat gtg tac ttg     1738
Glu Leu Tyr Asn Ala Pro Glu Gly Arg Tyr Lys Gln Asp Val Tyr Leu
        470                 475                 480
ctt cct aag aaa atg gat gaa tac gtt gcc agc ttg cat ctg cca tca     1786

Leu Pro Lys Lys Met Asp Glu Tyr Val Ala Ser Leu His Leu Pro Ser
    485                 490                 495
ttt gat gcc cac ctt aca gag ctg aca gat gac caa gca aaa tat ctg      1834
Phe Asp Ala His Leu Thr Glu Leu Thr Asp Asp Gln Ala Lys Tyr Leu
500                 505                 510                 515
gga ctc aac aaa aat ggg cca ttc aaa cct aat tat tac aga tac taa      1882
Gly Leu Asn Lys Asn Gly Pro Phe Lys Pro Asn Tyr Tyr Arg Tyr
                520                 525                 530
tggaccatac taccaaggac cagtccacct gaaccacaca ctctaaagaa atatttttta   1942
agataacttt tattttcttc ttactccttt cctcttgatt tttttcctat aatttcattc   2002
ttgttttttc atctcattat ccaagttctg cagaccacac aggaacttgc ttcatggctc   2062
tttagatgaa atagaagttc agggttcctc actctagtca ctaaagaagg attttactct   2122
cccagcccag aaaggtgatt ctttctttac catttctggg gactttagtc ttaattaggt   2182
accttattaa caggaaatgc taaggtacct tctctgtgga acaatctgca atgtctaaat   2242
cgccttaaaa gagcccattt cttagctgct gaaatcagtg ctctttcact tcttcagaga   2302
agcagggatg gtacctaccc ggcaggtagg ttagatgtgg gtggtgcatg ttaatttccc   2362
ttagaagttc caagccctgt ttcctgcgta aaggtggtat gtccagttca gagatgtgta   2422
taatgagcat ggcttgttaa gatcaggagg cccacttgga tttatagtat agcccttcct   2482
ccactcccac cagacttgct catttttcga gtttttaact agactacact ctattgagtt   2542
taattttgtc ctctaggatt tatttctgtt gtccaaaaaa aaaaaaaaag aaaagaaaaa   2602
ttaaggagaa tttttggtgt taatgctgag gaattgcttg agtggttagt tgttaccaat   2662
ttctcttttg aacctttgga gctaaggatg ctgagtctag agaaatgcta gtctcaagcc   2722
ctgttaagtc cctctgtttc tagcccgtag ttcatagcat cagtgaactg gagccacaac   2782
agcaaattct atcagctgtg taccatacag cttgtgctga aggcgaattt cttgagccat   2842
tactcagtat aaagcactga gttctatctt taggatttat ctttaagagc aaatttctgg   2902
tcagctgtgc ttctgcaacc taaaatattt aaagggaggt aggtgtgggc aggaggagga   2962
atgataaatt gggccagggc aagaaaaatc tagcttcata taatttgtct gggactatac   3022
accctatata atgttagttt tacagaagta atatgacttt tgattgctac ataccacaaa   3082
gagtttatga actgagatca taaagggcaa ctgatgtgtg aagaaagtag tcagtacatc   3142
ctggctcatg ctctgaaaga atatccagag aggctctctc aaagatcagg gagatgtatt   3202
cccatgccat gcaccctgct tcccagcatt tctgcatggt caagtgagct ttatgctcat   3262
gagctttaag tatataatta tccaggattt taaatcctca acttgttcta gcttgtgatc   3322
cctcaaagtt gggtcatacg ttagtgctag atactagaaa ttttcacttt tccactgatc   3382
agagagacag acattaaaaa caaaaataga agaaaggaaa gctttcaccc tgcagcttct   3442
tagcagggaa caattgtctt gccaaaactt ttttcccttt tctctcccat tttcttttac   3502
ccaatccctt cttactcctt gccagtgtga ccatgctttc ttctctgtag atgttaacag   3562
ttaaggccta ttttcctcgg gcacttaacc aaccaatcag aacaccacat ctgttagggg   3622
aggtaacctg gccaacagtg tatccatcac gttagccctg ctggagggaa gggacccaca   3682
ttcacctgcc ctctgacctg ccccttgatc ccatatctat taccgtgtcc ataggaataa   3742
taggtaaggg ctctgtctct gtcaagccat gtaacaaagg acactgttaa aaaaaaaaaa   3802
aagtctggca tcagagggag catgtggaga gcaacttggg aagaacaagt tcattttgta   3862
ttgaatgatt tttaatgaat gcaatattaa tccttgcaga tgagcaataa tcattaaaat   3922
cgattaaaat gataagacct taaaaaa                                        3949

<210>  6
<211>  530
<212>  PRT
<213>  Homo sapiens
<400>  6
Met Ser Met Pro Asp Ala Met Pro Leu Pro Gly Val Gly Glu Glu Leu
1               5                   10                  15
Lys Gln Ala Lys Glu Ile Glu Asp Ala Glu Lys Tyr Ser Phe Met Ala
            20                  25                  30
Thr Val Thr Lys Ala Pro Lys Lys Gln Ile Gln Phe Ala Asp Asp Met
        35                  40                  45
Gln Glu Phe Thr Lys Phe Pro Thr Lys Thr Gly Arg Arg Ser Leu Ser
    50                  55                  60
Arg Ser Ile Ser Gln Ser Ser Thr Asp Ser Tyr Ser Ser Ala Ala Ser
65                  70                  75                  80
Tyr Thr Asp Ser Ser Asp Asp Glu Val Ser Pro Arg Glu Lys Gln Gln
                85                  90                  95
Thr Asn Ser Lys Gly Ser Ser Asn Phe Cys Val Lys Asn Ile Lys Gln
            100                 105                 110

Ala Glu Phe Gly Arg Arg Glu Ile Glu Ile Ala Glu Gln Asp Met Ser
        115                 120                 125
Ala Leu Ile Ser Leu Arg Lys Arg Ala Gln Gly Glu Lys Pro Leu Ala
    130                 135                 140
Gly Ala Lys Ile Val Gly Cys Thr His Ile Thr Ala Gln Thr Ala Val
145                 150                 155                 160
Leu Ile Glu Thr Leu Cys Ala Leu Gly Ala Gln Cys Arg Trp Ser Ala
                165                 170                 175
Cys Asn Ile Tyr Ser Thr Gln Asn Glu Val Ala Ala Ala Leu Ala Glu
            180                 185                 190
Ala Gly Val Ala Val Phe Ala Trp Lys Gly Glu Ser Glu Asp Asp Phe
        195                 200                 205
Trp Trp Cys Ile Asp Arg Cys Val Asn Met Asp Gly Trp Gln Ala Asn
    210                 215                 220
Met Ile Leu Asp Asp Gly Gly Asp Leu Thr His Trp Val Tyr Lys Lys
225                 230                 235                 240
Tyr Pro Asn Val Phe Lys Lys Ile Arg Gly Ile Val Glu Glu Ser Val
                245                 250                 255
Thr Gly Val His Arg Leu Tyr Gln Leu Ser Lys Ala Gly Lys Leu Cys
            260                 265                 270
Val Pro Ala Met Asn Val Asn Asp Ser Val Thr Lys Gln Lys Phe Asp
        275                 280                 285
Asn Leu Tyr Cys Cys Arg Glu Ser Ile Leu Asp Gly Leu Lys Arg Thr
    290                 295                 300
Thr Asp Val Met Phe Gly Gly Lys Gln Val Val Val Cys Gly Tyr Gly
305                 310                 315                 320
Glu Val Gly Lys Gly Cys Cys Ala Ala Leu Lys Ala Leu Gly Ala Ile
                325                 330                 335
Val Tyr Ile Thr Glu Ile Asp Pro Ile Cys Ala Leu Gln Ala Cys Met
            340                 345                 350
Asp Gly Phe Arg Val Val Lys Leu Asn Glu Val Ile Arg Gln Val Asp
        355                 360                 365
Val Val Ile Thr Cys Thr Gly Asn Lys Asn Val Val Thr Arg Glu His
    370                 375                 380
Leu Asp Arg Met Lys Asn Ser Cys Ile Val Cys Asn Met Gly His Ser
385                 390                 395                 400
Asn Thr Glu Ile Asp Val Thr Ser Leu Arg Thr Pro Glu Leu Thr Trp
                405                 410                 415
Glu Arg Val Arg Ser Gln Val Asp His Val Ile Trp Pro Asp Gly Lys
            420                 425                 430
Arg Val Val Leu Leu Ala Glu Gly Arg Leu Leu Asn Leu Ser Cys Ser
        435                 440                 445
Thr Val Pro Thr Phe Val Leu Ser Ile Thr Ala Thr Thr Gln Ala Leu
    450                 455                 460
Ala Leu Ile Glu Leu Tyr Asn Ala Pro Glu Gly Arg Tyr Lys Gln Asp
465                 470                 475                 480
Val Tyr Leu Leu Pro Lys Lys Met Asp Glu Tyr Val Ala Ser Leu His
                485                 490                 495
Leu Pro Ser Phe Asp Ala His Leu Thr Glu Leu Thr Asp Asp Gln Ala
            500                 505                 510
Lys Tyr Leu Gly Leu Asn Lys Asn Gly Pro Phe Lys Pro Asn Tyr Tyr
        515                 520                 525
Arg Tyr
    530

<210>  7
<211>  5830
<212>  DNA
<213>  Homo sapiens
<220>
<221>  CDS
<222>  (648)..(5636)
<223>

<400> 7
ggcggcggct ggaggagagc gcggtggaga gccgagcggg cgggcggcgg gtgcggagcg 60
ggcgagggag cgcgcgcggc cgccacaaag ctcgggcgcc gcggggctgc atgcggcgta 120
cctggcccgg cgcggcgact gctctccggg ctggcggggg ccggccgcga gccccggggg 180
ccccgaggcc gcagcttgcc tgcgcgctct gagccttcgc aactcgcgag caaagtttgg 240
tggaggcaac gccaagcctg agtcctttct tcctctcgtt ccccaaatcc gagggcagcc 300
cgcgggcgtc atgcccgcgc tcctccgcag cctggggtac gcgtgaagcc cgggaggctt 360
ggcgccggcg aagacccaag gaccactctt ctgcgtttgg agttgctccc cgcaaccccg 420
ggctcgtcgc tttctccatc ccgacccacg cggggcgcgg ggacaacaca ggtcgcggag 480
gagcgttgcc attcaagtga ctgcagcagc agcggcagcg cctcggttcc tgagcccacc 540
gcaggctgaa ggcattgcgc gtagtccatg cccgtagagg aagtgtgcag atgggattaa 600
cgtccacatg gagatatgga agaggaccgg ggattggtac cgtaacc atg gtc agc 656
Met Val Ser
1
tgg ggt cgt ttc atc tgc ctg gtc gtg gtc acc atg gca acc ttg tcc 704
Trp Gly Arg Phe Ile Cys Leu Val Val Val Thr Met Ala Thr Leu Ser
    5                   10                  15
ctg gcc cgg ccc tcc ttc agt tta gtt gag gat acc aca tta gag cca 752
Leu Ala Arg Pro Ser Phe Ser Leu Val Glu Asp Thr Thr Leu Glu Pro
20                  25                  30                  35
gaa gag cca cca acc aaa tac caa atc tct caa cca gaa gtg tac gtg 800
Glu Glu Pro Pro Thr Lys Tyr Gln Ile Ser Gln Pro Glu Val Tyr Val
                40                  45                  50
gct gcg cca ggg gag tcg cta gag gtg cgc tgc ctg ttg aaa gat gcc 848
Ala Ala Pro Gly Glu Ser Leu Glu Val Arg Cys Leu Leu Lys Asp Ala
            55                  60                  65
gcc gtg atc agt tgg act aag gat ggg gtg cac ttg ggg ccc aac aat 896
Ala Val Ile Ser Trp Thr Lys Asp Gly Val His Leu Gly Pro Asn Asn
        70                  75                  80
agg aca gtg ctt att ggg gag tac ttg cag ata aag ggc gcc acg cct 944
Arg Thr Val Leu Ile Gly Glu Tyr Leu Gln Ile Lys Gly Ala Thr Pro
    85                  90                  95
aga gac tcc ggc ctc tat gct tgt act gcc agt agg act gta gac agt 992
Arg Asp Ser Gly Leu Tyr Ala Cys Thr Ala Ser Arg Thr Val Asp Ser
100                 105                 110                 115
gaa act tgg tac ttc atg gtg aat gtc aca gat gcc atc tca tcc gga 1040
Glu Thr Trp Tyr Phe Met Val Asn Val Thr Asp Ala Ile Ser Ser Gly
                120                 125                 130
gat gat gag gat gac acc gat ggt gcg gaa gat ttt gtc agt gag aac 1088
Asp Asp Glu Asp Asp Thr Asp Gly Ala Glu Asp Phe Val Ser Glu Asn
            135                 140                 145
agt aac aac aag aga gca cca tac tgg acc aac aca gaa aag atg gaa 1136
Ser Asn Asn Lys Arg Ala Pro Tyr Trp Thr Asn Thr Glu Lys Met Glu
        150                 155                 160
aag cgg ctc cat gct gtg cct gcg gcc aac act gtc aag ttt cgc tgc 1184
Lys Arg Leu His Ala Val Pro Ala Ala Asn Thr Val Lys Phe Arg Cys
    165                 170                 175
cca gcc ggg ggg aac cca atg cca acc atg cgg tgg ctg aaa aac ggg 1232
Pro Ala Gly Gly Asn Pro Met Pro Thr Met Arg Trp Leu Lys Asn Gly
180                 185                 190                 195
aag gag ttt aag cag gag cat cgc att gga ggc tac aag gta cga aac 1280
Lys Glu Phe Lys Gln Glu His Arg Ile Gly Gly Tyr Lys Val Arg Asn
                200                 205                 210
cag cac tgg agc ctc att atg gaa agt gtg gtc cca tct gac aag gga 1328
Gln His Trp Ser Leu Ile Met Glu Ser Val Val Pro Ser Asp Lys Gly
            215                 220                 225
aat tat acc tgt gta gtg gag aat gaa tac ggg tcc atc aat cac acg 1376
Asn Tyr Thr Cys Val Val Glu Asn Glu Tyr Gly Ser Ile Asn His Thr
        230                 235                 240
tac cac ctg gat gtt gtg gag cga tcg cct cac cgg ccc atc ctc caa 1424
Tyr His Leu Asp Val Val Glu Arg Ser Pro His Arg Pro Ile Leu Gln
    245                 250                 255
gcc gga ctg ccg gca aat gcc tcc aca gtg gtc gga gga gac gta gag 1472

Ala Gly Leu Pro Ala Asn Ala Ser Thr Val Val Gly Gly Asp Val Glu
260                 265                 270                 275
ttt gtc tgc aag gtt tac agt gat gcc cag ccc cac atc cag tgg atc      1520
Phe Val Cys Lys Val Tyr Ser Asp Ala Gln Pro His Ile Gln Trp Ile
                280                 285                 290
aag cac gtg gaa aag aac ggc agt aaa tac ggg ccc gac ggg ctg ccc      1568
Lys His Val Glu Lys Asn Gly Ser Lys Tyr Gly Pro Asp Gly Leu Pro
            295                 300                 305
tac ctc aag gtt ctc aag gcc gcc ggt gtt aac acc acg gac aaa gag      1616
Tyr Leu Lys Val Leu Lys Ala Ala Gly Val Asn Thr Thr Asp Lys Glu
        310                 315                 320
att gag gtt ctc tat att cgg aat gta act ttt gag gac gct ggg gaa      1664
Ile Glu Val Leu Tyr Ile Arg Asn Val Thr Phe Glu Asp Ala Gly Glu
    325                 330                 335
tat acg tgc ttg gcg ggt aat tct att ggg ata tcc ttt cac tct gca      1712
Tyr Thr Cys Leu Ala Gly Asn Ser Ile Gly Ile Ser Phe His Ser Ala
340                 345                 350                 355
tgg ttg aca gtt ctg cca gcg cct gga aga gaa aag gag att aca gct      1760
Trp Leu Thr Val Leu Pro Ala Pro Gly Arg Glu Lys Glu Ile Thr Ala
                360                 365                 370
tcc cca gac tac ctg gag ata gcc att tac tgc ata ggg gtc ttc tta      1808
Ser Pro Asp Tyr Leu Glu Ile Ala Ile Tyr Cys Ile Gly Val Phe Leu
            375                 380                 385
atc gcc tgt atg gtg gta aca gtc atc ctg tgc cga atg aag aac acg      1856
Ile Ala Cys Met Val Val Thr Val Ile Leu Cys Arg Met Lys Asn Thr
        390                 395                 400
acc aag aag cca gac ttc agc agc cag ccg gct gtg cac aag ctg acc      1904
Thr Lys Lys Pro Asp Phe Ser Ser Gln Pro Ala Val His Lys Leu Thr
    405                 410                 415
aaa cgt atc ccc ctg cgg aga cag gta aca gtt tcg gct gag tcc agc      1952
Lys Arg Ile Pro Leu Arg Arg Gln Val Thr Val Ser Ala Glu Ser Ser
420                 425                 430                 435
tcc tcc atg aac tcc aac acc ccg ctg gtg agg ata aca aca cgc ctc      2000
Ser Ser Met Asn Ser Asn Thr Pro Leu Val Arg Ile Thr Thr Arg Leu
                440                 445                 450
tct tca acg gca gac acc ccc atg ctg gca ggg gtc tcc gag tat gaa      2048
Ser Ser Thr Ala Asp Thr Pro Met Leu Ala Gly Val Ser Glu Tyr Glu
            455                 460                 465
ctt cca gag gac cca aaa tgg gag ttt cca aga gat aag ctg aca ctg      2096
Leu Pro Glu Asp Pro Lys Trp Glu Phe Pro Arg Asp Lys Leu Thr Leu
        470                 475                 480
ggc aag ccc ctg gga gaa ggt tgc ttt ggg caa gtg gtc atg gcg gaa      2144
Gly Lys Pro Leu Gly Glu Gly Cys Phe Gly Gln Val Val Met Ala Glu
    485                 490                 495
gca gtg gga att gac aaa gac aag ccc aag gag gcg gtc acc gtg gcc      2192
Ala Val Gly Ile Asp Lys Asp Lys Pro Lys Glu Ala Val Thr Val Ala
500                 505                 510                 515
gtg aag atg ttg aaa gat gat gcc aca gag aaa gac ctt tct gat ctg      2240
Val Lys Met Leu Lys Asp Asp Ala Thr Glu Lys Asp Leu Ser Asp Leu
                520                 525                 530
gtg tca gag atg gag atg atg aag atg att ggg aaa cac aag aat atc      2288
Val Ser Glu Met Glu Met Met Lys Met Ile Gly Lys His Lys Asn Ile
            535                 540                 545
ata aat ctt ctt gga gcc tgc aca cag gat ggg cct ctc tat gtc ata      2336
Ile Asn Leu Leu Gly Ala Cys Thr Gln Asp Gly Pro Leu Tyr Val Ile
        550                 555                 560
gtt gag tat gcc tct aaa ggc aac ctc cga gaa tac ctc cga gcc cgg      2384
Val Glu Tyr Ala Ser Lys Gly Asn Leu Arg Glu Tyr Leu Arg Ala Arg
    565                 570                 575
agg cca ccc ggg atg gag tac tcc tat gac att aac cgt gtt cct gag      2432
Arg Pro Pro Gly Met Glu Tyr Ser Tyr Asp Ile Asn Arg Val Pro Glu
580                 585                 590                 595
gag cag atg acc ttc aag gac ttg gtg tca tgc acc tac cag ctg gcc      2480

Glu Gln Met Thr Phe Lys Asp Leu Val Ser Cys Thr Tyr Gln Leu Ala
                600                 605                 610

aga ggc atg gag tac ttg gct tcc caa aaa tgt att cat cga gat tta      2528
Arg Gly Met Glu Tyr Leu Ala Ser Gln Lys Cys Ile His Arg Asp Leu
            615                 620                 625

gca gcc aga aat gtt ttg gta aca gaa aac aat gtg atg aaa ata gca      2576
Ala Ala Arg Asn Val Leu Val Thr Glu Asn Asn Val Met Lys Ile Ala
        630                 635                 640

gac ttt gga ctc gcc aga gat atc aac aat ata gac tat tac aaa aag      2624
Asp Phe Gly Leu Ala Arg Asp Ile Asn Asn Ile Asp Tyr Tyr Lys Lys
    645                 650                 655

acc acc aat ggg cgg ctt cca gtc aag tgg atg gct cca gaa gcc ctg      2672
Thr Thr Asn Gly Arg Leu Pro Val Lys Trp Met Ala Pro Glu Ala Leu
660                 665                 670                 675

ttt gat aga gta tac act cat cag agt gat gtc tgg tcc ttc ggg gtg      2720
Phe Asp Arg Val Tyr Thr His Gln Ser Asp Val Trp Ser Phe Gly Val
                680                 685                 690

tta atg tgg gag atc ttc act tta ggg ggc tcg ccc tac cca ggg att      2768
Leu Met Trp Glu Ile Phe Thr Leu Gly Gly Ser Pro Tyr Pro Gly Ile
            695                 700                 705

ccc gtg gag gaa ctt ttt aag ctg ctg aag gaa gga cac aga atg gat      2816
Pro Val Glu Glu Leu Phe Lys Leu Leu Lys Glu Gly His Arg Met Asp
        710                 715                 720

aag cca gcc aac tgc acc aac gaa ctg tac atg atg atg agg gac tgt      2864
Lys Pro Ala Asn Cys Thr Asn Glu Leu Tyr Met Met Met Arg Asp Cys
    725                 730                 735

tgg cat gca gtg ccc tcc cag aga cca acg ttc aag cag ttg gta gaa      2912
Trp His Ala Val Pro Ser Gln Arg Pro Thr Phe Lys Gln Leu Val Glu
740                 745                 750                 755

gac ttg gat cga att ctc act ctc aca acc aat gag atc atg gag gaa      2960
Asp Leu Asp Arg Ile Leu Thr Leu Thr Thr Asn Glu Ile Met Glu Glu
                760                 765                 770

aca aat acg cag att gct tgg cca tca aaa ctg aag atc gga gcc aaa      3008
Thr Asn Thr Gln Ile Ala Trp Pro Ser Lys Leu Lys Ile Gly Ala Lys
            775                 780                 785

tcc aag aaa gat ccc cat att aag gtt tct gga aag aaa gaa gat gtt      3056
Ser Lys Lys Asp Pro His Ile Lys Val Ser Gly Lys Lys Glu Asp Val
        790                 795                 800

aaa gaa gcc aag gaa atg atc atg tct gtc tta gac aca aaa agc aat      3104
Lys Glu Ala Lys Glu Met Ile Met Ser Val Leu Asp Thr Lys Ser Asn
    805                 810                 815

cga gtc aca ctg aag atg gat gtt tca cat aca gaa cat tca cat gta      3152
Arg Val Thr Leu Lys Met Asp Val Ser His Thr Glu His Ser His Val
820                 825                 830                 835

atc ggc aaa ggt ggc aac aat att aaa aaa gtg atg gaa gaa acc gga      3200
Ile Gly Lys Gly Gly Asn Asn Ile Lys Lys Val Met Glu Glu Thr Gly
                840                 845                 850

tgc cat atc cac ttt cca gat tcc aac agg aat aac caa gca gaa aaa      3248
Cys His Ile His Phe Pro Asp Ser Asn Arg Asn Asn Gln Ala Glu Lys
            855                 860                 865

agc aac cag gta tct ata gcg gga caa cca gca gga gta gaa tct gcc      3296
Ser Asn Gln Val Ser Ile Ala Gly Gln Pro Ala Gly Val Glu Ser Ala
        870                 875                 880

cga gtt aga att cgg gag ctg ctt cct ttg gtg ctg atg ttt gag cta      3344
Arg Val Arg Ile Arg Glu Leu Leu Pro Leu Val Leu Met Phe Glu Leu
    885                 890                 895

cca att gct gga att ctt caa ccg gtt cct gat cct aat tcc ccc tct      3392
Pro Ile Ala Gly Ile Leu Gln Pro Val Pro Asp Pro Asn Ser Pro Ser
900                 905                 910                 915

att cag cat ata tca caa acg tac aat att tca gta tca ttt aaa cag      3440
Ile Gln His Ile Ser Gln Thr Tyr Asn Ile Ser Val Ser Phe Lys Gln
                920                 925                 930

cgt tcc cga atg tat ggt gct act gtc ata gta cga ggg tct cag aat      3488

Arg Ser Arg Met Tyr Gly Ala Thr Val Ile Val Arg Gly Ser Gln Asn
        935                 940                 945
aac act agt gct gtg aag gaa gga act gcc atg ctg tta gaa cat ctt      3536
Asn Thr Ser Ala Val Lys Glu Gly Thr Ala Met Leu Leu Glu His Leu
        950                 955                 960
gct ggg agc tta gca tca gct att cct gtg agc aca caa cta gat att      3584
Ala Gly Ser Leu Ala Ser Ala Ile Pro Val Ser Thr Gln Leu Asp Ile
    965                 970                 975
gca gct caa cat cat ctc ttt atg atg ggt cga aat ggg agc aac atc      3632
Ala Ala Gln His His Leu Phe Met Met Gly Arg Asn Gly Ser Asn Ile
980                 985                 990                 995
aaa cat atc atg cag  aga aca ggt gct cag  atc cac ttt cct gat      3677
Lys His Ile Met Gln  Arg Thr Gly Ala Gln  Ile His Phe Pro Asp
                1000                1005                1010
ccc agt aat cca caa  aag aaa tct acc gtc  tac ctc cag ggc acc      3722
Pro Ser Asn Pro Gln  Lys Lys Ser Thr Val  Tyr Leu Gln Gly Thr
                1015                1020                1025
att gag tct gtc tgt  ctt gca agg caa tat  ctc atg ggt tgt ctt      3767
Ile Glu Ser Val Cys  Leu Ala Arg Gln Tyr  Leu Met Gly Cys Leu
                1030                1035                1040
cct ctt gtg ttg atg  ttt gat atg aag gaa  gaa att gaa gta gat      3812
Pro Leu Val Leu Met  Phe Asp Met Lys Glu  Glu Ile Glu Val Asp
                1045                1050                1055
cca caa ttc att gcg  cag ttg atg gaa cag  ctt gat gtc ttc atc      3857
Pro Gln Phe Ile Ala  Gln Leu Met Glu Gln  Leu Asp Val Phe Ile
                1060                1065                1070
agt att aaa cca aag  ccc aaa cag cca agc  aag tct gtg att gtg      3902
Ser Ile Lys Pro Lys  Pro Lys Gln Pro Ser  Lys Ser Val Ile Val
                1075                1080                1085
aaa agt gtt gag cga  aat gcc tta aat atg  tat gaa gca agg aaa      3947
Lys Ser Val Glu Arg  Asn Ala Leu Asn Met  Tyr Glu Ala Arg Lys
                1090                1095                1100
tgt ctc ctc gga ctt  gaa agc agt ggg gtt  acc ata gca acc agt      3992
Cys Leu Leu Gly Leu  Glu Ser Ser Gly Val  Thr Ile Ala Thr Ser
                1105                1110                1115
cca tcc cca gca tcc  tgc cct gcc ggc ctg  gca tgt ccc agc ctg      4037
Pro Ser Pro Ala Ser  Cys Pro Ala Gly Leu  Ala Cys Pro Ser Leu
                1120                1125                1130
gat atc tta gct tca  gca ggc ctt gga ctc  act gga cta ggt ctt      4082
Asp Ile Leu Ala Ser  Ala Gly Leu Gly Leu  Thr Gly Leu Gly Leu
                1135                1140                1145
ttg gga ccc acc acc  tta tct ctg aac act  tca aca acc cca aac      4127
Leu Gly Pro Thr Thr  Leu Ser Leu Asn Thr  Ser Thr Thr Pro Asn
                1150                1155                1160
tca ctc ttg aat gct  ctt aat agc tca gtc  agt cct ttg caa agt      4172
Ser Leu Leu Asn Ala  Leu Asn Ser Ser Val  Ser Pro Leu Gln Ser
                1165                1170                1175
cca agt tct ggt aca  ccc agc ccc aca tta  tgg gca ccc cca ctt      4217
Pro Ser Ser Gly Thr  Pro Ser Pro Thr Leu  Trp Ala Pro Pro Leu
                1180                1185                1190
gct aat act tca agt  gcc aca ggt ttt tct  gct ata cca cac ctt      4262
Ala Asn Thr Ser Ser  Ala Thr Gly Phe Ser  Ala Ile Pro His Leu
                1195                1200                1205
atg att cca tct act  gcc caa gcc aca tta  act aat att ttg ttg      4307
Met Ile Pro Ser Thr  Ala Gln Ala Thr Leu  Thr Asn Ile Leu Leu
                1210                1215                1220
tct gga gtg ccc acc  tat ggg cac aca gct  cca tct ccc cct cct      4352
Ser Gly Val Pro Thr  Tyr Gly His Thr Ala  Pro Ser Pro Pro Pro
                1225                1230                1235
ggc ttg act cct gtt  gat gtc cat atc aac  agt atg cag acc gaa      4397
Gly Leu Thr Pro Val  Asp Val His Ile Asn  Ser Met Gln Thr Glu
                1240                1245                1250
ggc aaa aaa atc tct  gct gct tta aat gga  cat gca cag tct cca      4442

Gly Lys Lys Ile Ser Ala Ala Leu Asn Gly His Ala Gln Ser Pro
                1255                1260                1265
gat ata aaa tat ggt gca ata tcc act tca tca ctt gga gaa aaa 4487
Asp Ile Lys Tyr Gly Ala Ile Ser Thr Ser Ser Leu Gly Glu Lys
                1270                1275                1280
gtg ctg agt gca aat cac ggg gat ccg tcc atc cag aca agt ggg 4532
Val Leu Ser Ala Asn His Gly Asp Pro Ser Ile Gln Thr Ser Gly
                1285                1290                1295
tct gag cag aca tct ccc aaa tca agc ccc act gaa ggt tgt aat 4577
Ser Glu Gln Thr Ser Pro Lys Ser Ser Pro Thr Glu Gly Cys Asn
                1300                1305                1310
gat gct ttt gtt gaa gta ggc atg cct cga agt cct tcc cat tct 4622
Asp Ala Phe Val Glu Val Gly Met Pro Arg Ser Pro Ser His Ser
                1315                1320                1325
ggg aat gct ggt gac ttg aaa cag atg atg tgt ccc tcc aag gtt 4667
Gly Asn Ala Gly Asp Leu Lys Gln Met Met Cys Pro Ser Lys Val
                1330                1335                1340
tcc tgt gcc aaa agg cag aca gtg gaa cta ttg caa ggc acg aaa 4712
Ser Cys Ala Lys Arg Gln Thr Val Glu Leu Leu Gln Gly Thr Lys
                1345                1350                1355
aac tca cac tta cac agc act gac agg ttg ctc tca gac cct gaa 4757
Asn Ser His Leu His Ser Thr Asp Arg Leu Leu Ser Asp Pro Glu
                1360                1365                1370
ctg agt gct acc gaa agc cct ttg gct gac aag aag gct cca ggg 4802
Leu Ser Ala Thr Glu Ser Pro Leu Ala Asp Lys Lys Ala Pro Gly
                1375                1380                1385
agt gag cgc gct gca gag agg gca gca gct gcc cag caa aac tcc 4847
Ser Glu Arg Ala Ala Glu Arg Ala Ala Ala Ala Gln Gln Asn Ser
                1390                1395                1400
gaa agg gcc cac ctt gct cca cgg tca tca tat gtc aac atg cag 4892
Glu Arg Ala His Leu Ala Pro Arg Ser Ser Tyr Val Asn Met Gln
                1405                1410                1415
gca ttt gac tat gaa cag aag aag cta tta gcc acc aaa gct atg 4937
Ala Phe Asp Tyr Glu Gln Lys Lys Leu Leu Ala Thr Lys Ala Met
                1420                1425                1430
tta aag aaa cca gtg gtg acg gag gtc aga acg ccc aca aat acc 4982
Leu Lys Lys Pro Val Val Thr Glu Val Arg Thr Pro Thr Asn Thr
                1435                1440                1445
tgg agt ggc ctg ggt ttt tct aaa tcc atg cca gct gaa act atc 5027
Trp Ser Gly Leu Gly Phe Ser Lys Ser Met Pro Ala Glu Thr Ile
                1450                1455                1460
aag gag ttg aga agg gcc aat cat gtg tcc tat aag ccc aca atg 5072
Lys Glu Leu Arg Arg Ala Asn His Val Ser Tyr Lys Pro Thr Met
                1465                1470                1475
aca acc act tat gag ggc tca tcc atg tcc ctt tca cgg tcc aac 5117
Thr Thr Thr Tyr Glu Gly Ser Ser Met Ser Leu Ser Arg Ser Asn
                1480                1485                1490
agt cgt gag cac ttg gga ggt gga agc gaa tct gat aac tgg aga 5162
Ser Arg Glu His Leu Gly Gly Gly Ser Glu Ser Asp Asn Trp Arg
                1495                1500                1505
gac cga aat gga att gga cct gga agt cat agt gaa ttt gca gct 5207
Asp Arg Asn Gly Ile Gly Pro Gly Ser His Ser Glu Phe Ala Ala
                1510                1515                1520
tct att ggc agc cct aag cgt aaa caa aac aaa tca acg gaa cac 5252
Ser Ile Gly Ser Pro Lys Arg Lys Gln Asn Lys Ser Thr Glu His
                1525                1530                1535
tat ctc agc agt agc aat tac atg gac tgc att tcc tcg ctg aca 5297
Tyr Leu Ser Ser Ser Asn Tyr Met Asp Cys Ile Ser Ser Leu Thr
                1540                1545                1550
gga agc aat ggc tgt aac tta aat agc tct ttc aaa ggt tct gac 5342
Gly Ser Asn Gly Cys Asn Leu Asn Ser Ser Phe Lys Gly Ser Asp
                1555                1560                1565
ctc cct gag ctc ttc agc aaa ctg ggc ctg ggc aaa tac aca gat 5387

Leu Pro Glu Leu Phe Ser Lys Leu Gly Leu Gly Lys Tyr Thr Asp
                1570                1575                1580
gtt ttc cag caa caa gag atc gat ctt cag aca ttc ctc act ctc 5432
Val Phe Gln Gln Gln Glu Ile Asp Leu Gln Thr Phe Leu Thr Leu
                1585                1590                1595
aca gat cag gat ctg aag gag ctg gga ata act act ttt ggt gcc 5477
Thr Asp Gln Asp Leu Lys Glu Leu Gly Ile Thr Thr Phe Gly Ala
                1600                1605                1610
agg agg aaa atg ctg ctt gca att tca gaa cta aat aaa aac cga 5522
Arg Arg Lys Met Leu Leu Ala Ile Ser Glu Leu Asn Lys Asn Arg
                1615                1620                1625
aga aag ctt ttt gaa tcg cca aat gca cgc acc tct ttc ctg gaa 5567
Arg Lys Leu Phe Glu Ser Pro Asn Ala Arg Thr Ser Phe Leu Glu
                1630                1635                1640
ggt gga gcg agt gga agg cta ccc cgt cag tat cac tca gac att 5612
Gly Gly Ala Ser Gly Arg Leu Pro Arg Gln Tyr His Ser Asp Ile
                1645                1650                1655
gct agt gtc agt ggc cgc tgg tag cagcaccctc ttggcacatg cccgctgact 5666
Ala Ser Val Ser Gly Arg Trp
                1660
aactgtaaag tggacacagg agatgtatga acagccttca cagcacacca tccttagcac 5726
tctgggtgtc tggtatcagg accaaagcat tttattcgca cctgtacttt atggcaaaaa 5786
ggaagaagag agagaagatg ttcttatgat gtcatacaga acac 5830

<210> 8
<211> 1662
<212> PRT
<213> Homo sapiens
<400> 8
Met Val Ser Trp Gly Arg Phe Ile Cys Leu Val Val Val Thr Met Ala
1               5                   10                  15
Thr Leu Ser Leu Ala Arg Pro Ser Phe Ser Leu Val Glu Asp Thr Thr
            20                  25                  30
Leu Glu Pro Glu Glu Pro Pro Thr Lys Tyr Gln Ile Ser Gln Pro Glu
        35                  40                  45
Val Tyr Val Ala Ala Pro Gly Glu Ser Leu Glu Val Arg Cys Leu Leu
    50                  55                  60
Lys Asp Ala Ala Val Ile Ser Trp Thr Lys Asp Gly Val His Leu Gly
65                  70                  75                  80
Pro Asn Asn Arg Thr Val Leu Ile Gly Glu Tyr Leu Gln Ile Lys Gly
                85                  90                  95
Ala Thr Pro Arg Asp Ser Gly Leu Tyr Ala Cys Thr Ala Ser Arg Thr
            100                 105                 110
Val Asp Ser Glu Thr Trp Tyr Phe Met Val Asn Val Thr Asp Ala Ile
        115                 120                 125
Ser Ser Gly Asp Asp Glu Asp Asp Thr Asp Gly Ala Glu Asp Phe Val
    130                 135                 140
Ser Glu Asn Ser Asn Asn Lys Arg Ala Pro Tyr Trp Thr Asn Thr Glu
145                 150                 155                 160
Lys Met Glu Lys Arg Leu His Ala Val Pro Ala Ala Asn Thr Val Lys
                165                 170                 175
Phe Arg Cys Pro Ala Gly Gly Asn Pro Met Pro Thr Met Arg Trp Leu
            180                 185                 190
Lys Asn Gly Lys Glu Phe Lys Gln Glu His Arg Ile Gly Gly Tyr Lys
        195                 200                 205
Val Arg Asn Gln His Trp Ser Leu Ile Met Glu Ser Val Val Pro Ser
    210                 215                 220
Asp Lys Gly Asn Tyr Thr Cys Val Val Glu Asn Glu Tyr Gly Ser Ile
225                 230                 235                 240
Asn His Thr Tyr His Leu Asp Val Val Glu Arg Ser Pro His Arg Pro
                245                 250                 255
Ile Leu Gln Ala Gly Leu Pro Ala Asn Ala Ser Thr Val Val Gly Gly
            260                 265                 270

Asp Val Glu Phe Val Cys Lys Val Tyr Ser Asp Ala Gln Pro His Ile
        275                 280                 285
Gln Trp Ile Lys His Val Glu Lys Asn Gly Ser Lys Tyr Gly Pro Asp
    290                 295                 300
Gly Leu Pro Tyr Leu Lys Val Leu Lys Ala Ala Gly Val Asn Thr Thr
305                 310                 315                 320
Asp Lys Glu Ile Glu Val Leu Tyr Ile Arg Asn Val Thr Phe Glu Asp
                325                 330                 335
Ala Gly Glu Tyr Thr Cys Leu Ala Gly Asn Ser Ile Gly Ile Ser Phe
            340                 345                 350
His Ser Ala Trp Leu Thr Val Leu Pro Ala Pro Gly Arg Glu Lys Glu
        355                 360                 365
Ile Thr Ala Ser Pro Asp Tyr Leu Glu Ile Ala Ile Tyr Cys Ile Gly
    370                 375                 380
Val Phe Leu Ile Ala Cys Met Val Val Thr Val Ile Leu Cys Arg Met
385                 390                 395                 400
Lys Asn Thr Thr Lys Lys Pro Asp Phe Ser Ser Gln Pro Ala Val His
                405                 410                 415
Lys Leu Thr Lys Arg Ile Pro Leu Arg Arg Gln Val Thr Val Ser Ala
            420                 425                 430
Glu Ser Ser Ser Ser Met Asn Ser Asn Thr Pro Leu Val Arg Ile Thr
        435                 440                 445
Thr Arg Leu Ser Ser Thr Ala Asp Thr Pro Met Leu Ala Gly Val Ser
    450                 455                 460
Glu Tyr Glu Leu Pro Glu Asp Pro Lys Trp Glu Phe Pro Arg Asp Lys
465                 470                 475                 480
Leu Thr Leu Gly Lys Pro Leu Gly Glu Gly Cys Phe Gly Gln Val Val
                485                 490                 495
Met Ala Glu Ala Val Gly Ile Asp Lys Asp Lys Pro Lys Glu Ala Val
            500                 505                 510
Thr Val Ala Val Lys Met Leu Lys Asp Asp Ala Thr Glu Lys Asp Leu
        515                 520                 525
Ser Asp Leu Val Ser Glu Met Glu Met Met Lys Met Ile Gly Lys His
    530                 535                 540
Lys Asn Ile Ile Asn Leu Leu Gly Ala Cys Thr Gln Asp Gly Pro Leu
545                 550                 555                 560
Tyr Val Ile Val Glu Tyr Ala Ser Lys Gly Asn Leu Arg Glu Tyr Leu
                565                 570                 575
Arg Ala Arg Arg Pro Pro Gly Met Glu Tyr Ser Tyr Asp Ile Asn Arg
            580                 585                 590
Val Pro Glu Glu Gln Met Thr Phe Lys Asp Leu Val Ser Cys Thr Tyr
        595                 600                 605
Gln Leu Ala Arg Gly Met Glu Tyr Leu Ala Ser Gln Lys Cys Ile His
    610                 615                 620
Arg Asp Leu Ala Ala Arg Asn Val Leu Val Thr Glu Asn Asn Val Met
625                 630                 635                 640
Lys Ile Ala Asp Phe Gly Leu Ala Arg Asp Ile Asn Asn Ile Asp Tyr
                645                 650                 655
Tyr Lys Lys Thr Thr Asn Gly Arg Leu Pro Val Lys Trp Met Ala Pro
            660                 665                 670
Glu Ala Leu Phe Asp Arg Val Tyr Thr His Gln Ser Asp Val Trp Ser
        675                 680                 685
Phe Gly Val Leu Met Trp Glu Ile Phe Thr Leu Gly Gly Ser Pro Tyr
    690                 695                 700
Pro Gly Ile Pro Val Glu Glu Leu Phe Lys Leu Leu Lys Glu Gly His
705                 710                 715                 720
Arg Met Asp Lys Pro Ala Asn Cys Thr Asn Glu Leu Tyr Met Met Met
                725                 730                 735
Arg Asp Cys Trp His Ala Val Pro Ser Gln Arg Pro Thr Phe Lys Gln
            740                 745                 750
Leu Val Glu Asp Leu Asp Arg Ile Leu Thr Leu Thr Thr Asn Glu Ile
        755                 760                 765
Met Glu Glu Thr Asn Thr Gln Ile Ala Trp Pro Ser Lys Leu Lys Ile

770                 775                 780
Gly Ala Lys Ser Lys Lys Asp Pro His Ile Lys Val Ser Gly Lys Lys
785                 790                 795                 800
Glu Asp Val Lys Glu Ala Lys Glu Met Ile Met Ser Val Leu Asp Thr
                805                 810                 815
Lys Ser Asn Arg Val Thr Leu Lys Met Asp Val Ser His Thr Glu His
            820                 825                 830
Ser His Val Ile Gly Lys Gly Gly Asn Asn Ile Lys Lys Val Met Glu
        835                 840                 845
Glu Thr Gly Cys His Ile His Phe Pro Asp Ser Asn Arg Asn Asn Gln
    850                 855                 860
Ala Glu Lys Ser Asn Gln Val Ser Ile Ala Gly Gln Pro Ala Gly Val
865                 870                 875                 880
Glu Ser Ala Arg Val Arg Ile Arg Glu Leu Leu Pro Leu Val Leu Met
                885                 890                 895
Phe Glu Leu Pro Ile Ala Gly Ile Leu Gln Pro Val Pro Asp Pro Asn
            900                 905                 910
Ser Pro Ser Ile Gln His Ile Ser Gln Thr Tyr Asn Ile Ser Val Ser
        915                 920                 925
Phe Lys Gln Arg Ser Arg Met Tyr Gly Ala Thr Val Ile Val Arg Gly
    930                 935                 940
Ser Gln Asn Asn Thr Ser Ala Val Lys Glu Gly Thr Ala Met Leu Leu
945                 950                 955                 960
Glu His Leu Ala Gly Ser Leu Ala Ser Ala Ile Pro Val Ser Thr Gln
                965                 970                 975
Leu Asp Ile Ala Ala Gln His His Leu Phe Met Met Gly Arg Asn Gly
            980                 985                 990
Ser Asn Ile Lys His Ile Met Gln  Arg Thr Gly Ala Gln  Ile His Phe
        995                 1000                1005
Pro Asp  Pro Ser Asn Pro Gln  Lys Lys Ser Thr Val  Tyr Leu Gln
    1010                1015                1020
Gly Thr  Ile Glu Ser Val Cys  Leu Ala Arg Gln Tyr  Leu Met Gly
    1025                1030                1035
Cys Leu  Pro Leu Val Leu Met  Phe Asp Met Lys Glu  Glu Ile Glu
    1040                1045                1050
Val Asp  Pro Gln Phe Ile Ala  Gln Leu Met Glu Gln  Leu Asp Val
    1055                1060                1065
Phe Ile  Ser Ile Lys Pro Lys  Pro Lys Gln Pro Ser  Lys Ser Val
    1070                1075                1080
Ile Val  Lys Ser Val Glu Arg  Asn Ala Leu Asn Met  Tyr Glu Ala
    1085                1090                1095
Arg Lys  Cys Leu Leu Gly Leu  Glu Ser Ser Gly Val  Thr Ile Ala
    1100                1105                1110
Thr Ser  Pro Ser Pro Ala Ser  Cys Pro Ala Gly Leu  Ala Cys Pro
    1115                1120                1125
Ser Leu  Asp Ile Leu Ala Ser  Ala Gly Leu Gly Leu  Thr Gly Leu
    1130                1135                1140
Gly Leu  Leu Gly Pro Thr Thr  Leu Ser Leu Asn Thr  Ser Thr Thr
    1145                1150                1155
Pro Asn  Ser Leu Leu Asn Ala  Leu Asn Ser Ser Val  Ser Pro Leu
    1160                1165                1170
Gln Ser  Pro Ser Ser Gly Thr  Pro Ser Pro Thr Leu  Trp Ala Pro
    1175                1180                1185
Pro Leu  Ala Asn Thr Ser Ser  Ala Thr Gly Phe Ser  Ala Ile Pro
    1190                1195                1200
His Leu  Met Ile Pro Ser Thr  Ala Gln Ala Thr Leu  Thr Asn Ile
    1205                1210                1215
Leu Leu  Ser Gly Val Pro Thr  Tyr Gly His Thr Ala  Pro Ser Pro
    1220                1225                1230
Pro Pro  Gly Leu Thr Pro Val  Asp Val His Ile Asn  Ser Met Gln
    1235                1240                1245
Thr Glu  Gly Lys Lys Ile Ser  Ala Ala Leu Asn Gly  His Ala Gln
    1250                1255                1260

Ser Pro Asp Ile Lys Tyr Gly Ala Ile Ser Thr Ser Ser Leu Gly
    1265                1270                1275
Glu Lys Val Leu Ser Ala Asn His Gly Asp Pro Ser Ile Gln Thr
    1280                1285                1290
Ser Gly Ser Glu Gln Thr Ser Pro Lys Ser Ser Pro Thr Glu Gly
    1295                1300                1305
Cys Asn Asp Ala Phe Val Glu Val Gly Met Pro Arg Ser Pro Ser
    1310                1315                1320
His Ser Gly Asn Ala Gly Asp Leu Lys Gln Met Met Cys Pro Ser
    1325                1330                1335
Lys Val Ser Cys Ala Lys Arg Gln Thr Val Glu Leu Leu Gln Gly
    1340                1345                1350
Thr Lys Asn Ser His Leu His Ser Thr Asp Arg Leu Leu Ser Asp
    1355                1360                1365
Pro Glu Leu Ser Ala Thr Glu Ser Pro Leu Ala Asp Lys Lys Ala
    1370                1375                1380
Pro Gly Ser Glu Arg Ala Ala Glu Arg Ala Ala Ala Ala Gln Gln
    1385                1390                1395
Asn Ser Glu Arg Ala His Leu Ala Pro Arg Ser Ser Tyr Val Asn
    1400                1405                1410
Met Gln Ala Phe Asp Tyr Glu Gln Lys Lys Leu Leu Ala Thr Lys
    1415                1420                1425
Ala Met Leu Lys Lys Pro Val Val Thr Glu Val Arg Thr Pro Thr
    1430                1435                1440
Asn Thr Trp Ser Gly Leu Gly Phe Ser Lys Ser Met Pro Ala Glu
    1445                1450                1455
Thr Ile Lys Glu Leu Arg Arg Ala Asn His Val Ser Tyr Lys Pro
    1460                1465                1470
Thr Met Thr Thr Thr Tyr Glu Gly Ser Ser Met Ser Leu Ser Arg
    1475                1480                1485
Ser Asn Ser Arg Glu His Leu Gly Gly Gly Ser Glu Ser Asp Asn
    1490                1495                1500
Trp Arg Asp Arg Asn Gly Ile Gly Pro Gly Ser His Ser Glu Phe
    1505                1510                1515
Ala Ala Ser Ile Gly Ser Pro Lys Arg Lys Gln Asn Lys Ser Thr
    1520                1525                1530
Glu His Tyr Leu Ser Ser Ser Asn Tyr Met Asp Cys Ile Ser Ser
    1535                1540                1545
Leu Thr Gly Ser Asn Gly Cys Asn Leu Asn Ser Ser Phe Lys Gly
    1550                1555                1560
Ser Asp Leu Pro Glu Leu Phe Ser Lys Leu Gly Leu Gly Lys Tyr
    1565                1570                1575
Thr Asp Val Phe Gln Gln Gln Glu Ile Asp Leu Gln Thr Phe Leu
    1580                1585                1590
Thr Leu Thr Asp Gln Asp Leu Lys Glu Leu Gly Ile Thr Thr Phe
    1595                1600                1605
Gly Ala Arg Arg Lys Met Leu Leu Ala Ile Ser Glu Leu Asn Lys
    1610                1615                1620
Asn Arg Arg Lys Leu Phe Glu Ser Pro Asn Ala Arg Thr Ser Phe
    1625                1630                1635
Leu Glu Gly Gly Ala Ser Gly Arg Leu Pro Arg Gln Tyr His Ser
    1640                1645                1650
Asp Ile Ala Ser Val Ser Gly Arg Trp
    1655                1660

<210> 9
<211> 6488
<212> DNA
<213> Homo sapiens
<220>
<221> CDS
<222> (648)..(4421)
<223>

<400> 9
ggcggcggct ggaggagagc gcggtggaga gccgagcggg cgggcggcgg gtgcggagcg 60
ggcgagggag cgcgcgcggc cgccacaaag ctcgggcgcc gcggggctgc atgcggcgta 120
cctggcccgg cgcggcgact gctctccggg ctggcggggg ccggccgcga gccccggggg 180
ccccgaggcc gcagcttgcc tgcgcgctct gagccttcgc aactcgcgag caaagtttgg 240
tggaggcaac gccaagcctg agtcctttct tcctctcgtt ccccaaatcc gagggcagcc 300
cgcgggcgtc atgcccgcgc tcctccgcag cctggggtac gcgtgaagcc cgggaggctt 360
ggcgccggcg aagacccaag gaccactctt ctgcgtttgg agttgctccc cgcaaccccg 420
ggctcgtcgc tttctccatc ccgacccacg cggggcgcgg ggacaacaca ggtcgcggag 480
gagcgttgcc attcaagtga ctgcagcagc agcggcagcg cctcggttcc tgagcccacc 540
gcaggctgaa ggcattgcgc gtagtccatg cccgtagagg aagtgtgcag atgggattaa 600
cgtccacatg gagatatgga agaggaccgg ggattggtac cgtaacc atg gtc agc 656
Met Val Ser
1
tgg ggt cgt ttc atc tgc ctg gtc gtg gtc acc atg gca acc ttg tcc 704
Trp Gly Arg Phe Ile Cys Leu Val Val Val Thr Met Ala Thr Leu Ser
    5                   10                  15
ctg gcc cgg ccc tcc ttc agt tta gtt gag gat acc aca tta gag cca 752
Leu Ala Arg Pro Ser Phe Ser Leu Val Glu Asp Thr Thr Leu Glu Pro
20                  25                  30                  35
gaa gag cca cca acc aaa tac caa atc tct caa cca gaa gtg tac gtg 800
Glu Glu Pro Pro Thr Lys Tyr Gln Ile Ser Gln Pro Glu Val Tyr Val
                40                  45                  50
gct gcg cca ggg gag tcg cta gag gtg cgc tgc ctg ttg aaa gat gcc 848
Ala Ala Pro Gly Glu Ser Leu Glu Val Arg Cys Leu Leu Lys Asp Ala
            55                  60                  65
gcc gtg atc agt tgg act aag gat ggg gtg cac ttg ggg ccc aac aat 896
Ala Val Ile Ser Trp Thr Lys Asp Gly Val His Leu Gly Pro Asn Asn
        70                  75                  80
agg aca gtg ctt att ggg gag tac ttg cag ata aag ggc gcc acg cct 944
Arg Thr Val Leu Ile Gly Glu Tyr Leu Gln Ile Lys Gly Ala Thr Pro
    85                  90                  95
aga gac tcc ggc ctc tat gct tgt act gcc agt agg act gta gac agt 992
Arg Asp Ser Gly Leu Tyr Ala Cys Thr Ala Ser Arg Thr Val Asp Ser
100                 105                 110                 115
gaa act tgg tac ttc atg gtg aat gtc aca gat gcc atc tca tcc gga 1040
Glu Thr Trp Tyr Phe Met Val Asn Val Thr Asp Ala Ile Ser Ser Gly
                120                 125                 130
gat gat gag gat gac acc gat ggt gcg gaa gat ttt gtc agt gag aac 1088
Asp Asp Glu Asp Asp Thr Asp Gly Ala Glu Asp Phe Val Ser Glu Asn
            135                 140                 145
agt aac aac aag aga gca cca tac tgg acc aac aca gaa aag atg gaa 1136
Ser Asn Asn Lys Arg Ala Pro Tyr Trp Thr Asn Thr Glu Lys Met Glu
        150                 155                 160
aag cgg ctc cat gct gtg cct gcg gcc aac act gtc aag ttt cgc tgc 1184
Lys Arg Leu His Ala Val Pro Ala Ala Asn Thr Val Lys Phe Arg Cys
    165                 170                 175
cca gcc ggg ggg aac cca atg cca acc atg cgg tgg ctg aaa aac ggg 1232
Pro Ala Gly Gly Asn Pro Met Pro Thr Met Arg Trp Leu Lys Asn Gly
180                 185                 190                 195
aag gag ttt aag cag gag cat cgc att gga ggc tac aag gta cga aac 1280
Lys Glu Phe Lys Gln Glu His Arg Ile Gly Gly Tyr Lys Val Arg Asn
                200                 205                 210
cag cac tgg agc ctc att atg gaa agt gtg gtc cca tct gac aag gga 1328
Gln His Trp Ser Leu Ile Met Glu Ser Val Val Pro Ser Asp Lys Gly
            215                 220                 225
aat tat acc tgt gta gtg gag aat gaa tac ggg tcc atc aat cac acg 1376
Asn Tyr Thr Cys Val Val Glu Asn Glu Tyr Gly Ser Ile Asn His Thr
        230                 235                 240
tac cac ctg gat gtt gtg gag cga tcg cct cac cgg ccc atc ctc caa 1424
Tyr His Leu Asp Val Val Glu Arg Ser Pro His Arg Pro Ile Leu Gln
    245                 250                 255
gcc gga ctg ccg gca aat gcc tcc aca gtg gtc gga gga gac gta gag 1472

Ala Gly Leu Pro Ala Asn Ala Ser Thr Val Val Gly Gly Asp Val Glu
260                 265                 270                 275
ttt gtc tgc aag gtt tac agt gat gcc cag ccc cac atc cag tgg atc      1520
Phe Val Cys Lys Val Tyr Ser Asp Ala Gln Pro His Ile Gln Trp Ile
                280                 285                 290
aag cac gtg gaa aag aac ggc agt aaa tac ggg ccc gac ggg ctg ccc      1568
Lys His Val Glu Lys Asn Gly Ser Lys Tyr Gly Pro Asp Gly Leu Pro
            295                 300                 305
tac ctc aag gtt ctc aag gcc gcc ggt gtt aac acc acg gac aaa gag      1616
Tyr Leu Lys Val Leu Lys Ala Ala Gly Val Asn Thr Thr Asp Lys Glu
        310                 315                 320
att gag gtt ctc tat att cgg aat gta act ttt gag gac gct ggg gaa      1664
Ile Glu Val Leu Tyr Ile Arg Asn Val Thr Phe Glu Asp Ala Gly Glu
    325                 330                 335
tat acg tgc ttg gcg ggt aat tct att ggg ata tcc ttt cac tct gca      1712
Tyr Thr Cys Leu Ala Gly Asn Ser Ile Gly Ile Ser Phe His Ser Ala
340                 345                 350                 355
tgg ttg aca gtt ctg cca gcg cct gga aga gaa aag gag att aca gct      1760
Trp Leu Thr Val Leu Pro Ala Pro Gly Arg Glu Lys Glu Ile Thr Ala
                360                 365                 370
tcc cca gac tac ctg gag ata gcc att tac tgc ata ggg gtc ttc tta      1808
Ser Pro Asp Tyr Leu Glu Ile Ala Ile Tyr Cys Ile Gly Val Phe Leu
            375                 380                 385
atc gcc tgt atg gtg gta aca gtc atc ctg tgc cga atg aag aac acg      1856
Ile Ala Cys Met Val Val Thr Val Ile Leu Cys Arg Met Lys Asn Thr
        390                 395                 400
acc aag aag cca gac ttc agc agc cag ccg gct gtg cac aag ctg acc      1904
Thr Lys Lys Pro Asp Phe Ser Ser Gln Pro Ala Val His Lys Leu Thr
    405                 410                 415
aaa cgt atc ccc ctg cgg aga cag gta aca gtt tcg gct gag tcc agc      1952
Lys Arg Ile Pro Leu Arg Arg Gln Val Thr Val Ser Ala Glu Ser Ser
420                 425                 430                 435
tcc tcc atg aac tcc aac acc ccg ctg gtg agg ata aca aca cgc ctc      2000
Ser Ser Met Asn Ser Asn Thr Pro Leu Val Arg Ile Thr Thr Arg Leu
                440                 445                 450
tct tca acg gca gac acc ccc atg ctg gca ggg gtc tcc gag tat gaa      2048
Ser Ser Thr Ala Asp Thr Pro Met Leu Ala Gly Val Ser Glu Tyr Glu
            455                 460                 465
ctt cca gag gac cca aaa tgg gag ttt cca aga gat aag ctg aca ctg      2096
Leu Pro Glu Asp Pro Lys Trp Glu Phe Pro Arg Asp Lys Leu Thr Leu
        470                 475                 480
ggc aag ccc ctg gga gaa ggt tgc ttt ggg caa gtg gtc atg gcg gaa      2144
Gly Lys Pro Leu Gly Glu Gly Cys Phe Gly Gln Val Val Met Ala Glu
    485                 490                 495
gca gtg gga att gac aaa gac aag ccc aag gag gcg gtc acc gtg gcc      2192
Ala Val Gly Ile Asp Lys Asp Lys Pro Lys Glu Ala Val Thr Val Ala
500                 505                 510                 515
gtg aag atg ttg aaa gat gat gcc aca gag aaa gac ctt tct gat ctg      2240
Val Lys Met Leu Lys Asp Asp Ala Thr Glu Lys Asp Leu Ser Asp Leu
                520                 525                 530
gtg tca gag atg gag atg atg aag atg att ggg aaa cac aag aat atc      2288
Val Ser Glu Met Glu Met Met Lys Met Ile Gly Lys His Lys Asn Ile
            535                 540                 545
ata aat ctt ctt gga gcc tgc aca cag gat ggg cct ctc tat gtc ata      2336
Ile Asn Leu Leu Gly Ala Cys Thr Gln Asp Gly Pro Leu Tyr Val Ile
        550                 555                 560
gtt gag tat gcc tct aaa ggc aac ctc cga gaa tac ctc cga gcc cgg      2384
Val Glu Tyr Ala Ser Lys Gly Asn Leu Arg Glu Tyr Leu Arg Ala Arg
    565                 570                 575
agg cca ccc ggg atg gag tac tcc tat gac att aac cgt gtt cct gag      2432
Arg Pro Pro Gly Met Glu Tyr Ser Tyr Asp Ile Asn Arg Val Pro Glu
580                 585                 590                 595
gag cag atg acc ttc aag gac ttg gtg tca tgc acc tac cag ctg gcc      2480

Glu Gln Met Thr Phe Lys Asp Leu Val Ser Cys Thr Tyr Gln Leu Ala
            600                 605                 610
aga ggc atg gag tac ttg gct tcc caa aaa tgt att cat cga gat tta      2528
Arg Gly Met Glu Tyr Leu Ala Ser Gln Lys Cys Ile His Arg Asp Leu
        615                 620                 625
gca gcc aga aat gtt ttg gta aca gaa aac aat gtg atg aaa ata gca      2576
Ala Ala Arg Asn Val Leu Val Thr Glu Asn Asn Val Met Lys Ile Ala
    630                 635                 640
gac ttt gga ctc gcc aga gat atc aac aat ata gac tat tac aaa aag      2624
Asp Phe Gly Leu Ala Arg Asp Ile Asn Asn Ile Asp Tyr Tyr Lys Lys
645                 650                 655
acc acc aat ggg cgg ctt cca gtc aag tgg atg gct cca gaa gcc ctg      2672
Thr Thr Asn Gly Arg Leu Pro Val Lys Trp Met Ala Pro Glu Ala Leu
660                 665                 670                 675
ttt gat aga gta tac act cat cag agt gat gtc tgg tcc ttc ggg gtg      2720
Phe Asp Arg Val Tyr Thr His Gln Ser Asp Val Trp Ser Phe Gly Val
                680                 685                 690
tta atg tgg gag atc ttc act tta ggg ggc tcg ccc tac cca ggg att      2768
Leu Met Trp Glu Ile Phe Thr Leu Gly Gly Ser Pro Tyr Pro Gly Ile
            695                 700                 705
ccc gtg gag gaa ctt ttt aag ctg ctg aag gaa gga cac aga atg gat      2816
Pro Val Glu Glu Leu Phe Lys Leu Leu Lys Glu Gly His Arg Met Asp
        710                 715                 720
aag cca gcc aac tgc acc aac gaa ctg tac atg atg atg agg gac tgt      2864
Lys Pro Ala Asn Cys Thr Asn Glu Leu Tyr Met Met Met Arg Asp Cys
    725                 730                 735
tgg cat gca gtg ccc tcc cag aga cca acg ttc aag cag ttg gta gaa      2912
Trp His Ala Val Pro Ser Gln Arg Pro Thr Phe Lys Gln Leu Val Glu
740                 745                 750                 755
gac ttg gat cga att ctc act ctc aca acc aat gag caa atc cag ttt      2960
Asp Leu Asp Arg Ile Leu Thr Leu Thr Thr Asn Glu Gln Ile Gln Phe
                760                 765                 770
gct gat gac atg cag gag ttc acc aaa ttc ccc acc aaa act ggc cga      3008
Ala Asp Asp Met Gln Glu Phe Thr Lys Phe Pro Thr Lys Thr Gly Arg
            775                 780                 785
aga tct ttg tct cgc tcg atc tca cag tcc tcc act gac agc tac agt      3056
Arg Ser Leu Ser Arg Ser Ile Ser Gln Ser Ser Thr Asp Ser Tyr Ser
        790                 795                 800
tca gct gca tcc tac aca gat agc tct gat gat gag gtt tct ccc cga      3104
Ser Ala Ala Ser Tyr Thr Asp Ser Ser Asp Asp Glu Val Ser Pro Arg
    805                 810                 815
gag aag cag caa acc aac tcc aag ggc agc agc aat ttc tgt gtg aag      3152
Glu Lys Gln Gln Thr Asn Ser Lys Gly Ser Ser Asn Phe Cys Val Lys
820                 825                 830                 835
aac atc aag cag gca gaa ttt gga cgc cgg gag att gag att gca gag      3200
Asn Ile Lys Gln Ala Glu Phe Gly Arg Arg Glu Ile Glu Ile Ala Glu
                840                 845                 850
caa gac atg tct gct ctg att tca ctc agg aaa cgt gct cag ggg gag      3248
Gln Asp Met Ser Ala Leu Ile Ser Leu Arg Lys Arg Ala Gln Gly Glu
            855                 860                 865
aag ccc ttg gct ggt gct aaa ata gtg ggc tgt aca cac atc aca gcc      3296
Lys Pro Leu Ala Gly Ala Lys Ile Val Gly Cys Thr His Ile Thr Ala
        870                 875                 880
cag aca gcg gtg ttg att gag aca ctc tgt gcc ctg ggg gct cag tgc      3344
Gln Thr Ala Val Leu Ile Glu Thr Leu Cys Ala Leu Gly Ala Gln Cys
    885                 890                 895
cgc tgg tct gct tgt aac atc tac tca act cag aat gaa gta gct gca      3392
Arg Trp Ser Ala Cys Asn Ile Tyr Ser Thr Gln Asn Glu Val Ala Ala
900                 905                 910                 915
gca ctg gct gag gct gga gtt gca gtg ttc gct tgg aag ggc gag tca      3440
Ala Leu Ala Glu Ala Gly Val Ala Val Phe Ala Trp Lys Gly Glu Ser
                920                 925                 930
gaa gat gac ttc tgg tgg tgt att gac cgc tgt gtg aac atg gat ggg      3488

Glu Asp Asp Phe Trp Trp Cys Ile Asp Arg Cys Val Asn Met Asp Gly
        935                 940                 945
tgg cag gcc aac atg atc ctg gat gat ggg gga gac tta acc cac tgg     3536
Trp Gln Ala Asn Met Ile Leu Asp Asp Gly Gly Asp Leu Thr His Trp
        950                 955                 960
gtt tat aag aag tat cca aac gtg ttt aag aag atc cga ggc att gtg     3584
Val Tyr Lys Lys Tyr Pro Asn Val Phe Lys Lys Ile Arg Gly Ile Val
    965                 970                 975
gaa gag agc gtg act ggt gtt cac agg ctg tat cag ctc tcc aaa gct     3632
Glu Glu Ser Val Thr Gly Val His Arg Leu Tyr Gln Leu Ser Lys Ala
980                 985                 990                 995
ggg aag ctc tgt gtt  ccg gcc atg aac gtc  aat gat tct gtt acc     3677
Gly Lys Leu Cys Val  Pro Ala Met Asn Val  Asn Asp Ser Val Thr
                1000                1005                1010
aaa cag aag ttt gat  aac ttg tac tgc tgc  cga gaa tcc att ttg     3722
Lys Gln Lys Phe Asp  Asn Leu Tyr Cys Cys  Arg Glu Ser Ile Leu
                1015                1020                1025
gat ggc ctg aag agg  acc aca gat gtg atg  ttt ggt ggg aaa caa     3767
Asp Gly Leu Lys Arg  Thr Thr Asp Val Met  Phe Gly Gly Lys Gln
                1030                1035                1040
gtg gtg gtg tgt ggc  tat ggt gag gta ggc  aag ggc tgc tgt gct     3812
Val Val Val Cys Gly  Tyr Gly Glu Val Gly  Lys Gly Cys Cys Ala
                1045                1050                1055
gct ctc aaa gct ctt  gga gca att gtc tac  att acc gaa atc gac     3857
Ala Leu Lys Ala Leu  Gly Ala Ile Val Tyr  Ile Thr Glu Ile Asp
                1060                1065                1070
ccc atc tgt gct ctg  cag gcc tgc atg gat  ggg ttc agg gtg gta     3902
Pro Ile Cys Ala Leu  Gln Ala Cys Met Asp  Gly Phe Arg Val Val
                1075                1080                1085
aag cta aat gaa gtc  atc cgg caa gtc gat  gtc gta ata act tgc     3947
Lys Leu Asn Glu Val  Ile Arg Gln Val Asp  Val Val Ile Thr Cys
                1090                1095                1100
aca gga aat aag aat  gta gtg aca cgg gag  cac ttg gat cgc atg     3992
Thr Gly Asn Lys Asn  Val Val Thr Arg Glu  His Leu Asp Arg Met
                1105                1110                1115
aaa aac agt tgt atc  gta tgc aat atg ggc  cac tcc aac aca gaa     4037
Lys Asn Ser Cys Ile  Val Cys Asn Met Gly  His Ser Asn Thr Glu
                1120                1125                1130
atc gat gtg acc agc  ctc cgc act ccg gag  ctg acg tgg gag cga     4082
Ile Asp Val Thr Ser  Leu Arg Thr Pro Glu  Leu Thr Trp Glu Arg
                1135                1140                1145
gta cgt tct cag gtg  gac cat gtc atc tgg  cca gat ggc aaa cga     4127
Val Arg Ser Gln Val  Asp His Val Ile Trp  Pro Asp Gly Lys Arg
                1150                1155                1160
gtt gtc ctc ctg gca  gag ggt cgt cta ctc  aat ttg agc tgc tcc     4172
Val Val Leu Leu Ala  Glu Gly Arg Leu Leu  Asn Leu Ser Cys Ser
                1165                1170                1175
aca gtt ccc acc ttt  gtt ctg tcc atc aca  gcc aca aca cag gct     4217
Thr Val Pro Thr Phe  Val Leu Ser Ile Thr  Ala Thr Thr Gln Ala
                1180                1185                1190
ttg gca ctg ata gaa  ctc tat aat gca ccc  gag ggg cga tac aag     4262
Leu Ala Leu Ile Glu  Leu Tyr Asn Ala Pro  Glu Gly Arg Tyr Lys
                1195                1200                1205
cag gat gtg tac ttg  ctt cct aag aaa atg  gat gaa tac gtt gcc     4307
Gln Asp Val Tyr Leu  Leu Pro Lys Lys Met  Asp Glu Tyr Val Ala
                1210                1215                1220
agc ttg cat ctg cca  tca ttt gat gcc cac  ctt aca gag ctg aca     4352
Ser Leu His Leu Pro  Ser Phe Asp Ala His  Leu Thr Glu Leu Thr
                1225                1230                1235
gat gac caa gca aaa  tat ctg gga ctc aac  aaa aat ggg cca ttc     4397
Asp Asp Gln Ala Lys  Tyr Leu Gly Leu Asn  Lys Asn Gly Pro Phe
                1240                1245                1250
aaa cct aat tat tac  aga tac taa tggaccatac taccaaggac cagtccacct     4451

Lys Pro Asn Tyr Tyr Arg Tyr
                1255
gaaccacaca ctctaaagaa atatttttta agataacttt tattttcttc ttactccttt 4511
cctcttgatt tttttcctat aatttcattc ttgttttttc atctcattat ccaagttctg 4571
cagaccacac aggaacttgc ttcatggctc tttagatgaa atagaagttc agggttcctc 4631
actctagtca ctaaagaagg attttactct cccagcccag aaaggtgatt ctttctttac 4691
catttctggg gactttagtc ttaattaggt accttattaa caggaaatgc taaggtacct 4751
tctctgtgga acaatctgca atgtctaaat cgccttaaaa gagcccattt cttagctgct 4811
gaaatcagtg ctctttcact tcttcagaga agcagggatg gtacctaccc ggcaggtagg 4871
ttagatgtgg gtggtgcatg ttaatttccc ttagaagttc caagccctgt ttcctgcgta 4931
aaggtggtat gtccagttca gagatgtgta taatgagcat ggcttgttaa gatcaggagg 4991
cccacttgga tttatagtat agcccttcct ccactcccac cagacttgct catttttcga 5051
gtttttaact agactacact ctattgagtt taattttgtc ctctaggatt tatttctgtt 5111
gtccaaaaaa aaaaaaaaag aaaagaaaaa ttaaggagaa tttttggtgt taatgctgag 5171
gaattgcttg agtggttagt tgttaccaat ttctcttttg aacctttgga gctaaggatg 5231
ctgagtctag agaaatgcta gtctcaagcc ctgttaagtc cctctgtttc tagcccgtag 5291
ttcatagcat cagtgaactg gagccacaac agcaaattct atcagctgtg taccatacag 5351
cttgtgctga aggcgaattt cttgagccat tactcagtat aaagcactga gttctatctt 5411
taggatttat ctttaagagc aaatttctgg tcagctgtgc ttctgcaacc taaaatattt 5471
aaagggaggt aggtgtgggc aggaggagga atgataaatt gggccagggc aagaaaaatc 5531
tagcttcata taatttgtct gggactatac accctatata atgttagttt tacagaagta 5591
atatgacttt tgattgctac ataccacaaa gagtttatga actgagatca taaagggcaa 5651
ctgatgtgtg aagaaagtag tcagtacatc ctggctcatg ctctgaaaga atatccagag 5711
aggctctctc aaagatcagg gagatgtatt cccatgccat gcaccctgct tcccagcatt 5771
tctgcatggt caagtgagct ttatgctcat gagctttaag tatataatta tccaggattt 5831
taaatcctca acttgttcta gcttgtgatc cctcaaagtt gggtcatacg ttagtgctag 5891
atactagaaa ttttcacttt tccactgatc agagagacag acattaaaaa caaaaataga 5951
agaaaggaaa gctttcaccc tgcagcttct tagcagggaa caattgtctt gccaaaactt 6011
ttttcccttt tctctcccat tttcttttac ccaatccctt cttactcctt gccagtgtga 6071
ccatgctttc ttctctgtag atgttaacag ttaaggccta ttttcctcgg gcacttaacc 6131
aaccaatcag aacaccacat ctgttagggg aggtaacctg gccaacagtg tatccatcac 6191
gttagccctg ctggagggaa gggacccaca ttcacctgcc ctctgacctg ccccttgatc 6251
ccatatctat taccgtgtcc ataggaataa taggtaaggg ctctgtctct gtcaagccat 6311
gtaacaaagg acactgttaa aaaaaaaaaa aagtctggca tcagagggag catgtggaga 6371
gcaacttggg aagaacaagt tcattttgta ttgaatgatt tttaatgaat gcaatattaa 6431
tccttgcaga tgagcaataa tcattaaaat cgattaaaat gataagacct taaaaaa 6488

<210> 10
<211> 1257
<212> PRT
<213> Homo sapiens
<400> 10
Met Val Ser Trp Gly Arg Phe Ile Cys Leu Val Val Val Thr Met Ala
1               5                   10                  15
Thr Leu Ser Leu Ala Arg Pro Ser Phe Ser Leu Val Glu Asp Thr Thr
            20                  25                  30
Leu Glu Pro Glu Glu Pro Pro Thr Lys Tyr Gln Ile Ser Gln Pro Glu
        35                  40                  45
Val Tyr Val Ala Ala Pro Gly Glu Ser Leu Glu Val Arg Cys Leu Leu
    50                  55                  60
Lys Asp Ala Ala Val Ile Ser Trp Thr Lys Asp Gly Val His Leu Gly
65                  70                  75                  80
Pro Asn Asn Arg Thr Val Leu Ile Gly Glu Tyr Leu Gln Ile Lys Gly
                85                  90                  95
Ala Thr Pro Arg Asp Ser Gly Leu Tyr Ala Cys Thr Ala Ser Arg Thr
            100                 105                 110
Val Asp Ser Glu Thr Trp Tyr Phe Met Val Asn Val Thr Asp Ala Ile
        115                 120                 125
Ser Ser Gly Asp Asp Glu Asp Asp Thr Asp Gly Ala Glu Asp Phe Val
    130                 135                 140
Ser Glu Asn Ser Asn Asn Lys Arg Ala Pro Tyr Trp Thr Asn Thr Glu
145                 150                 155                 160
Lys Met Glu Lys Arg Leu His Ala Val Pro Ala Ala Asn Thr Val Lys

                    165                 170                 175
Phe Arg Cys Pro Ala Gly Gly Asn Pro Met Pro Thr Met Arg Trp Leu
            180                 185                 190
Lys Asn Gly Lys Glu Phe Lys Gln Glu His Arg Ile Gly Gly Tyr Lys
        195                 200                 205
Val Arg Asn Gln His Trp Ser Leu Ile Met Glu Ser Val Val Pro Ser
    210                 215                 220
Asp Lys Gly Asn Tyr Thr Cys Val Val Glu Asn Glu Tyr Gly Ser Ile
225                 230                 235                 240
Asn His Thr Tyr His Leu Asp Val Val Glu Arg Ser Pro His Arg Pro
                245                 250                 255
Ile Leu Gln Ala Gly Leu Pro Ala Asn Ala Ser Thr Val Val Gly Gly
            260                 265                 270
Asp Val Glu Phe Val Cys Lys Val Tyr Ser Asp Ala Gln Pro His Ile
        275                 280                 285
Gln Trp Ile Lys His Val Glu Lys Asn Gly Ser Lys Tyr Gly Pro Asp
    290                 295                 300
Gly Leu Pro Tyr Leu Lys Val Leu Lys Ala Ala Gly Val Asn Thr Thr
305                 310                 315                 320
Asp Lys Glu Ile Glu Val Leu Tyr Ile Arg Asn Val Thr Phe Glu Asp
                325                 330                 335
Ala Gly Glu Tyr Thr Cys Leu Ala Gly Asn Ser Ile Gly Ile Ser Phe
            340                 345                 350
His Ser Ala Trp Leu Thr Val Leu Pro Ala Pro Gly Arg Glu Lys Glu
        355                 360                 365
Ile Thr Ala Ser Pro Asp Tyr Leu Glu Ile Ala Ile Tyr Cys Ile Gly
    370                 375                 380
Val Phe Leu Ile Ala Cys Met Val Val Thr Val Ile Leu Cys Arg Met
385                 390                 395                 400
Lys Asn Thr Thr Lys Lys Pro Asp Phe Ser Ser Gln Pro Ala Val His
                405                 410                 415
Lys Leu Thr Lys Arg Ile Pro Leu Arg Arg Gln Val Thr Val Ser Ala
            420                 425                 430
Glu Ser Ser Ser Ser Met Asn Ser Asn Thr Pro Leu Val Arg Ile Thr
        435                 440                 445
Thr Arg Leu Ser Ser Thr Ala Asp Thr Pro Met Leu Ala Gly Val Ser
    450                 455                 460
Glu Tyr Glu Leu Pro Glu Asp Pro Lys Trp Glu Phe Pro Arg Asp Lys
465                 470                 475                 480
Leu Thr Leu Gly Lys Pro Leu Gly Glu Gly Cys Phe Gly Gln Val Val
                485                 490                 495
Met Ala Glu Ala Val Gly Ile Asp Lys Asp Lys Pro Lys Glu Ala Val
            500                 505                 510
Thr Val Ala Val Lys Met Leu Lys Asp Asp Ala Thr Glu Lys Asp Leu
        515                 520                 525
Ser Asp Leu Val Ser Glu Met Glu Met Met Lys Met Ile Gly Lys His
    530                 535                 540
Lys Asn Ile Ile Asn Leu Leu Gly Ala Cys Thr Gln Asp Gly Pro Leu
545                 550                 555                 560
Tyr Val Ile Val Glu Tyr Ala Ser Lys Gly Asn Leu Arg Glu Tyr Leu
                565                 570                 575
Arg Ala Arg Arg Pro Pro Gly Met Glu Tyr Ser Tyr Asp Ile Asn Arg
            580                 585                 590
Val Pro Glu Glu Gln Met Thr Phe Lys Asp Leu Val Ser Cys Thr Tyr
        595                 600                 605
Gln Leu Ala Arg Gly Met Glu Tyr Leu Ala Ser Gln Lys Cys Ile His
    610                 615                 620
Arg Asp Leu Ala Ala Arg Asn Val Leu Val Thr Glu Asn Asn Val Met
625                 630                 635                 640
Lys Ile Ala Asp Phe Gly Leu Ala Arg Asp Ile Asn Asn Ile Asp Tyr
                645                 650                 655
Tyr Lys Lys Thr Thr Asn Gly Arg Leu Pro Val Lys Trp Met Ala Pro
            660                 665                 670

Glu Ala Leu Phe Asp Arg Val Tyr Thr His Gln Ser Asp Val Trp Ser
        675                 680                 685
Phe Gly Val Leu Met Trp Glu Ile Phe Thr Leu Gly Gly Ser Pro Tyr
    690                 695                 700
Pro Gly Ile Pro Val Glu Glu Leu Phe Lys Leu Leu Lys Glu Gly His
705                 710                 715                 720
Arg Met Asp Lys Pro Ala Asn Cys Thr Asn Glu Leu Tyr Met Met Met
                725                 730                 735
Arg Asp Cys Trp His Ala Val Pro Ser Gln Arg Pro Thr Phe Lys Gln
            740                 745                 750
Leu Val Glu Asp Leu Asp Arg Ile Leu Thr Leu Thr Thr Asn Glu Gln
        755                 760                 765
Ile Gln Phe Ala Asp Asp Met Gln Glu Phe Thr Lys Phe Pro Thr Lys
    770                 775                 780
Thr Gly Arg Arg Ser Leu Ser Arg Ser Ile Ser Gln Ser Ser Thr Asp
785                 790                 795                 800
Ser Tyr Ser Ser Ala Ala Ser Tyr Thr Asp Ser Ser Asp Asp Glu Val
                805                 810                 815
Ser Pro Arg Glu Lys Gln Gln Thr Asn Ser Lys Gly Ser Ser Asn Phe
            820                 825                 830
Cys Val Lys Asn Ile Lys Gln Ala Glu Phe Gly Arg Arg Glu Ile Glu
        835                 840                 845
Ile Ala Glu Gln Asp Met Ser Ala Leu Ile Ser Leu Arg Lys Arg Ala
    850                 855                 860
Gln Gly Glu Lys Pro Leu Ala Gly Ala Lys Ile Val Gly Cys Thr His
865                 870                 875                 880
Ile Thr Ala Gln Thr Ala Val Leu Ile Glu Thr Leu Cys Ala Leu Gly
                885                 890                 895
Ala Gln Cys Arg Trp Ser Ala Cys Asn Ile Tyr Ser Thr Gln Asn Glu
            900                 905                 910
Val Ala Ala Ala Leu Ala Glu Ala Gly Val Ala Val Phe Ala Trp Lys
        915                 920                 925
Gly Glu Ser Glu Asp Asp Phe Trp Trp Cys Ile Asp Arg Cys Val Asn
    930                 935                 940
Met Asp Gly Trp Gln Ala Asn Met Ile Leu Asp Asp Gly Gly Asp Leu
945                 950                 955                 960
Thr His Trp Val Tyr Lys Lys Tyr Pro Asn Val Phe Lys Lys Ile Arg
                965                 970                 975
Gly Ile Val Glu Glu Ser Val Thr Gly Val His Arg Leu Tyr Gln Leu
            980                 985                 990
Ser Lys Ala Gly Lys Leu Cys Val  Pro Ala Met Asn Val  Asn Asp Ser
        995                 1000                1005
Val Thr  Lys Gln Lys Phe Asp  Asn Leu Tyr Cys Cys  Arg Glu Ser
    1010                1015                1020
Ile Leu  Asp Gly Leu Lys Arg  Thr Thr Asp Val Met  Phe Gly Gly
    1025                1030                1035
Lys Gln  Val Val Val Cys Gly  Tyr Gly Glu Val Gly  Lys Gly Cys
    1040                1045                1050
Cys Ala  Ala Leu Lys Ala Leu  Gly Ala Ile Val Tyr  Ile Thr Glu
    1055                1060                1065
Ile Asp  Pro Ile Cys Ala Leu  Gln Ala Cys Met Asp  Gly Phe Arg
    1070                1075                1080
Val Val  Lys Leu Asn Glu Val  Ile Arg Gln Val Asp  Val Val Ile
    1085                1090                1095
Thr Cys  Thr Gly Asn Lys Asn  Val Val Thr Arg Glu  His Leu Asp
    1100                1105                1110
Arg Met  Lys Asn Ser Cys Ile  Val Cys Asn Met Gly  His Ser Asn
    1115                1120                1125
Thr Glu  Ile Asp Val Thr Ser  Leu Arg Thr Pro Glu  Leu Thr Trp
    1130                1135                1140
Glu Arg  Val Arg Ser Gln Val  Asp His Val Ile Trp  Pro Asp Gly
    1145                1150                1155
Lys Arg  Val Val Leu Leu Ala  Glu Gly Arg Leu Leu  Asn Leu Ser

Cys Ser Thr Val Pro Thr Phe Val Leu Ser Ile Thr Ala Thr Thr
    1160                1165                1170
Gln Ala Leu Ala Leu Ile Glu Leu Tyr Asn Ala Pro Glu Gly Arg
    1175                1180                1185
Tyr Lys Gln Asp Val Tyr Leu Leu Pro Lys Lys Met Asp Glu Tyr
    1190                1195                1200
Val Ala Ser Leu His Leu Pro Ser Phe Asp Ala His Leu Thr Glu
    1205                1210                1215
Leu Thr Asp Asp Gln Ala Lys Tyr Leu Gly Leu Asn Lys Asn Gly
    1220                1225                1230
Pro Phe Lys Pro Asn Tyr Tyr Arg Tyr
    1235                1240                1245
    1250                1255